# EUROPÄISCHE PATENTANMELDUNG

(11) **EP 1 136 501 A2**
(43) Veröffentlichungstag der Anmeldung: **26.09.2001**
(21) Anmeldenummer: 01104576.2
(22) Anmeldetag: 06.03.2001
(51) Int. Cl.: C07K 14/435, C07K 14/705, C12N 15/12, C12N 15/66, C12N 15/71, C12N 5/10, C07K 16/18, A01K 67/027, C12N 15/11, C12Q 1/68, G01N 33/48, A01N 63/00

(54) **Rezeptoren für Peptide aus Insekten**

(30) Priorität: 18.03.2000 DE 10013618
(71) Anmelder: Bayer Aktiengesellschaft, 51368 Leverkusen (DE)
(72) Erfinder: Antonicek, Horst-Peter, Dr., 51467 Bergisch Gladbach (DE); Friedrich, Gabi, Dr., 51377 Leverkusen (DE); Schulte, Thomas, Dr., 51061 Köln (DE)

(57) **Zusammenfassung**

Die Erfindung betrifft Polypeptide, welche die biologische Aktivität von Peptid-Rezeptoren ausüben, sowie Nukleinsäuren, die für diese Polypeptide kodieren und insbesondere deren Verwendung zum Auffinden von Wirkstoffen für den Pflanzenschutz.

## Beschreibung

Die Erfindung betrifft Polypeptide, welche die biologische Aktivität von Peptid-Rezeptoren ausüben, sowie Nukleinsäuren, die für diese Polypeptide kodieren und insbesondere deren Verwendung zum Auffinden von Wirkstoffen für den Pflanzenschutz.

Neuronale oder endokrine Peptide aus Insekten sind wichtige Zielproteine für die Entwicklung neuartiger Insektizide, da solche Peptide die meisten wichtigen Schlüsselfunktionen, wie zum Beispiel die Embryonal- und Post-Embryonal-Entwicklung, die Homöostase, Osmoregulation oder Muskelaktivität in Insekten regulieren (siehe Gäde et al., 1997a; Osborne, 1996). Die biologische Wirkung dieser Peptide wird dabei durch die Bindung an spezifische Rezeptor-Proteine in Insektenzellen vermittelt. Viele dieser endokrinen oder neuronalen Peptide interagieren mit G-Protein-gekoppelten Rezeptoren (GPCRs; King und Wilson, 1999), die nach der Bindung eines korresponierenden Peptid-Liganden heterotrimere G-Proteine aktivieren (Vanden Broeck et al., 1997). Antagonisten oder Agonisten von Peptid-Rezeptoren können beispielsweise mit der normalen Insekten-Entwicklung, dem Wachstum, dem Verhalten oder der Homöostase interferieren und auf diese Weise Insekten-spezifische und Rezeptor-spezifische neuartige Insektizide darstellen. Diese Antagonisten oder Agonisten von Peptid-Rezeptoren können entweder von den natürlichen Peptiden abgeleitet sein oder eine völlig neuartige chemische Struktur besitzen.

Seit der Isolierung des Neuropeptids Proctolin aus Insekten-Muskelpräparationen (Brown und Starratt, 1975) ist eine Vielzahl von Peptiden aus verschiedenen Insekten-Spezies isoliert und charakterisiert worden (zur Übersicht siehe Vanden Broeck et al., 1997; Osborne, 1996). Substantieller Fortschritt wurde vor allem bei der Aufklärung der Aminsäuresequenz solcher Peptide und bei der Aufklärung der biologischen Funktion in den entsprechenden Insekten-Spezies gemacht. So konnten beispielsweise Peptide, die die Juvenil-Hormon-Biosynthese beeinflussen können (Allatotropine und Allatostatine; Tobe et al., 1994), Insulin-artige Peptide (Lagueux et al., 1990), Peptide, die den Wasserhaushalt regulieren (Coast, 1998), oder solche Peptide, die die Muskelaktivität steuern können (Holman, 1986; zur Übersicht siehe Gäde, 1997b), aus verschiedenen Spezies isoliert werden. Die biologischen Funktionen der Peptide kann in verschiedenen Tests überprüft werden, welche beispielsweise die Muskelaktivität (Holman et al., 1991) oder die Ausscheidung von Wasser und Elektrolyten (Ramsey, 1954) messen.

Während eine Vielzahl von Peptiden isoliert, in der Struktur aufgeklärt und die Aminosäuresequenz beschrieben werden konnte, sind in Insekten nur wenige Rezeptoren bekannt, die endokrine oder neuronale Peptide binden können (Reagan, 1994; Osborne, 1996; Birgul et al., 1999). Es ist daher von großer praktischer Bedeutung, beispielsweise für die Suche nach neuen Insektiziden, Peptid-bindende Rezeptoren aus Insekten bereitzustellen.

Der vorliegenden Erfindung liegt somit insbesondere die Aufgabe zugrunde, Rezeptoren aus Insekten, im folgenden Rezeptoren genannt, an die endokrine oder neuronale Peptide aus Insekten binden können und über deren Bindung die biologische Funktionen dieser Peptide vermittelt werden kann, sowie darauf aufbauende Testsysteme mit einem hohen Durchsatz an Testverbindungen (High Throughput Screening Assays; HTS-Assays) bereitzustellen.

Die Aufgabe wird gelöst durch die Bereitstellung von Polypeptiden, welche zumindest eine biologische Aktivität eines Peptid-Rezeptors ausüben und eine Aminosäuresequenz umfassen, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit einer Sequenz gemäß SEQ ID NO: 2, 4, 6, 8, 10, 12, 14, 16, 18, 20, 22, 24, 26, 28, 30, 32, 34, 36, 38, 40, 42, 44 oder 46 über eine Länge von wenigstens 20, vorzugsweise wenigstens 25, besonders bevorzugt wenigstens 30 fortlaufenden Aminosäuren und ganz besonders bevorzugt über deren Gesamtlängen aufweisen.

Der Grad der Identität der Aminosäuresequenzen wird vorzugsweise bestimmt mit Hilfe des Programms GAP aus dem Programmpaket GCG, Version 9.1 unter Standardeinstellungen (Devereux et al., 1984).

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen.

Die erfindungsgemäßen Polypeptide müssen nicht vollständige Rezeptoren darstellen, sondern können auch nur Fragmente davon sein, solange sie zumindest noch eine biologische Aktivität der vollständigen Rezeptoren aufweisen. Polypeptide, die im Vergleich zu Rezeptoren, die aus den erfindungsgemäßen Polypeptiden mit einer Aminosäuresequenz gemäß SEQ ID NO: 2, 4, 6, 8, 10, 12, 14, 16, 18, 20, 22, 24, 26, 28, 30, 32, 34, 36, 38, 40, 42, 44 oder 46 bestehen, eine um 50 % höhere oder verminderte Aktivität ausüben, werden noch als erfindungsgemäß betrachtet. Dabei müssen die erfindungsgemäßen Polypeptide nicht von Rezeptoren aus Drosophila melanogaster ableitbar sein. Als erfindungsgemäß werden auch Polypeptide betrachtet, die Rezeptoren beispielsweise der folgenden Invertebraten entsprechen oder Fragmenten davon, die noch die biologische Aktivität dieser Rezeptoren ausüben können: Insekten, Nematoden, Arthropoden, Mollusken.

Die erfindungsgemäßen Polypeptide können im Vergleich zu der entsprechenden Region natürlich vorkommender Rezeptoren Deletionen oder Aminosäuresubstitutionen aufweisen, solange sie zumindest noch eine biologische Aktivität der vollständigen Rezeptoren ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Der Ausdruck "biologische Aktivität eines Peptid-Rezeptors", wie er hierin verwendet wird, bedeutet die Bindung von einem Peptid an den Rezeptor.

Bevorzugte Ausführungsformen der erfindungsgemäßen Polypeptide stellen Rezeptoren von Drosophila melanogaster dar, welche die Aminosäuresequenz gemäß SEQ ID NO: 2, 4, 6, 8, 10, 12, 14, 16, 18, 20, 22, 24, 26, 28, 30, 32, 34, 36, 38, 40, 42, 44 oder 46 besitzen.

Gegenstand der vorliegenden Erfindung sind auch Nukleinsäuren, die für die erfindungsgemäßen Polypeptide kodieren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Bevorzugte Ausführungsformen der erfindungsgemäßen Nukleinsäuren stellen cDNAs dar, welche eine Nukleinsäuresequenz gemäß SEQ ID NO 1, 3, 5, 7, 9, 11, 13, 15, 17, 19, 21, 23, 25, 27, 29, 31, 33, 35, 37, 39, 41, 43 oder 45 besitzen.

Nukleinsäuren, welche unter stringenten Bedingungen an die Sequenzen gemäß SEQ ID NO: 1, 3, 5, 7, 9, 11, 13, 15, 17, 19, 21, 23, 25, 27, 29, 31, 33, 35, 37, 39, 41, 43 oder 45 hybridisieren, sind ebenfalls von der vorliegenden Erfindung umfasst.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin offenbarten Sequenzinformation beispielsweise DNA-Fragmente aus anderen Insekten als Drosophila melanogaster isoliert werden, welche für Polypeptide mit der biologischen Aktivität von Rezeptoren kodieren.

Bevorzugte Hybridisierungsbedingungen sind nachstehend angegeben:

Hybridisierungslösung: 6X SSC / 0 % Formamid, bevorzugte Hybridisierungslösung: 6X SSC / 25 % Formamid.

Hybridisierungstemperatur: 34°C, bevorzugte Hybridisierungstemperatur: 42°C.
1. Waschschritt: 2X SSC bei 40°C,
2. Waschschritt: 2X SSC bei 45°C; bevorzugter 2. Waschschritt: 0,6X SSC bei 55°C; besonders bevorzugter 2. Waschschritt: 0,3X SSC bei 65°C.

Weiterhin sind von der vorliegenden Erfindung Nukleinsäuren umfasst, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit einer Sequenz gemäß SEQ ID NO: 1, 3, 5, 7, 9, 11, 13, 15, 17, 19, 21, 23, 25, 27, 29, 31, 33, 35, 37, 39, 41, 43 oder 45 über eine Länge von wenigstens 20, vorzugsweise wenigstens 25, besonders bevorzugt wenigstens 30 fortlaufenden Nukleotiden und ganz besonders bevorzugt über deren Gesamtlängen aufweisen.

Der Grad der Identität der Nukleinsäuresequenzen wird vorzugsweise bestimmt mit Hilfe des Programms GAP aus dem Programmpaket GCG, Version 9.1 unter Standardeinstellungen.

Gegenstand der vorliegenden Erfindung sind weiterhin DNA-Konstrukte, die eine erfindungsgemäße Nukleinsäure und einen heterologen Promotor umfassen.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert. Der Ausdruck "Promotor", wie er hierin verwendet wird, bezieht sich allgemein auf Expressionskontrollsequenzen.

Die Auswahl von heterologen Promotoren ist davon abhängig, ob zur Expression pro- oder eukaryotische Zellen oder zellfreie Systeme verwendet werden. Beispiele für heterologe Promotoren sind der frühe oder späte Promotor des SV40, des Adenovirus oder des Cytomegalovirus, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase und der Promotor des α-Mating-Faktors der Hefe.

Gegenstand der Erfindung sind weiterhin Vektoren, die eine erfindungsgemäße Nukleinsäure bzw. ein erfindungsgemäßes DNA-Konstrukt enthalten. Als Vektoren können alle in molekularbiologischen Laboratorien verwendete Plasmide, Phasmide, Cosmide, YACs oder künstliche Chromosomen verwendet werden.

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, wie Bakterien der Gattungen Bacillus, Pseudomonas, Streptomyces, Streptococcus, Staphylococcus, vorzugsweise E. coli, als auch eukaryotische Zellen, wie Hefen, Säuger-, Amphibien-, Insekten- oder Pflanzenzellen. Bevorzugte eukaryotische Wirtszellen sind HEK-293-, Schneider S2-, Spodoptera Sf9-, Kc-, CHO-, COS1-, COS7-, HeLa-, C127-, 3T3-oder BHK-Zellen und insbesondere Xenopus-Oocyten.

Weiterhin sind Antikörper Gegenstand der Erfindung, die spezifisch an die vorstehend genannten Polypeptide bzw. Rezeptoren binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Polypeptids oder eines Fragments davon und durch nachfolgende Gewinnung dieses Antikörpers. Weiterhin lässt sich in an sich bekannter Weise eine immortalisierte Zellinie erhalten, die monoklonale Antikörper produziert. Die Antikörper können gegebenenfalls mit einem Nachweisreagenz markiert sein. Bevorzugte Beispiele für ein solches Nachweis-Reagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften besitzen. Daher erstreckt sich der Ausdruck "Antikörper", wie er hierin verwendet wird, auch auf Teile vollständiger Antikörper, wie Fa-, F(ab')₂- oder Fv-Fragmente, welche noch die Fähigkeit besitzen, an die Epitope der erfindungsgemäßen Polypeptide zu binden.

Die erfindungsgemäßen Nukleinsäuren können insbesondere zur Herstellung transgener Invertebraten verwendet werden. Diese können in Testsysteme eingesetzt werden, die auf einer vom Wildtyp abweichenden Expression der erfindungsgemäßen Polypeptide basieren. Ferner kann man auf der Grundlage der hierin offenbarten Informationen transgene Invertebraten herstellen, bei denen durch die Modifikation anderer Gene oder Promotoren eine Veränderung der Expression der erfindungsgemäßen Polypeptide eintritt.

Die Herstellung der transgenen Invertebraten erfolgt beispielsweise bei Drosophila melanogaster durch P-Element vermittelten Gentransfer (Hay et al., 1997) oder in Caenorhabditis elegans durch Transposon-vermittelten Gentransfer (z.B. durch Tc1; Plasterk, 1996).

Gegenstand der Erfindung sind somit auch transgene Invertebraten, die zumindest eine der erfindungsgemäßen Nukleinsäuren enthalten, vorzugsweise transgene Invertebraten der Arten Drosophila melanogaster oder Caenorhabditis elegans sowie deren transgene Nachkommen. Vorzugsweise enthalten die transgenen Invertebraten die erfindungsgemäßen Polypeptide in einer vom Wildtyp abweichenden Form.

Gegenstand der vorliegenden Erfindung sind weiterhin Verfahren zum Herstellen der erfindungsgemäßen Polypeptide. Zur Herstellung der Polypeptide, die von den erfindungsgemäßen Nukleinsäuren codiert werden, können Wirtszellen, die eine der erfindungsgemäßen Nukleinsäuren enthalten, unter geeigneten Bedingungen kultiviert werden. Dabei kann die zu exprimierende Nukleinsäure an die "Codon Usage" der Wirtszellen angepasst werden. Die gewünschten Polypeptide können danach auf übliche Weise aus den Zellen oder dem Kulturmedium isoliert werden. Die Polypeptide können auch in *in vitro*-Systemen hergestellt werden.

Ein schnelles Verfahren zum Isolieren der erfindungsgemäßen Polypeptide, die von Wirtszellen unter Verwendung einer erfindungsgemäßen Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkern zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentieller Präzipitation, Ionenaustausch-Chromatographie und Affinitätschromatographie.

Da die Rezeptoren Membranproteine darstellen, werden in den Reinigungsverfahren vorzugsweise Detergensextraktionen durchgeführt, beispielsweise unter Verwendung von Detergenzien, die die Sekundär- und Tertiärstrukturen der Polypeptide nicht oder nur wenig beeinflussen, wie nicht-ionische Detergenzien.

Die Reinigung der erfindungsgemäßen Polypeptide kann die Isolierung von Membranen ausgehend von Wirtszellen, die die erfindungsgemäßen Nukleinsäuren exprimieren, umfassen. Vorzugsweise exprimieren solche Zellen die erfindungsgemäßen Polypeptide in einer ausreichenden Kopienanzahl, so dass die Menge der Polypeptide in einer Membranfraktion mindestens 10-fach höher ist als diejenige, die in vergleichbaren Membranen von Zellen gefunden wird, die Rezeptoren natürlicherweise exprimieren; besonders bevorzugt ist Menge mindestens 100-fach, ganz besonders bevorzugt mindestens 1000-fach höher.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Ferner sind auch Verfahren zum Herstellen der erfindungsgemäßen Nukleinsäuren Gegenstand der vorliegenden Erfindung. Die erfindungsgemäßen Nukleinsäuren können auf die übliche Weise hergestellt werden. Beispielsweise können die Nukleinsäuremoleküle vollständig chemisch synthetisiert werden. Man kann auch nur kurze Stücke der erfindungsgemäßen Sequenzen chemisch synthetisieren und solche Oligonukleotide radioaktiv oder mit einem Fluoreszenzfarbstoff markieren. Die markierten Oligonukleotide können verwendet werden, um ausgehend von Insekten-mRNA hergestellte cDNA-Banken oder ausgehend von genomischer Insekten-DNA hergestellte genomische Banken zu durchsuchen. Klone, an die die markierten Oligonukleotide hybridisieren, werden zur Isolierung der betreffenden DNA ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Mit Hilfe der erfindungsgemäßen Nukleinsäuren bzw. Polypeptide können neue Wirkstoffe für den Pflanzenschutz bzw. pharmazeutische Wirkstoffe für die Behandlung von Mensch und Tier, wie chemische Verbindungen, welche als Modulatoren, insbesondere als Agonisten oder Antagonisten, die Eigenschaften der erfindungsgemäßen Rezeptoren verändern, identifiziert werden. Dazu wird ein rekombinantes DNA-Molekül, das zumindest eine erfindungsgemäße Nukleinsäure umfasst, in eine geeignete Wirtszelle eingebracht. Die Wirtzelle wird in Gegenwart einer Verbindung oder einer Probe, welche eine Vielzahl von Verbindungen umfasst, unter Bedingungen kultiviert, die die Expression der erfindungsgemäßen Rezeptoren erlauben. Eine Veränderung der Rezeptoreigenschaften kann beispielsweise wie nachstehend in Beispiel 2 beschrieben detektiert werden. Auf diese Weise ist es möglich, beispielsweise insektizide Substanzen aufzufinden.

Rezeptoren verändern vorzugsweise nach Aktivierung die Konzentration von intrazellulärem cAMP über eine Interaktion mit G-Proteinen. Veränderungen der Rezeptoreigenschaften durch chemische Verbindungen können deshalb nach heterologer Expression zum Beispiel durch Messung der intrazellulären cAMP-Konzentrationen direkt über ELISA-Assaysysteme (Biomol, Hamburg, Deutschland) oder RIA-Assaysysteme (NEN, Schwalbach, Deutschland) im HTS-Format gemessen werden. Eine indirekte Messung der cAMP-Konzentration ist mit Hilfe von Reportergenen (z.B. Luziferase) möglich, deren Expression von der cAMP-Konzentration abhängig ist (Stratowa et al., 1995). Die Koexpression von Rezeptoren mit besonderen G-Proteinen, z.B. Gα15, Gα16 oder auch chimären G-Proteinen, in heterologen Systemen und die Messung des Anstiegs von Calcium, z.B. mit Fluoreszenzfarbstoffen oder Äquorin, ist eine alternative Screeningmöglichkeit (Stables et al., 1997, Conklin et al., 1993).

Weiterhin kann die Bindung von GTP an das aktivierte G-Protein als read-out-System für die Prüfung von Substanzen herangezogen werden. Auch Bindungsexperimente mit markiertem Peptiden können zum Screening eingesetzt werden.

Der Ausdruck "Agonist", wie er hierin verwendet wird, bezieht sich auf ein Molekül, das den Rezeptoren aktiviert.

Der Ausdruck "Antagonist", wie er hierin verwendet wird, bezieht sich auf ein Molekül, das einen Agonisten von seiner Bindungsstelle verdrängt.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können Mimetika von natürlichen Substraten und Liganden darstellen.

Vorzugsweise handelt es sich bei den Modulatoren um kleine organisch-chemische Verbindungen.

Die Bindung der Modulatoren an die erfindungsgemäßen Polypeptide kann die zellulären Vorgänge auf eine Weise verändern, die zum Absterben der damit behandelten Insekten führt.

Daher erstreckt sich die vorliegende Erfindung auch auf die Verwendung von Modulatoren der erfindungsgemäßen Polypeptide als Insektizide oder Arzneimittel.

Die erfindungsgemäßen Nukleinsäuren bzw. Polypeptide ermöglichen auch das Auffinden von Verbindungen, die an die erfindungsgemäßen Rezeptoren binden. Diese können ebenfalls als Insektizide auf Pflanzen bzw. pharmazeutische Wirkstoffe für die Behandlung von Mensch und Tier angewandt werden. Beispielsweise werden Wirtszellen, die die erfindungsgemäßen Nukleinsäuren enthalten und die entsprechenden Rezeptoren bzw. Polypeptide exprimieren oder die Genprodukte selbst mit einer Verbindung oder einem Gemisch von Verbindungen unter Bedingungen in Kontakt gebracht, die die Wechselwirkung zumindest einer Verbindung mit den Wirtszellen, den Rezeptoren oder den einzelnen Polypeptiden erlauben.

Unter Verwendung von Wirtszellen oder transgenen Invertebraten, die die erfindungsgemäßen Nukleinsäuren enthalten, ist es auch möglich, Substanzen aufzufinden, welche die Expression der Rezeptoren verändern.

Die vorstehend beschriebenen erfindungsgemäßen Nukleinsäuren, Vektoren und regulatorischen Regionen können außerdem zum Auffinden von Genen verwendet werden, die für Polypeptide kodieren, welche am Aufbau von funktionell ähnlichen Rezeptoren in Insekten beteiligt sind. Unter funktionell ähnlichen Rezeptoren werden gemäß der vorliegenden Erfindung Rezeptoren verstanden, die Polypeptide umfassen, welche sich zwar hinsichtlich der Aminosäuresequenz von den hierin beschriebenen Polypeptiden unterscheiden, aber im wesentlichen dieselben Funktionen haben.

### Erläuterungen zum Sequenzprotokoll und zu der Abbildung:

SEQ ID NO: 1, 3, 5, 7, 9, 11, 13, 15, 17, 19, 21, 23, 25, 27, 29, 31, 33, 35, 37, 39, 41, 43 und 45 zeigen die Nukleotid- und Aminosäuresequenzen der isolierten Rezeptor-cDNAs. SEQ ID NO: 2, 4, 6, 8, 10, 12, 14, 16, 18, 20, 22, 24, 26, 28, 30, 32, 34, 36, 38, 40 ,42, 44 und 46 zeigen ferner die Aminosäuresequenzen der von den Rezeptor-cDNA-Sequenzen abgeleiteten Proteine.

### Beispiele:

### Beispiel 1

Isolierung der beschriebenen Polynukleotide

Die Manipulation von Polynukleotiden erfolgte nach Standardmethoden der rekombinanten DNA Technologie (Sambrook et al., 1989). Die bioinformatische Bearbeitung von Nukleotid- und Proteinsequenzen erfolgten mit dem Programmpaket GCG Version 9.1 (GCG Genetics Computer Group, Inc., Madison Wisconsin, USA).

Isolierung von poly A enthaltender RNA aus Drosophila-Gewebe und Konstruktion der cDNA-Bibliotheken

Die RNA für die cDNA-Bibliothek I wurde aus gesamten Drosophila melanogaster Embryonen und Larven (RNAzol, Life Technologies, Karlsruhe, Deutschland, nach Angaben des Herstellers) isoliert. Aus dieser RNA wurden nun die poly A enthaltenden RNAs durch Reinigung über Dyna Beads 280 (Dynal, Hamburg, Deutschland) isoliert. 5 µg dieser poly A enthaltenden RNAs wurden anschließend in die Konstruktion der cDNA-Bibliothek mit dem λ-ZAP-CMV Vektor eingesetzt (cDNA Synthesis Kit, ZAP-cDNA Synthesis Kit und ZAP-cDNA Gigapack III Gold Cloning Kit, alle Stratagene-Europe, Amsterdam, Niederlande).

### Erzeugung von Plasmid Pools

Die cDNA-Bibliothek in Lambda-pCMV wurde nach Angaben des Herstellers einer Massen in vivo Exzision unterzogen, um eine Phagemid-Bibliothek zu erzeugen. Anschließend wurden 10 x 96 Minipräparations-Kulturen angeimpft, die rechnerisch 1000 Klone pro Präparation enthielten. Die DNA wurde dann mit dem Qiawell Ultra DNA Präparationssystem von Qiagen (Hilden, Deutschland) aufgereinigt und in 96-Loch-Mikrotiterplatten abgelegt. Dadurch wurde die Bibliothek in Form von 960 Pools ä 1000 cDNA Klonen abgebildet.

### PCR mit Bibliothek-Pools

Von jeder Mikrotiterplatte wurde ein Meta-Pool angelegt, der die gesamte Platte repräsentierte. Je 0,5 µl dieses Metapools wurde in eine PCR mit den folgenden Oligodesoxynukleotid-Primern eingesetzt:

Primer 1s :AAGGTCATCAAAATGCTGATT (SEQ ID NO: 47)
Primer 1a :ATTGTAGCAGCTATTGCTCAT (SEQ ID NO: 48)
Primer 2s :CAGCTCGTTCGATTCGGTCCT (SEQ ID NO: 49)
Primer 2a :GTGACAGCGGTCATAGTCCGA (SEQ ID NO: 50)
Primer 3s : ATCGAGGCATCCACCTATGGC (SEQ ID NO: 51)
Primer 3a : AGGTGGGCGCAGGCATCGTAG (SEQ ID NO: 52)
Primer 4s :TTGCTACGTAGTTCTGAGGAATC (SEQ ID NO: 53)
Primer 4a :ATGCAGGTGGAGAGCTTCATG (SEQ ID NO: 54)
Primer 5s :TGGCAGACGAGTGCTTCCTGA (SEQ ID NO: 55)
Primer 5a :GGACCGCTGAAGTTGACCAG (SEQ ID NO: 56)
Primer 6s :TGGTCTGGTACCTGCTGGTCA (SEQ ID NO: 57)
Primer 6a :GCGATGAGCCATTTGACCAGC (SEQ ID NO: 58)
Primer 7s :GTGACCCATGCGTTCATCATC (SEQ ID NO: 59)
Primer 7a :CTGCAGCATGGGCAGAAAG (SEQ ID NO: 60)
Primer 8s :GCGATCACCTGGAAGATCTGC (SEQ ID NO: 61)
Primer 8a :TGGTGATGCCAATAGGATACC (SEQ ID NO: 62)
Primer 9s :CTGTTGCACTTCCTGGTCTAC (SEQ ID NO: 63)
Primer 9a :ACGCACAGCTCCCTGAATTTC (SEQ ID NO: 64)
Primer 10s:GAGGAGCACGATGTGAGTGG (SEQ ID NO: 65)
Primer 10a:CGTGTAAACGGATAATTCTG (SEQ ID NO: 66)
Primer 11s:GTCTGGCTGATACCCAGCTA (SEQ ID NO: 67)
Primer 11a:ACGCTCTTGACTTTCTCGAAC (SEQ ID NO: 68)

Primer 12s:TTCGCACACCAGTTCTACGAC (SEQ ID NO: 69)
Primer 12a:GCGTTCATGAAGCAGTAGGTG (SEQ ID NO: 70)
Primer 13s:TACATCTGCATCGGACGTGG (SEQ ID NO: 71)
Primer 13a:CACTATGCCGCATTGCTCC (SEQ ID NO: 72)
Primer 14s:GTGATCTATGTGGTGATGAGG (SEQ ID NO: 73)
Primer 14a:CCTCGACTGCACGGTGCTGGC (SEQ ID NO: 74)
Primer 15s:GTCATCGTTCTGGGCAATTCA (SEQ ID NO: 75)
Primer 15a:GATGCTCATGGCCACCAGCAC (SEQ ID NO: 76)
Primer 16s:AACGTACTGCGAGTGATCGTG (SEQ ID NO: 77)
Primer 16a:AATGGCAAAGGTGACATCGTG (SEQ ID NO: 78)
Primer 17s:ATGTGCCGCATCAGCGAGTTC (SEQ ID NO: 79)
Primer 17a:GTTACCGGTGGCTGTGAACAC (SEQ ID NO: 80)
Primer 18s:CAGAAACCGCTCAAGGAGACG (SEQ ID NO: 81)
Primer 18a:CCTCAGACGAGCCGCAGTTAG (SEQ ID NO: 82)
Primer 19s:GCACTGGCACTGCTGCTGG (SEQ ID NO: 83)
Primer 19a:CACAGCCACCACGGTGATGC (SEQ ID NO:84)
Primer 20s:GGCACTTTGCCGTGGATAGTG (SEQ ID NO: 85)
Primer 20a:GAACCGATCGATGGACATCAG (SEQ ID NO: 86)
Primer 21s:CATCAGCTCCTACCTGCTGC (SEQ ID NO: 87)
Primer 21a:CGTAGAGCAGCGGATTGATAC (SEQ ID NO: 88)
Primer 22s:CATCTCACTGGCCTGCAGTGA (SEQ ID NO: 89)
Primer 22a:CATGCTCAGAGTCGACTTCG (SEQ ID NO: 90)
Primer 23s:GCGGTAATGGCACTGTTCTC (SEQ ID NO: 91)
Primer 23a:GGATACTGTGGAGAACCGGTA (SEQ ID NO: 92)

Die PCR-Parameter waren wie folgt: 94°C, 1 min; 35 mal (94°C, 30 s; 55°C, 30 s; 72°C, 45 s). Die PCRs liefen auf einem Biometra Uno II (Biometra, Göttingen, Deutschland).

In der PCR positive Bibliothek-Pools wurden in Xl-lBlue (Stratagene, Amsterdam, Niederlande) transformiert und einem Kolonie-Lift unterzogen (Sambrook et al.,1989). Als Sonde für die Hybridisierung diente ein mittels Psoralen-Biotin (BrightStar, Psoralen-Biotin Kit, Ambion, Austin, Texas, USA) markiertes PCR-Produkt aus der Reaktion mit dem jeweiligen Primerpaar (Hybridisierung und Detektion mittels BrightStar, Psoralen-Biotin Kit, Ambion, Austin, Texas, USA). Positive Kolonien wurden gepickt, vermehrt und die DNA per Plasmid-Präparation (Qiagen, Hilden, Deutschland) isoliert.

Die isolierten Genbankplasmide wurden zur Identifikation mittels T3-und T7-Primer ansequenziert (ABI Prism Dye Terminator Cycle Sequencing Kit, ABI, mit ABI Prism 310 Genetic Analyzer, ABI-Deutschland, Weiterstadt, Deutschland). Die Bestimmung der vollständigen Polynukleotidsequenzen des DB3 erfolgte durch Primer Walking mittels Cycle Sequencing ABI Prism Dye Terminator Cycle Sequencing Kit, ABI, mit ABI Prism 310 Genetic Analyzer (ABI-Deutschland, Weiterstadt, Deutschland).

### Beispiel 2

Die Zuordnung der Sequenzen aus SEQ ID NO: 2, 4, 6, 8, 10, 12, 14, 16, 18, 20, 22, 24, 26, 28, 30, 32, 34, 36, 38, 40, 42, 44 oder 46 erfolgte mittels Blast-Analyse (Altschul et al., 1997). Gezeigt ist der jeweils beste Treffer aus der Blast-Analyse (Swissprot Datenbank, vom 4.März, 2000). Der Parameter E-value ist ein Maß für die Nichtzufälligkeit der Zuordnung. Alle Sequenzen konnten mit hinreichender Sicherheit als Neuropeptidrezeptoren identifiziert werden.

| **Sequenzvergleiche und Zuordnung der Sequenzen** | | |
|---|---|---|
| **Seq ID** | **E-value** | **Accession No. / Accession aus Swissprot Datenbank (vom 4. März 2000)** |
| 2 | 8e-45 | P25931 / NEUROPEPTIDE Y RECEPTOR (NPY-R) (PR4 RECEPTOR) |
| 4 | 1e-81 | Q16983 / DIURETIC HORMONE RECEPTOR PRECURSOR (DH-R) |
| 6 | 1e-76 | Q16983 / DIURETIC HORMONE RECEPTOR PRECURSOR (DH-R) |
| 8 | 7e-41 | Q9Z2D5 / NEUROPEPTIDE Y RECEPTOR TYPE 2 (NPY2-R) |
| 10 | 8e-52 | P35346 / SOMATOSTATIN RECEPTOR TYPE 5 (SS5R) |
| 12 | | Q16602 / CALCITONIN GENE-RELATED PEPTIDE TYPE 1 RECEPTOR |
| 14 | 2e-05 | P56718 / OREXIN RECEPTOR TYPE 1 (OX1R) |
| 16 | 6e-25 | Q9Z2D5 / NEUROPEPTIDE Y RECEPTOR TYPE 2 (NPY2-R) |
| 18 | 1e-40 | P21729 / GASTRIN-RELEASING PEPTIDE RECEPTOR (GRP-R) |
| 20 | le-86 | O02721 / LUTROPIN-CHORIOGONADOTROPIC HORMONE RECEPTOR |
| 22 | 6e-19 | Q63931 / CHOLECYSTOKININ TYPE A RECEPTOR (CCK-A RECEPTOR) |
| 24 | 1e-33 | P56418 / GASTRIN/CHOLECYSTOKININ TYPE B RECEPTOR |
| 26 | 4e-34 | Q95254 / GROWTH HORMONE SECRETAGOGUE RECEPTOR TYPE 1 |
| 28 | 7e-54 | P34993 / SOMATOSTATIN RECEPTOR TYPE 2 (SS2R) |
| 30 | 3e-41 | P48043 / VASOPRESSIN V1A RECEPTOR |
| 32 | 5e-42 | Q60755 / CALCITONIN RECEPTOR PRECURSOR (CT-R) |
| 34 | 7e-39 | P47751 / PHE(13) BOMBESIN RECEPTOR |
| 36 | 6e-22 | P70031 / CHOLECYSTOKININ RECEPTOR (CCK-XLR) |
| 38 | 6e-41 | Q9Z2D5 / NEUROPEPTIDE Y RECEPTOR TYPE 2 (NPY2-R) |
| 40 | 1e-19 | O88626 / GALANIN RECEPTOR TYPE 3 (GAL3-R) |
| 42 | 5e-15 | P47211 / GALANIN RECEPTOR TYPE 1 (GAL1-R) |
| 44 | 2e-08 | Q95254 / GROWTH HORMONE SECRETAGOGUE RECEPTOR TYPE 1 |
| 46 | 7e-17 | Q62463 / VASOPRESSIN V1A RECEPTOR |

### Beispiel 3

### Heterologe Expression

Eine funktionelle Expression der Rezeptoren aus Insekten kann in Xenopus-Oocyten erfolgen. Dazu werden G-Protein aktivierbare Kalium-Kanäle (GIRK1 und GIRK4) koexprimiert, um die Aktivierung der Rezeptoren zu messen (White et al., 1998). Die erfindungsgemäßen Nukleotidsequenzen wurden, da sie sich bereits in einem Expressionsvektor mit CMV-Promoter befinden, direkt in die Expressionsversuche eingesetzt.

### Oocyten-Messungen

### 1. Präparation der Oocyten

Die Oocyten stammen von einem adulten weiblichen *Xenopus laevis*-Frosch (Firma Horst Kähler, Hamburg, Deutschland). Die Frösche werden in großen Tanks mit zirkulierendem Wasser bei einer Wassertemperatur von 20 - 24°C gehalten. Teile des Frosch-Ovars werden unter vollständiger Anaesthesie durch einen kleinen Schnitt im Abdomen (ca. 1 cm) entnommen. Anschließend wird das Ovar unter ständigem Schütteln für ca. 140 min in 25 ml Kollagenase (Typ I, C-0130, SIGMA-ALDRICH CHEMIE GmbH, Deisenhofen, Deutschland; 355U/ml, angesetzt in Barth's Lösung ohne Calcium in mM: NaCl 88, KCl 1, MgSO₄ 0.82, NaHCO₃ 2.4, Tris/HCl 5, pH 7,4) behandelt. Die Oocyten werden dann mit Barth's Lösung ohne Calcium gewaschen. Nur Oocyten im Reifestadium V (Dumont, 1972) werden für die weitere Behandlung ausgewählt und in Mikrotiterplatten (Nunc Micro Well™ Platten, Kat. Nr. 245128 + 263339 (Deckel), Nunc GmbH & Co. KG, Wiesbaden, Deutschland), gefüllt mit Barth's-Lösung (in mM: NaCl 88, KCl 1, MgSO₄ 0,82, Ca(NO₃)₂ 0,33, CaCl₂ 0,41, NaHCO₃ 2,4, Tris/HCl 5, pH 7,4) sowie Gentamicin (Gentamicin Sulfate, G-3632, SIGMA-ALDRICH CHEMIE GmbH, Deisenhofen, Deutschland; 100U/ml), überführt. Die Oocyten werden dann bei 19,2°C in einem Kühl-Brutschrank (Typ KB 53, WTB Binder Labortechnik GmbH, Tuttlingen, Deutschland) aufbewahrt.

### 2. Injektion der Oocyten

Injektionselektroden, mit einem Durchmesser von 10 bis 15 µm, werden mit einem Pipetten-Puller (Typ L/M-3P-A, List-electronic, Darmstadt-Eberstadt, Deutschland) hergestellt. Vor der Injektion werden Aliquots mit der Rezeptor-DNA bzw. GIRK1/4-DNA aufgetaut und mit Wasser auf eine Endkonzentration von 10 ng/µl verdünnt. Die DNA-Proben werden mit 3200 g für 120 s zentrifugiert (Typ Biofuge 13, Heraeus Instruments GmbH, Hanau, Deutschland). Anschließend wird ein ausgezogener PE-Schlauch als Transferschlauch benutzt, um die Pipetten von hinten zu befüllen. Die Injektionselektroden werden an einer X,Y,Z-Verfahreinheit (Bearbeitungszentrum EP1090, isel-automation, Eiterfeld, Deutschland) befestigt. Mit Hilfe eines Macintosh Computer wird die Oocyten in den Vertiefungen der Mikrotiterplatten angefahren und durch kurze Druckapplikation (0,5-3,0 bar, 3-6 s) ca. 50 nl der DNA-Lösung in die Oocyte injiziert.

### 3. Elektrophysiologische Messungen

Für die elektrophysiologischen Messungen wird eine Zwei-Elektroden-Spannungsklemme mit einem TURBO TEC-10CD (npi electronic GmbH, Tamm, Deutschland) Verstärker durchgeführt. Die hierfür notwendigen Mikropipetten werden aus Aluminiumsilikatglas (Kapillarrohr, Art.-Nr. 14 630 29, 1 = 100 mm, Ø a = 1,60 mm, Øi=1,22mm, Hilgenberg GmbH, Malsfeld, Deutschland) in zwei Zügen gezogen (Hamill et al., 1981). Strom- und Spannungselektroden haben einen Durchmesser von 1 - 3 µm und werden mit 1,5 M KCl und 1,5 M Kaliumacetat gefüllt. Die Pipetten haben einen kapazitiven Widerstand von 0,2 - 0,5 MW. Für die elektrophysiologischen Messungen werden die Oocyten in eine kleine Kammer überführt, die kontinuierlich mit normaler Rimland-Lösung (in mM: KCl 90, MgCl₂ 3, HEPES 5, pH 7,2) gespült wird. Für eine Substanzapplikation wird die Perfusionslösung durch eine Substanzlösung mit gleicher Zusammensetzung und zusätzlich der gewünschten Substanzkonzentration ausgetauscht. Bei einem Klemmpotential von -60mV wird die erfolgreiche Expression der Rezeptor-DNA nach einer Woche überprüft. Nicht reagierende Oocyten werden verworfen. Alle weiteren werden für die Substanztestung verwendet. Die Daten werden mittels eines YT-Schreibers (YT-Schreiber, Model BD 111, Kipp & Zonen Delft BV, AM Delft, Niederlande) dokumentiert. Wenn Testsubstanzen in Konzentrationsreihen untersucht werden, werden diese Messungen an mindestens zwei verschiedenen Oocyten und an mindestens fünf verschiedenen Konzentrationen durchgeführt. Die Substanzen werden direkt und ohne Vorinkubation in Gegenwart von Glutamat (Gamma-Amino-N-butyric acid, A2129, SIGMA-ALDRICH CHEMIE GmbH, Deisenhofen, Deutschland) auf ihren Antagonismus geprüft. Die einzelnen Werte werden in Origin (Auswertesoftware Microcal Origin, Microcal Software, Inc., Northampton, MA 01060-4410, USA) (Additive GmbH, Friedrichsdorf/Ts, Deutschland) eingegeben. Mittelwerte, Standardabweichung, IC₅₀-Werte und IC₅₀-Kurven werden mit Origin berechnet. Diese Messungen werden mindestens zweimal durchgeführt.

### Literatur:

Altschul et al. (1997), Gapped BLAST and PSI-BLAST: a new generation of protein database search programs, Nucleic Acids Res. 25:3389-3402.

Birgul, N. et al. (1999), Reverse physiology in Drosophila: identification of a novel allatostatin-like neuropeptide and its cognate receptor structurally related to the mammalian somatostatin/galanin/opioid receptor family, EMBO Journal 18, 5892-5900.

Brown, B.E. und Starrall, A.N. (1975), Isolation of proctolin, a myotropic peptide from Periplaneta americana, Journal of Insect Physiology 21, 1879-1881.

Coast, G.M. (1998), Insect Diuretic Peptides: Structures, Evolution and Actions, American Zoology 38, 442-449.

Conklin et al. (1993), Substitution of three amino acids switches receptor specificity of Gq alpha to that of Gi alpha, Nature 20;363(6426):274-6.

Devereux et al. (1984), Nucleic Acids Research 12, 387.

Dumont, J.N. (1972), Oogenesis in *Xenopus laevis* (Daudin). 1. Stages of oocyte development in laboratory maintained animals, J. Morphol. 136: 153-180.

Gäde, G. (1997a), The explosion of structural information on insect neuropeptides, In: Progress in the chemistry of organic natural products (Herz, W., Kirby, G.W., Moore, R.E., Steglich, W., Tamm, C. eds), 1-128.

Gäde, G. et al. (1997b), Hormonal regulation in Insects: Facts, Gaps, and Future Directions, Physiological Reviews 77, 963-1032.

Hamill, O.P., Marty, A., Neher, E., Sakmann, B. Sigworth, F.J. (1981), Improved patch-clamp techniques for high-resolution current recording from cells and cell-free membrane patches, Pfügers Arch. 391: 85-100.

Hay et al. (1997), P element insertion-dependent gene activation in the Drosophila eye, Proceedings of The National Academy of Sciences of The United States of America 94 (10), 5195-5200.

Holman, G.M. et al. (1986), Isolation, primary structure and synthesis of two neuropeptides from Leucophaea maderae: Members of a new family of Cephalomaotrophins, Comparative Biochemical Physiology 84C, 205.

Holman, G.M. (1991), Insect myotropic peptides: isolation, structural characterization and biological properties, In: Insect Neuropeptides: Chemistry, Biology and Action (Menn, J.J., Kelly, T.J., Masler, E.P., eds), 40-50.

King, F.D. und Wilson, S. (1999), Recent advances in 7-transmembrane receptor research, Current Opinion in Drug Discovery & Development 2, 83-95.

Lagueux, M. et al. (1990), cDNAs from neurosecretory cells of brains of Locusta migratoria encoding a novel member of the superfamily of insulins, European Journal of Biochemistry 187, 249-254.

Osborne, R.H. (1996), Insect Neurotransmission: Neurotransmitters and their Receptors, Pharmacology & Therapeutics 69, 117-142.

Plasterk (1996), The Tc1/mariner transposon family, Transposable Elements/Current Topics in Microbiology and Immunology 204, 125-143

Ramsey, J.A. (1954), Active transport of water by the Malpighian tubules of the stick insect, Dixippus morosus, Journal of Experimental Biology 31, 104-113.

Reagan, J.D. (1994), Expression cloning of an insect diuretic hormone receptor, Journal of Biological Chemistry 269, 9-12.

Sambrook et al. (1989), Molecular Cloning, A Laboratory Manual, 2nd ed. Cold Spring Harbour Press.

Stables et al. (1997), A Bioluminescent Assay for Agonist Activity at Potentially Any G-protein coupled Receptor, Analytical Biochemistry 252, 115-126.

Stratowa C et al. (1995), Use of a luciferase reporter system for characterizing G-protein-linked receptors, Curent Opinion in Biotechnology 6, 574-581.

Tobe, S.S. et al. (1994), Allatostatins, peptide inhibitors of juvenile hormone production in insects, In: Perspectives in Comparative Endocrinology (Davey, K.G., Peter, R.E., Tobe, S.S., eds), 12-19.

Vanden Broeck, J. et al. (1997), Insect Neuropeptides and Their Receptors, Trends in Endocrinology & Metabolism 8, 321-326.

White JH et al. (1998), Heterodimerization is required for the formation of a functional GABA(B) receptor, Nature 396, 679-82.

## Patentansprüche

1. Polypeptid, welches die biologische Aktivität eines Peptid-Rezeptors ausübt und eine Aminosäuresequenz umfasst, die eine zumindest 70%ige Identität mit einer Sequenz gemäß SEQ ID NO: 2, 4, 6, 8, 10, 12, 14, 16, 18, 20, 22, 24, 26, 28, 30, 32, 34, 36, 38, 40, 42, 44 oder 46 aufweist.

2. Polypeptid gemäß Anspruch 1, **dadurch gekennzeichnet, dass** die Aminosäuresequenz einer Sequenz gemäß SEQ ID NO: 2, 4, 6, 8, 10, 12, 14, 16, 18, 20, 22, 24, 26, 28, 30, 32, 34, 36, 38, 40, 42, 44 oder 46 entspricht.

3. Nukleinsäure umfassend eine Nukleotidsequenz, die für ein Polypeptid gemäß Anspruch 1 oder 2 codiert.

4. Nukleinsäure gemäß Anspruch 3, **dadurch gekennzeichnet, dass** es sich um einzelsträngige oder doppelsträngige DNA oder RNA handelt.

5. Nukleinsäure gemäß Anspruch 4, **dadurch gekennzeichnet, dass** es sich um Fragmente genomischer DNA oder cDNA handelt.

6. Nukleinsäure gemäß Anspruch 3, **dadurch gekennzeichnet, dass** die Nukle-otidsequenz einer Sequenz gemäß SEQ ID NO: 1, 3, 5, 7, 9, 11, 13, 15, 17, 19, 21, 23, 25, 27, 29, 31, 33, 35, 37, 39, 41, 43 oder 45 entspricht.

7. Nukleinsäure gemäß Anspruch 3, **dadurch gekennzeichnet, dass** sie unter stringenten Bedingungen an die Sequenzen gemäß SEQ ID NO: 1, 3, 5, 7, 9, 11, 13, 15, 17, 19, 21, 23, 25, 27, 29, 31, 33, 35, 37, 39, 41, 43 oder 45 hybridisiert.

8. DNA-Konstrukt umfassend eine Nukleinsäure gemäß einem der Ansprüche 3 bis 7 und einen heterologen Promotor.

9. Vektor umfassend eine Nukleinsäure gemäß einem der Ansprüche 3 bis 7 oder ein DNA-Konstrukt gemäß Anspruch 8.

10. Vektor gemäß nach Anspruch 9, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

11. Wirtszelle enthaltend eine Nukleinsäure gemäß einem der Ansprüche 3 bis 7, ein DNA-Konstrukt gemäß Anspruch 8 oder einen Vektor gemäß Anspruch 9 oder 10.

12. Wirtszelle gemäß Anspruch 11, **dadurch gekennzeichnet, dass** es sich um eine prokaryotische Zelle, insbesondere um E. coli, handelt.

13. Wirtszelle gemäß Anspruch 11, **dadurch gekennzeichnet, dass** es sich um eine eukaryotische Zelle, insbesondere um eine Säuger- oder Insektenzelle, handelt.

14. Antikörper, welcher spezifisch an ein Polypeptid gemäß Anspruch 1 oder 2 bindet.

15. Transgener Invertebrat enthaltend eine Nukleinsäure gemäß einem der Ansprüche 3 bis 7.

16. Transgener Invertebrat nach Anspruch 15, **dadurch gekennzeichnet, dass** es sich um Drosophila melanogaster oder Caenorhabditis elegans handelt.

17. Transgene Nachkommen eines Invertebraten gemäß Anspruch 15 oder 16.

18. Verfahren zum Herstellen eines Polypeptids gemäß Anspruch 1 oder 2, umfassend
(a) das Kultivieren einer Wirtszelle gemäß einem der Ansprüche 11 bis 13 unter Bedingungen, die die Expression der Nukleinsäure gemäß einem der Ansprüch 3 bis 7 gewährleisten, oder
(b) das Exprimieren einer Nukleinsäure gemäß einem der Ansprüche 3 bis 7 in einem *in vitro*-System, und
(c) die Gewinnung des Polypeptids aus der Zelle, dem Kulturmedium oder dem *in vitro*-System.

19. Verfahren zum Herstellen einer Nukleinsäure gemäß einem der Ansprüche 3 bis 7, umfassend die folgenden Schritte:
(a) Vollständige chemische Synthese auf an sich bekannte Weise, oder
(b) chemische Synthese von Oligonukleotiden, Markieren der Oligonukleotide, Hybridisieren der Oligonukleotide an DNA einer genomischen oder cDNA-Bank, die ausgehend von genomischer DNA bzw. mRNA aus Insektenzellen hergestellt wurde, Selektieren von positiven Klonen und Isolieren der hybridisierenden DNA aus positiven Klonen, oder
(c) chemische Synthese von Oligonukleotiden und Amplifizierung der Ziel-DNA mittels PCR.

20. Verfahren zum Herstellen eines transgenen Invertebraten gemäß Anspruch 15 oder 16, umfassend das Einbringen einer Nukleinsäure gemäß einem der Ansprüche 3 bis 7 oder eines Vektors gemäß Anspruch 9 oder 10.

21. Verfahren zum Auffinden neuer Wirkstoffe für den Pflanzenschutz, insbesondere von Verbindungen, welche die Eigenschaften von Polypeptiden gemäß Anspruch 1 oder 2 verändern, umfassend die folgenden Schritte:
(a) Bereitstellen einer Wirtszelle gemäß einem der Ansprüche 11 bis 13,
(b) Kultivieren der Wirtszelle in der Gegenwart einer chemischen Verbindung oder eines Gemischs von chemischen Verbindungen, und
(c) Detektieren veränderter Eigenschaften.

22. Verfahren zum Auffinden einer chemischen Verbindung, die an ein Polypeptid gemäß Anspruch 1 oder 2 bindet, umfassend die folgenden Schritte:
(a) Inkontaktbringen eines Polypeptids gemäß Anspruch 1 oder 2, oder einer Wirtszelle gemäß einem der Ansprüche 11 bis 13 mit einer chemischen Verbindung oder einem Gemisch von chemischen Verbindungen unter Bedingungen, die die Interaktion einer chemischen Verbindung mit dem Polypeptid erlauben, und
(b) Bestimmen der chemischen Verbindung, die spezifisch an das Polypeptid bindet.

23. Verfahren zum Auffinden einer chemischen Verbindung, die die Expression eines Polypeptids gemäß Anspruch 1 oder 2 verändert, umfassend die folgenden Schritte:
(a) Inkontaktbringen einer Wirtszelle gemäß einem der Ansprüche 11 bis 13 oder eines transgenen Invertebraten gemäß Anspruch 15 oder 16 mit einer chemischen Verbindung oder einem Gemisch von chemischen Verbindungen,
(b) Bestimmen der Konzentration des Polypeptids gemäß Anspruch 1 oder 2, und
(c) Bestimmen der chemischen Verbindung, die die Expression des Polypeptids spezifisch beeinflusst.

24. Verwendung eines Polypeptids gemäß Anspruch 1 oder 2, einer Nukleinsäure gemäß einem der Ansprüche 3 bis 7, eines Vektors gemäß Anspruch 9 oder 10, einer Wirtszelle gemäß einem der Ansprüche 11 bis 13, eines Antikörpers gemäß Anspruch 14 oder eines transgenen Invertebraten gemäß Anspruch 15 oder 16 zum Auffinden neuer Wirkstoffe für den Pflanzenschutz oder zum Auffinden von Genen, die für Polypeptide kodieren, welche am Aufbau von funktionell ähnlichen Peptid-Rezeptoren in Insekten beteiligt sind.

25. Verwendung eines Modulators eines Polypeptids gemäß Anspruch 1 oder 2 als Insektizid.


SEQUENZPROTOKOLL

<110> Bayer Aktiengesellschaft

<120> Rezeptoren für Peptide aus Insekten

<130> Le A 34 394

<140>
<141>

<150> DE 100 13 618.4
<151> 2000-03-18

<160> 92

<170> PatentIn Ver. 2.1

<210> 1
<211> 1623
<212> DNA
<213> Drosophila melanogaster

<220>
<221> CDS
<222> (1)..(1620)

<400> 1
atg gac tta atc gag cag gag tcc cgc ctg gaa ttc ctg ccc gga gcc 48
Met Asp Leu Ile Glu Gln Glu Ser Arg Leu Glu Phe Leu Pro Gly Ala
1               5                   10                  15

gag gag gaa gca gaa ttt gag cgt cta tac gcg gct ccc gct gag att 96
Glu Glu Glu Ala Glu Phe Glu Arg Leu Tyr Ala Ala Pro Ala Glu Ile
            20                  25                  30

gtg gcc ctg ttg tcc att ttc tat ggg gga atc agt atc gtg gcc gtc 144
Val Ala Leu Leu Ser Ile Phe Tyr Gly Gly Ile Ser Ile Val Ala Val
        35                  40                  45

att ggc aac act ttg gtc atc tgg gtg gtg gcc acg acc agg caa atg 192
Ile Gly Asn Thr Leu Val Ile Trp Val Val Ala Thr Thr Arg Gln Met
    50                  55                  60

cgg acc gtg aca aat atg tat atc gct aat ttg gct ttt gcc gat gtg 240
Arg Thr Val Thr Asn Met Tyr Ile Ala Asn Leu Ala Phe Ala Asp Val
65                  70                  75                  80

att att ggc ctc ttc tgc ata cca ttt cag ttc cag gct gcc ctg ctg 288
Ile Ile Gly Leu Phe Cys Ile Pro Phe Gln Phe Gln Ala Ala Leu Leu
                85                  90                  95

cag agt tgg aac ctg ccg tgg ttc atg tgc agc ttc tgc ccc ttc gtc 336
Gln Ser Trp Asn Leu Pro Trp Phe Met Cys Ser Phe Cys Pro Phe Val
            100                 105                 110

cag gcc ctg agt gta aat gtc tcg gta ttc acg ctg acc gcc att gca 384
Gln Ala Leu Ser Val Asn Val Ser Val Phe Thr Leu Thr Ala Ile Ala
        115                 120                 125

atc gat cgg cat agg gcc atc att aat cca ctt agg gca cgt ccc acc 432
Ile Asp Arg His Arg Ala Ile Ile Asn Pro Leu Arg Ala Arg Pro Thr
    130                 135                 140

aag ttc gta tcg aag ttc ata att ggt gga att tgg atg ctg gcc ctg 480
Lys Phe Val Ser Lys Phe Ile Ile Gly Gly Ile Trp Met Leu Ala Leu
145                 150                 155                 160

cta ttt gcg gtg ccc ttt gcc att gcc ttt cgt gtg gag gag ttg acc 528
Leu Phe Ala Val Pro Phe Ala Ile Ala Phe Arg Val Glu Glu Leu Thr
                165                 170                 175

gaa aga ttt cgc gag aac aat gag acc tac aat gtg acg cgg cca ttc 576
Glu Arg Phe Arg Glu Asn Asn Glu Thr Tyr Asn Val Thr Arg Pro Phe
            180                 185                 190

tgc atg aac aag aac cta tcc gat gat caa ttg caa tcc ttt cgc tac 624
Cys Met Asn Lys Asn Leu Ser Asp Asp Gln Leu Gln Ser Phe Arg Tyr
        195                 200                 205

acc ctg gtt ttt gtg cag tat ctg gtt cca ttc tgt gtc atc agc ttt 672
Thr Leu Val Phe Val Gln Tyr Leu Val Pro Phe Cys Val Ile Ser Phe
    210                 215                 220

gtc tac atc cag atg gcg gta cga ttg tgg ggc aca cgt gct cct ggt 720
Val Tyr Ile Gln Met Ala Val Arg Leu Trp Gly Thr Arg Ala Pro Gly
225                 230                 235                 240

aac gca cag gat tca cgg gac ata acg ctg ttg aaa aac aag aag aag 768
Asn Ala Gln Asp Ser Arg Asp Ile Thr Leu Leu Lys Asn Lys Lys Lys
                245                 250                 255

gtc atc aaa atg ctg att atc gtg gtc att atc ttt gga ctc tgc tgg 816
Val Ile Lys Met Leu Ile Ile Val Val Ile Ile Phe Gly Leu Cys Trp
            260                 265                 270

ctg cca ctg cag ctc tat aat att ctg tat gtc acg ata ccg gaa atc 864
Leu Pro Leu Gln Leu Tyr Asn Ile Leu Tyr Val Thr Ile Pro Glu Ile
        275                 280                 285

aac gac tac cac ttc att agc atc gtc tgg ttt tgc tgc gat tgg ctg 912
Asn Asp Tyr His Phe Ile Ser Ile Val Trp Phe Cys Cys Asp Trp Leu
    290                 295                 300

gcc atg agc aat agc tgc tac aat ccc ttt att tat ggc atc tac aat 960
Ala Met Ser Asn Ser Cys Tyr Asn Pro Phe Ile Tyr Gly Ile Tyr Asn
305                 310                 315                 320

gaa aaa ttt aag cgg gaa ttc aac aag cga ttt gcg gcc tgt ttc tgc 1008
Glu Lys Phe Lys Arg Glu Phe Asn Lys Arg Phe Ala Ala Cys Phe Cys
                325                 330                 335

aag ttc aag acg agc atg gac gcc cac gaa agg acc ttt tcg atg cac 1056
Lys Phe Lys Thr Ser Met Asp Ala His Glu Arg Thr Phe Ser Met His
            340                 345                 350

acc cgc gcc agc tcc ata agg tca acc tac gcc aac tcc tcg atg cga 1104
Thr Arg Ala Ser Ser Ile Arg Ser Thr Tyr Ala Asn Ser Ser Met Arg
        355                 360                 365

atc cgg agt aat ctc ttt ggt ccg gcg cgt ggt ggt gtc aac aat ggg 1152
Ile Arg Ser Asn Leu Phe Gly Pro Ala Arg Gly Gly Val Asn Asn Gly
    370                 375                 380

aag ccg ggc ttg cat atg ccg cgg gtg cat gga tcc ggt gct aac agc 1200
Lys Pro Gly Leu His Met Pro Arg Val His Gly Ser Gly Ala Asn Ser
385                 390                 395                 400

ggc att tac aac gga agt agt ggg cag aac aac aat gtc aat ggc caa 1248
Gly Ile Tyr Asn Gly Ser Ser Gly Gln Asn Asn Asn Val Asn Gly Gln
                405                 410                 415

cat cat cag cat caa agc gtg gtt acc ttt gcg gcc act ccg ggt gtt 1296
His His Gln His Gln Ser Val Val Thr Phe Ala Ala Thr Pro Gly Val
            420                 425                 430

tcg gca cca ggt gtt ggc gtt gca atg ccg ccg tgg cgg cga aac aac 1344
Ser Ala Pro Gly Val Gly Val Ala Met Pro Pro Trp Arg Arg Asn Asn
        435                 440                 445

ttc aaa cct ctg cat ccg aac gta atc gaa tgc gag gac gac gtg gca 1392
Phe Lys Pro Leu His Pro Asn Val Ile Glu Cys Glu Asp Asp Val Ala
    450                 455                 460

ctc atg gag ctg cca tca acc acg ccc ccc agc gag gag ttg gca tcc    1440
Leu Met Glu Leu Pro Ser Thr Thr Pro Pro Ser Glu Glu Leu Ala Ser
465                 470                 475                 480

ggg gcc gga gtc cag ttg gcc ctg cta agc agg gag agc tcc agc tgc    1488
Gly Ala Gly Val Gln Leu Ala Leu Leu Ser Arg Glu Ser Ser Ser Cys
                485                 490                 495

att tgc gaa cag gaa ttt ggc agc caa acc gaa tgc gat ggc acc tgc    1536
Ile Cys Glu Gln Glu Phe Gly Ser Gln Thr Glu Cys Asp Gly Thr Cys
            500                 505                 510

ata ctc agc gag gtg tcg cga gtc cac ctg ccc ggc tcg cag gcg aag    1584
Ile Leu Ser Glu Val Ser Arg Val His Leu Pro Gly Ser Gln Ala Lys
        515                 520                 525

gac aag gat gcg ggc aag tcc ttg tgg caa cca ctt taa                1623
Asp Lys Asp Ala Gly Lys Ser Leu Trp Gln Pro Leu
    530                 535                 540

<210> 2
<211> 540
<212> PRT
<213> Drosophila melanogaster

<400> 2
Met Asp Leu Ile Glu Gln Glu Ser Arg Leu Glu Phe Leu Pro Gly Ala
1               5                   10                  15

Glu Glu Glu Ala Glu Phe Glu Arg Leu Tyr Ala Ala Pro Ala Glu Ile
            20                  25                  30

Val Ala Leu Leu Ser Ile Phe Tyr Gly Gly Ile Ser Ile Val Ala Val
        35                  40                  45

Ile Gly Asn Thr Leu Val Ile Trp Val Val Ala Thr Thr Arg Gln Met
    50                  55                  60

Arg Thr Val Thr Asn Met Tyr Ile Ala Asn Leu Ala Phe Ala Asp Val
65                  70                  75                  80

Ile Ile Gly Leu Phe Cys Ile Pro Phe Gln Phe Gln Ala Ala Leu Leu
                85                  90                  95

Gln Ser Trp Asn Leu Pro Trp Phe Met Cys Ser Phe Cys Pro Phe Val
            100                 105                 110

Gln Ala Leu Ser Val Asn Val Ser Val Phe Thr Leu Thr Ala Ile Ala
        115                 120                 125

Ile Asp Arg His Arg Ala Ile Ile Asn Pro Leu Arg Ala Arg Pro Thr
    130                 135                 140

Lys Phe Val Ser Lys Phe Ile Ile Gly Gly Ile Trp Met Leu Ala Leu
145                 150                 155                 160

Leu Phe Ala Val Pro Phe Ala Ile Ala Phe Arg Val Glu Glu Leu Thr
                165                 170                 175

Glu Arg Phe Arg Glu Asn Asn Glu Thr Tyr Asn Val Thr Arg Pro Phe
            180                 185                 190

Cys Met Asn Lys Asn Leu Ser Asp Asp Gln Leu Gln Ser Phe Arg Tyr
        195                 200                 205

Thr Leu Val Phe Val Gln Tyr Leu Val Pro Phe Cys Val Ile Ser Phe
    210                 215                 220

Val Tyr Ile Gln Met Ala Val Arg Leu Trp Gly Thr Arg Ala Pro Gly
225                 230                 235                 240

Asn Ala Gln Asp Ser Arg Asp Ile Thr Leu Leu Lys Asn Lys Lys Lys
                245                 250                 255

Val Ile Lys Met Leu Ile Ile Val Val Ile Ile Phe Gly Leu Cys Trp
            260                 265                 270

Leu Pro Leu Gln Leu Tyr Asn Ile Leu Tyr Val Thr Ile Pro Glu Ile
        275                 280                 285

Asn Asp Tyr His Phe Ile Ser Ile Val Trp Phe Cys Cys Asp Trp Leu
    290                 295                 300

Ala Met Ser Asn Ser Cys Tyr Asn Pro Phe Ile Tyr Gly Ile Tyr Asn
305                 310                 315                 320

Glu Lys Phe Lys Arg Glu Phe Asn Lys Arg Phe Ala Ala Cys Phe Cys
                325                 330                 335

Lys Phe Lys Thr Ser Met Asp Ala His Glu Arg Thr Phe Ser Met His
            340                 345                 350

Thr Arg Ala Ser Ser Ile Arg Ser Thr Tyr Ala Asn Ser Ser Met Arg
        355                 360                 365

Ile Arg Ser Asn Leu Phe Gly Pro Ala Arg Gly Gly Val Asn Asn Gly
    370                 375                 380

Lys Pro Gly Leu His Met Pro Arg Val His Gly Ser Gly Ala Asn Ser
385                 390                 395                 400

Gly Ile Tyr Asn Gly Ser Ser Gly Gln Asn Asn Asn Val Asn Gly Gln
                405                 410                 415

His His Gln His Gln Ser Val Val Thr Phe Ala Ala Thr Pro Gly Val
            420                 425                 430

Ser Ala Pro Gly Val Gly Val Ala Met Pro Pro Trp Arg Arg Asn Asn
        435                 440                 445

Phe Lys Pro Leu His Pro Asn Val Ile Glu Cys Glu Asp Asp Val Ala
    450                 455                 460

Leu Met Glu Leu Pro Ser Thr Thr Pro Pro Ser Glu Glu Leu Ala Ser
465                 470                 475                 480

Gly Ala Gly Val Gln Leu Ala Leu Leu Ser Arg Glu Ser Ser Ser Cys
                485                 490                 495

Ile Cys Glu Gln Glu Phe Gly Ser Gln Thr Glu Cys Asp Gly Thr Cys
            500                 505                 510

Ile Leu Ser Glu Val Ser Arg Val His Leu Pro Gly Ser Gln Ala Lys
        515                 520                 525

Asp Lys Asp Ala Gly Lys Ser Leu Trp Gln Pro Leu
    530                 535                 540

<210> 3
<211> 1050
<212> DNA
<213> Drosophila melanogaster

<220>
<221> CDS
<222> (1)..(1047)

<400> 3
atg ggt ttt ggg cta ggt cag ttg cgt tta cag agg gga aac acg aat     48
Met Gly Phe Gly Leu Gly Gln Leu Arg Leu Gln Arg Gly Asn Thr Asn
  1               5                  10                  15

tat act gca gta aat gct caa cga agt tta cct tgc caa tca ttg tat    96
Tyr Thr Ala Val Asn Ala Gln Arg Ser Leu Pro Cys Gln Ser Leu Tyr
            20                  25                  30

att ctc aat tgt tat ttc tta cag att tta caa tgt ccc agc tcg ttc    144
Ile Leu Asn Cys Tyr Phe Leu Gln Ile Leu Gln Cys Pro Ser Ser Phe
        35                  40                  45

gat tcg gtc ctg tgc tgg cca cgc aca aac gcc ggc agt ctg gct gta    192
Asp Ser Val Leu Cys Trp Pro Arg Thr Asn Ala Gly Ser Leu Ala Val
    50                  55                  60

cta ccc tgt ttc gag gaa ttc aag ggc gtg cac tac gac acc aca gac    240
Leu Pro Cys Phe Glu Glu Phe Lys Gly Val His Tyr Asp Thr Thr Asp
65                  70                  75                  80

aat gcc acc cgc ttt tgc ttt cca aac gga acg tgg gat cac tat tcg    288
Asn Ala Thr Arg Phe Cys Phe Pro Asn Gly Thr Trp Asp His Tyr Ser
                85                  90                  95

gac tat gac cgc tgt cac cag aac tcg ggc tcc ata ccg gtg gtg ccc    336
Asp Tyr Asp Arg Cys His Gln Asn Ser Gly Ser Ile Pro Val Val Pro
            100                 105                 110

gac ttc tca ccc aac gtc gaa ctg ccg gcc atc ata tat gcc ggc ggt    384
Asp Phe Ser Pro Asn Val Glu Leu Pro Ala Ile Ile Tyr Ala Gly Gly
        115                 120                 125

tat ttc ctg agc ttc gcc acc ttg gtg gtg gct ctc atc ata ttc ctc    432
Tyr Phe Leu Ser Phe Ala Thr Leu Val Val Ala Leu Ile Ile Phe Leu
    130                 135                 140

agc ttt aaa gat ctt cgt tgc ctg cga aac acc att cat gcc aat ttg    480
Ser Phe Lys Asp Leu Arg Cys Leu Arg Asn Thr Ile His Ala Asn Leu
145                 150                 155                 160

ttc ctc acc tac atc aca tcc gca ctc ctc tgg ata ctc aca ctg ttc    528
Phe Leu Thr Tyr Ile Thr Ser Ala Leu Leu Trp Ile Leu Thr Leu Phe
                165                 170                 175

ctg caa gtg tac ttt tac cta acc aac ttt ttc tgg atg ttt gtg gag    576
Leu Gln Val Tyr Phe Tyr Leu Thr Asn Phe Phe Trp Met Phe Val Glu
            180                 185                 190

ggc ctc tat ctg tac acg ctg gtg gtg caa aca ttc tcc agt gat aac    624
Gly Leu Tyr Leu Tyr Thr Leu Val Val Gln Thr Phe Ser Ser Asp Asn
        195                 200                 205

att agc ttt att atc tac gcc ctc atc ggc tgg ggc tgt cca gcc gta    672
Ile Ser Phe Ile Ile Tyr Ala Leu Ile Gly Trp Gly Cys Pro Ala Val
    210                 215                 220

tgc att ttg gtg tgg tcc att gcc aag gca ttt gct ccg cat ctc gag    720
Cys Ile Leu Val Trp Ser Ile Ala Lys Ala Phe Ala Pro His Leu Glu
225                 230                 235                 240

aac gag cac ttc aat ggg gta ctc atc act aaa tta cgt tct gct cat    768
Asn Glu His Phe Asn Gly Val Leu Ile Thr Lys Leu Arg Ser Ala His
                245                 250                 255

acc ctg gaa acg cgg cag tat tac aag gcc tcg aag gcg ctg ctg gtg    816
Thr Leu Glu Thr Arg Gln Tyr Tyr Lys Ala Ser Lys Ala Leu Leu Val
            260                 265                 270

ctg ata cct ctc ttt ggc atc acc tat ctg ttg gtg cta aca ggc ccg    864
Leu Ile Pro Leu Phe Gly Ile Thr Tyr Leu Leu Val Leu Thr Gly Pro
        275                 280                 285

gaa cag ggt atc agt cgt aat ctc ttc gag gcc ata aga gcc ttt ctc    912
Glu Gln Gly Ile Ser Arg Asn Leu Phe Glu Ala Ile Arg Ala Phe Leu
    290                 295                 300

ata agc acg cag ggc ttc ttt gtg gct ctg ttc tac tgt ttc ctt aac    960
Ile Ser Thr Gln Gly Phe Phe Val Ala Leu Phe Tyr Cys Phe Leu Asn
305                 310                 315                 320

tca gag gtg cgc cag acg ctg agg cat gga ttc acc cgg tgg cgg gag    1008
Ser Glu Val Arg Gln Thr Leu Arg His Gly Phe Thr Arg Trp Arg Glu
                325                 330                 335

agc agg aat atc cat cgg aac agt tcc atc aag aat cgc agn            1050
Ser Arg Asn Ile His Arg Asn Ser Ser Ile Lys Asn Arg
            340                 345

<210> 4
<211> 349
<212> PRT
<213> Drosophila melanogaster

<400> 4
Met Gly Phe Gly Leu Gly Gln Leu Arg Leu Gln Arg Gly Asn Thr Asn
  1               5                  10                  15

Tyr Thr Ala Val Asn Ala Gln Arg Ser Leu Pro Cys Gln Ser Leu Tyr

            20                  25                  30

Ile Leu Asn Cys Tyr Phe Leu Gln Ile Leu Gln Cys Pro Ser Ser Phe
        35                  40                  45

Asp Ser Val Leu Cys Trp Pro Arg Thr Asn Ala Gly Ser Leu Ala Val
    50                  55                  60

Leu Pro Cys Phe Glu Glu Phe Lys Gly Val His Tyr Asp Thr Thr Asp
65                  70                  75                  80

Asn Ala Thr Arg Phe Cys Phe Pro Asn Gly Thr Trp Asp His Tyr Ser
                85                  90                  95

Asp Tyr Asp Arg Cys His Gln Asn Ser Gly Ser Ile Pro Val Val Pro
            100                 105                 110

Asp Phe Ser Pro Asn Val Glu Leu Pro Ala Ile Ile Tyr Ala Gly Gly
        115                 120                 125

Tyr Phe Leu Ser Phe Ala Thr Leu Val Val Ala Leu Ile Ile Phe Leu
    130                 135                 140

Ser Phe Lys Asp Leu Arg Cys Leu Arg Asn Thr Ile His Ala Asn Leu
145                 150                 155                 160

Phe Leu Thr Tyr Ile Thr Ser Ala Leu Leu Trp Ile Leu Thr Leu Phe
                165                 170                 175

Leu Gln Val Tyr Phe Tyr Leu Thr Asn Phe Phe Trp Met Phe Val Glu
            180                 185                 190

Gly Leu Tyr Leu Tyr Thr Leu Val Val Gln Thr Phe Ser Ser Asp Asn
        195                 200                 205

Ile Ser Phe Ile Ile Tyr Ala Leu Ile Gly Trp Gly Cys Pro Ala Val
    210                 215                 220

Cys Ile Leu Val Trp Ser Ile Ala Lys Ala Phe Ala Pro His Leu Glu
225                 230                 235                 240

Asn Glu His Phe Asn Gly Val Leu Ile Thr Lys Leu Arg Ser Ala His
                245                 250                 255

Thr Leu Glu Thr Arg Gln Tyr Tyr Lys Ala Ser Lys Ala Leu Leu Val
            260                 265                 270

Leu Ile Pro Leu Phe Gly Ile Thr Tyr Leu Leu Val Leu Thr Gly Pro

275                 280                 285

Glu Gln Gly Ile Ser Arg Asn Leu Phe Glu Ala Ile Arg Ala Phe Leu
    290                 295                 300

Ile Ser Thr Gln Gly Phe Phe Val Ala Leu Phe Tyr Cys Phe Leu Asn
305                 310                 315                 320

Ser Glu Val Arg Gln Thr Leu Arg His Gly Phe Thr Arg Trp Arg Glu
                325                 330                 335

Ser Arg Asn Ile His Arg Asn Ser Ser Ile Lys Asn Arg
            340                 345

<210> 5
<211> 1113
<212> DNA
<213> Drosophila melanogaster

<220>
<221> CDS
<222> (1)..(1110)

<400> 5
atg agt gac cac aac cac atc gat tcg gtg aac gcc agc ggg agt gat      48
Met Ser Asp His Asn His Ile Asp Ser Val Asn Ala Ser Gly Ser Asp
1               5                   10                  15

ccg ctc ctg gat ctg cac aac cta gat ggc atc ggt gag agc gtg gag      96
Pro Leu Leu Asp Leu His Asn Leu Asp Gly Ile Gly Glu Ser Val Glu
            20                  25                  30

ttg cag tgc ctg gtg cag gag cac atc gag gca tcc acc tat ggc aac      144
Leu Gln Cys Leu Val Gln Glu His Ile Glu Ala Ser Thr Tyr Gly Asn
        35                  40                  45

gat agt ggc cac tgc ctc acc cag ttc gac tcg atc ctg tgc tgg ccg      192
Asp Ser Gly His Cys Leu Thr Gln Phe Asp Ser Ile Leu Cys Trp Pro
    50                  55                  60

agg aca gct cgc ggt acc ctg gcg gtg ctc cag tgc atg gac gag ttg      240
Arg Thr Ala Arg Gly Thr Leu Ala Val Leu Gln Cys Met Asp Glu Leu
65                  70                  75                  80

cag gga att cat tac gat agc agc aag aat gca acg agg ttc tgc cac      288
Gln Gly Ile His Tyr Asp Ser Ser Lys Asn Ala Thr Arg Phe Cys His

85                  90                  95

gcc aat gga act tgg gag aag tac acc aac tac gat gcc tgc gcc cac     336
Ala Asn Gly Thr Trp Glu Lys Tyr Thr Asn Tyr Asp Ala Cys Ala His
            100                 105                 110

ctg ccc gcc cca gaa tcc gtg ccg gag ttc gag gtc atc gtg gag ctg     384
Leu Pro Ala Pro Glu Ser Val Pro Glu Phe Glu Val Ile Val Glu Leu
        115                 120                 125

ccc acc att atc tac tac att gga tac acc ctc agc ctg gta tcg ctc     432
Pro Thr Ile Ile Tyr Tyr Ile Gly Tyr Thr Leu Ser Leu Val Ser Leu
    130                 135                 140

tcg ctg gcg ctg att gtt ttc gcc tac ttc aag gag ctg cgt tgc ctg     480
Ser Leu Ala Leu Ile Val Phe Ala Tyr Phe Lys Glu Leu Arg Cys Leu
145                 150                 155                 160

cgc aac acc atc cac gcc aac ttg ttc ttc acg tac atc atg tcg gct     528
Arg Asn Thr Ile His Ala Asn Leu Phe Phe Thr Tyr Ile Met Ser Ala
                165                 170                 175

ttg ttc tgg ata ctc ttg tta tcc gtc cag atc tcc atc cga agt gga     576
Leu Phe Trp Ile Leu Leu Leu Ser Val Gln Ile Ser Ile Arg Ser Gly
            180                 185                 190

gtg ggc agc tgc att gct ctg atc acc ctg ttc cac ttc ttc acc ctg     624
Val Gly Ser Cys Ile Ala Leu Ile Thr Leu Phe His Phe Phe Thr Leu
        195                 200                 205

acc aac ttc ttt tgg atg ctg gtc gag ggt ttg tac ctg tac atg ctg     672
Thr Asn Phe Phe Trp Met Leu Val Glu Gly Leu Tyr Leu Tyr Met Leu
    210                 215                 220

gtg gtt aag act ttc tcc ggg gac aat tta cgc ttt aat atc tac gcc     720
Val Val Lys Thr Phe Ser Gly Asp Asn Leu Arg Phe Asn Ile Tyr Ala
225                 230                 235                 240

tcc ata ggc tgg ggt ggc ccg gcc ttg ttt gtt gtc acc tgg gct gtg     768
Ser Ile Gly Trp Gly Gly Pro Ala Leu Phe Val Val Thr Trp Ala Val
                245                 250                 255

gcc aag agt ctg acg gtc acc tac agc acc ccc gaa aag gtt cta atc     816
Ala Lys Ser Leu Thr Val Thr Tyr Ser Thr Pro Glu Lys Val Leu Ile
            260                 265                 270

aca aag ctg cgc tcg gcc aat aca gtg gag act cgt cag tat agg aaa     864
Thr Lys Leu Arg Ser Ala Asn Thr Val Glu Thr Arg Gln Tyr Arg Lys

275                 280                 285

gct gct aag gca ctg ctg gtg cta atc cca ctt ttt ggc atc acc tac     912
Ala Ala Lys Ala Leu Leu Val Leu Ile Pro Leu Phe Gly Ile Thr Tyr
    290                 295                 300

ctg gtg gtg ctg gct ggt ccc tcg gaa tct ggc cta atg gga cac atg     960
Leu Val Val Leu Ala Gly Pro Ser Glu Ser Gly Leu Met Gly His Met
305                 310                 315                 320

ttt gcc gtc ctg cga gcg gta tta ctc agc act cag gga ttc tcg gtg    1008
Phe Ala Val Leu Arg Ala Val Leu Leu Ser Thr Gln Gly Phe Ser Val
                325                 330                 335

tcg ctg ttc tac tgt ttc ctc aac tcg gag gtg cga aat gca ctg aga    1056
Ser Leu Phe Tyr Cys Phe Leu Asn Ser Glu Val Arg Asn Ala Leu Arg
            340                 345                 350

cac cac att tcc acg tgg cgg gac acg cgc acc att cag ctt aac cag    1104
His His Ile Ser Thr Trp Arg Asp Thr Arg Thr Ile Gln Leu Asn Gln
        355                 360                 365

aac cga cgn                                                        1113
Asn Arg
    370

<210> 6
<211> 370
<212> PRT
<213> Drosophila melanogaster

<400> 6
Met Ser Asp His Asn His Ile Asp Ser Val Asn Ala Ser Gly Ser Asp
  1               5                  10                  15

Pro Leu Leu Asp Leu His Asn Leu Asp Gly Ile Gly Glu Ser Val Glu
             20                  25                  30

Leu Gln Cys Leu Val Gln Glu His Ile Glu Ala Ser Thr Tyr Gly Asn
         35                  40                  45

Asp Ser Gly His Cys Leu Thr Gln Phe Asp Ser Ile Leu Cys Trp Pro
     50                  55                  60

Arg Thr Ala Arg Gly Thr Leu Ala Val Leu Gln Cys Met Asp Glu Leu
 65                  70                  75                  80

Gln Gly Ile His Tyr Asp Ser Ser Lys Asn Ala Thr Arg Phe Cys His
                85                  90                  95

Ala Asn Gly Thr Trp Glu Lys Tyr Thr Asn Tyr Asp Ala Cys Ala His
            100                 105                 110

Leu Pro Ala Pro Glu Ser Val Pro Glu Phe Glu Val Ile Val Glu Leu
        115                 120                 125

Pro Thr Ile Ile Tyr Tyr Ile Gly Tyr Thr Leu Ser Leu Val Ser Leu
    130                 135                 140

Ser Leu Ala Leu Ile Val Phe Ala Tyr Phe Lys Glu Leu Arg Cys Leu
145                 150                 155                 160

Arg Asn Thr Ile His Ala Asn Leu Phe Phe Thr Tyr Ile Met Ser Ala
                165                 170                 175

Leu Phe Trp Ile Leu Leu Leu Ser Val Gln Ile Ser Ile Arg Ser Gly
            180                 185                 190

Val Gly Ser Cys Ile Ala Leu Ile Thr Leu Phe His Phe Phe Thr Leu
        195                 200                 205

Thr Asn Phe Phe Trp Met Leu Val Glu Gly Leu Tyr Leu Tyr Met Leu
    210                 215                 220

Val Val Lys Thr Phe Ser Gly Asp Asn Leu Arg Phe Asn Ile Tyr Ala
225                 230                 235                 240

Ser Ile Gly Trp Gly Gly Pro Ala Leu Phe Val Val Thr Trp Ala Val
                245                 250                 255

Ala Lys Ser Leu Thr Val Thr Tyr Ser Thr Pro Glu Lys Val Leu Ile
            260                 265                 270

Thr Lys Leu Arg Ser Ala Asn Thr Val Glu Thr Arg Gln Tyr Arg Lys
        275                 280                 285

Ala Ala Lys Ala Leu Leu Val Leu Ile Pro Leu Phe Gly Ile Thr Tyr
    290                 295                 300

Leu Val Val Leu Ala Gly Pro Ser Glu Ser Gly Leu Met Gly His Met
305                 310                 315                 320

Phe Ala Val Leu Arg Ala Val Leu Leu Ser Thr Gln Gly Phe Ser Val
                325                 330                 335

Ser Leu Phe Tyr Cys Phe Leu Asn Ser Glu Val Arg Asn Ala Leu Arg
            340                 345                 350

His His Ile Ser Thr Trp Arg Asp Thr Arg Thr Ile Gln Leu Asn Gln
        355                 360                 365

Asn Arg
    370

<210> 7
<211> 1557
<212> DNA
<213> Drosophila melanogaster

<220>
<221> CDS
<222> (1)..(1554)

<400> 7
atg gcc aac tta agc tgg ctg agc acc atc acc acc acc tcc tcc tcc      48
Met Ala Asn Leu Ser Trp Leu Ser Thr Ile Thr Thr Thr Ser Ser Ser
  1               5                  10                  15

atc agc acc agc cag ctg cca ttg gtc agc aca acc aac tgg agc cta      96
Ile Ser Thr Ser Gln Leu Pro Leu Val Ser Thr Thr Asn Trp Ser Leu
            20                  25                  30

acg tcg ccg gga act act agc gct atc ttg gcg gat gtg gct gca tcg     144
Thr Ser Pro Gly Thr Thr Ser Ala Ile Leu Ala Asp Val Ala Ala Ser
        35                  40                  45

gat gag gat agg agc ggc ggg atc att cac aac cag ttc gtg caa atc     192
Asp Glu Asp Arg Ser Gly Gly Ile Ile His Asn Gln Phe Val Gln Ile
    50                  55                  60

ttc ttc tac gtc ctg tac gcc acg gtc ttt gtc ctg ggt gtc ttc gga     240
Phe Phe Tyr Val Leu Tyr Ala Thr Val Phe Val Leu Gly Val Phe Gly
 65                  70                  75                  80

aat gtc ctg gtt tgc tac gta gtt ctg agg aat cgg gcc atg cag act     288
Asn Val Leu Val Cys Tyr Val Val Leu Arg Asn Arg Ala Met Gln Thr
                 85                  90                  95

gtg acc aat ata ttc atc acg aat ctg gcc ctg tcg gac ata ttg ctc     336
Val Thr Asn Ile Phe Ile Thr Asn Leu Ala Leu Ser Asp Ile Leu Leu
            100                 105                 110

tgc gtc ctg gcg gtg cca ttt act ccg ctt tac acg ttc atg ggt cgc    384
Cys Val Leu Ala Val Pro Phe Thr Pro Leu Tyr Thr Phe Met Gly Arg
        115                 120                 125

tgg gcc ttc ggc agg agt ctg tgc cat ctg gtg tcc ttt gcc cag gga    432
Trp Ala Phe Gly Arg Ser Leu Cys His Leu Val Ser Phe Ala Gln Gly
    130                 135                 140

tgc agc atc tac ata tcc acg ctg acc ctc acc tcg att gcc atc gat    480
Cys Ser Ile Tyr Ile Ser Thr Leu Thr Leu Thr Ser Ile Ala Ile Asp
145                 150                 155                 160

cgg tac ttc gtt atc ata tac ccc ttc cat ccg cgc atg aag ctc tcc    528
Arg Tyr Phe Val Ile Ile Tyr Pro Phe His Pro Arg Met Lys Leu Ser
                165                 170                 175

acc tgc atc ggg atc ata gtg agc atc tgg gtg ata gcc ctg ctg gcc    576
Thr Cys Ile Gly Ile Ile Val Ser Ile Trp Val Ile Ala Leu Leu Ala
            180                 185                 190

acc gtt ccc tac ggc atg tac atg aag atg acc aac gag ctg gtg aac    624
Thr Val Pro Tyr Gly Met Tyr Met Lys Met Thr Asn Glu Leu Val Asn
        195                 200                 205

gga acg cag aca ggc aac gag acc ctg gtg gag gcc act cta atg cta    672
Gly Thr Gln Thr Gly Asn Glu Thr Leu Val Glu Ala Thr Leu Met Leu
    210                 215                 220

aac gga agc ttt gtg gcc cag gga tca gga ttc atc gag gcg ccg gac    720
Asn Gly Ser Phe Val Ala Gln Gly Ser Gly Phe Ile Glu Ala Pro Asp
225                 230                 235                 240

tct acc tcg gcc acc cag gcc tat atg cag gtg atg acc gcc gga tca    768
Ser Thr Ser Ala Thr Gln Ala Tyr Met Gln Val Met Thr Ala Gly Ser
                245                 250                 255

acg gga ccg gag atg ccc tat gtg cgg gtg tac tgc gag gag aac tgg    816
Thr Gly Pro Glu Met Pro Tyr Val Arg Val Tyr Cys Glu Glu Asn Trp
            260                 265                 270

cca tcg gag cag tac cgg aag gtg ttc ggt gcc atc aca acc act ctg    864
Pro Ser Glu Gln Tyr Arg Lys Val Phe Gly Ala Ile Thr Thr Thr Leu
        275                 280                 285

cag ttt gtg ctg ccc ttc ttc atc atc tcg att tgc tac gtg tgg ata    912
Gln Phe Val Leu Pro Phe Phe Ile Ile Ser Ile Cys Tyr Val Trp Ile
    290                 295                 300

tcg gtg aag cta aac cag cgg gcc agg gcc aag ccg gga tcg aaa tcc 960
Ser Val Lys Leu Asn Gln Arg Ala Arg Ala Lys Pro Gly Ser Lys Ser
305                 310                 315                 320

tcg aga cgg gag gag gcg gat cgg gat cgc aag aag cgc acc aac cgc 1008
Ser Arg Arg Glu Glu Ala Asp Arg Asp Arg Lys Lys Arg Thr Asn Arg
                325                 330                 335

atg ctc atc gcc atg gtg gcg gta ttc gga ctc agc tgg ctg ccc atc 1056
Met Leu Ile Ala Met Val Ala Val Phe Gly Leu Ser Trp Leu Pro Ile
            340                 345                 350

aat gtg gtc aac ata ttc gat gac ttc gat gac aag tcc aac gag tgg 1104
Asn Val Val Asn Ile Phe Asp Asp Phe Asp Asp Lys Ser Asn Glu Trp
        355                 360                 365

cgc ttc tac atc cta ttc ttc ttt gtg gcc cac tct att gcc atg agc 1152
Arg Phe Tyr Ile Leu Phe Phe Phe Val Ala His Ser Ile Ala Met Ser
    370                 375                 380

tcc acc tgc tac aat ccc ttc ctg tac gcc tgg ctg aac gag aac ttc 1200
Ser Thr Cys Tyr Asn Pro Phe Leu Tyr Ala Trp Leu Asn Glu Asn Phe
385                 390                 395                 400

cgc aag gag ttc aag cac gtg ctg ccc tgc ttt aat ccc tcg aac aac 1248
Arg Lys Glu Phe Lys His Val Leu Pro Cys Phe Asn Pro Ser Asn Asn
                405                 410                 415

aac atc atc aac atc acc agg ggc tat aat cgg agt gat cgg aac acc 1296
Asn Ile Ile Asn Ile Thr Arg Gly Tyr Asn Arg Ser Asp Arg Asn Thr
            420                 425                 430

tgt ggt ccg cga ctg cat cat ggc aag ggg gat ggt ggc atg ggc ggt 1344
Cys Gly Pro Arg Leu His His Gly Lys Gly Asp Gly Gly Met Gly Gly
        435                 440                 445

ggc agt ctg gac gcc gac gac cag gac gag aac ggc atc acc cag gag 1392
Gly Ser Leu Asp Ala Asp Asp Gln Asp Glu Asn Gly Ile Thr Gln Glu
    450                 455                 460

acc tgt ctg ccc aag gag aag ctg ctg att atc ccc agg gag ccg act 1440
Thr Cys Leu Pro Lys Glu Lys Leu Leu Ile Ile Pro Arg Glu Pro Thr
465                 470                 475                 480

tac ggc aat ggc acg ggt gcc gtg tcg cca atc ctt agc ggg cgc ggc 1488
Tyr Gly Asn Gly Thr Gly Ala Val Ser Pro Ile Leu Ser Gly Arg Gly
                485                 490                 495

att aac gcc gcc ctg gtg cac ggt ggc gac cat cag atg cac cag ctg 1536
Ile Asn Ala Ala Leu Val His Gly Gly Asp His Gln Met His Gln Leu
            500                 505                 510

cag ccg tca cac cat caa cag 1557
Gln Pro Ser His His Gln
        515

<210> 8
<211> 518
<212> PRT
<213> Drosophila melanogaster

<400> 8
Met Ala Asn Leu Ser Trp Leu Ser Thr Ile Thr Thr Thr Ser Ser Ser
1               5                   10                  15

Ile Ser Thr Ser Gln Leu Pro Leu Val Ser Thr Thr Asn Trp Ser Leu
            20                  25                  30

Thr Ser Pro Gly Thr Thr Ser Ala Ile Leu Ala Asp Val Ala Ala Ser
        35                  40                  45

Asp Glu Asp Arg Ser Gly Gly Ile Ile His Asn Gln Phe Val Gln Ile
    50                  55                  60

Phe Phe Tyr Val Leu Tyr Ala Thr Val Phe Val Leu Gly Val Phe Gly
65                  70                  75                  80

Asn Val Leu Val Cys Tyr Val Val Leu Arg Asn Arg Ala Met Gln Thr
                85                  90                  95

Val Thr Asn Ile Phe Ile Thr Asn Leu Ala Leu Ser Asp Ile Leu Leu
            100                 105                 110

Cys Val Leu Ala Val Pro Phe Thr Pro Leu Tyr Thr Phe Met Gly Arg
        115                 120                 125

Trp Ala Phe Gly Arg Ser Leu Cys His Leu Val Ser Phe Ala Gln Gly
    130                 135                 140

Cys Ser Ile Tyr Ile Ser Thr Leu Thr Leu Thr Ser Ile Ala Ile Asp
145                 150                 155                 160

Arg Tyr Phe Val Ile Ile Tyr Pro Phe His Pro Arg Met Lys Leu Ser
                165                 170                 175

Thr Cys Ile Gly Ile Ile Val Ser Ile Trp Val Ile Ala Leu Leu Ala
            180                 185                 190

Thr Val Pro Tyr Gly Met Tyr Met Lys Met Thr Asn Glu Leu Val Asn
        195                 200                 205

Gly Thr Gln Thr Gly Asn Glu Thr Leu Val Glu Ala Thr Leu Met Leu
    210                 215                 220

Asn Gly Ser Phe Val Ala Gln Gly Ser Gly Phe Ile Glu Ala Pro Asp
225                 230                 235                 240

Ser Thr Ser Ala Thr Gln Ala Tyr Met Gln Val Met Thr Ala Gly Ser
                245                 250                 255

Thr Gly Pro Glu Met Pro Tyr Val Arg Val Tyr Cys Glu Glu Asn Trp
            260                 265                 270

Pro Ser Glu Gln Tyr Arg Lys Val Phe Gly Ala Ile Thr Thr Thr Leu
        275                 280                 285

Gln Phe Val Leu Pro Phe Phe Ile Ile Ser Ile Cys Tyr Val Trp Ile
    290                 295                 300

Ser Val Lys Leu Asn Gln Arg Ala Arg Ala Lys Pro Gly Ser Lys Ser
305                 310                 315                 320

Ser Arg Arg Glu Glu Ala Asp Arg Asp Arg Lys Lys Arg Thr Asn Arg
                325                 330                 335

Met Leu Ile Ala Met Val Ala Val Phe Gly Leu Ser Trp Leu Pro Ile
            340                 345                 350

Asn Val Val Asn Ile Phe Asp Asp Phe Asp Asp Lys Ser Asn Glu Trp
        355                 360                 365

Arg Phe Tyr Ile Leu Phe Phe Phe Val Ala His Ser Ile Ala Met Ser
    370                 375                 380

Ser Thr Cys Tyr Asn Pro Phe Leu Tyr Ala Trp Leu Asn Glu Asn Phe
385                 390                 395                 400

Arg Lys Glu Phe Lys His Val Leu Pro Cys Phe Asn Pro Ser Asn Asn
                405                 410                 415

Asn Ile Ile Asn Ile Thr Arg Gly Tyr Asn Arg Ser Asp Arg Asn Thr
            420                 425                 430

Cys Gly Pro Arg Leu His His Gly Lys Gly Asp Gly Gly Met Gly Gly
        435                 440                 445

Gly Ser Leu Asp Ala Asp Asp Gln Asp Glu Asn Gly Ile Thr Gln Glu
    450                 455                 460

Thr Cys Leu Pro Lys Glu Lys Leu Leu Ile Ile Pro Arg Glu Pro Thr
465                 470                 475                 480

Tyr Gly Asn Gly Thr Gly Ala Val Ser Pro Ile Leu Ser Gly Arg Gly
                485                 490                 495

Ile Asn Ala Ala Leu Val His Gly Gly Asp His Gln Met His Gln Leu
            500                 505                 510

Gln Pro Ser His His Gln
        515

<210> 9
<211> 1404
<212> DNA
<213> Drosophila melanogaster

<220>
<221> CDS
<222> (1)..(1401)

<400> 9
atg ttt acg tgg ctg atg atg gat gtc ctc cag ttt gtg aaa ggg gaa    48
Met Phe Thr Trp Leu Met Met Asp Val Leu Gln Phe Val Lys Gly Glu
  1               5                  10                  15

atg aca gcc gat tca gag gca aat gcc aca aat tgg tat aac acg aac    96
Met Thr Ala Asp Ser Glu Ala Asn Ala Thr Asn Trp Tyr Asn Thr Asn
             20                  25                  30

gag agc tta tat acc acg gaa ctg aac cat aga tgg att agt ggt agt   144
Glu Ser Leu Tyr Thr Thr Glu Leu Asn His Arg Trp Ile Ser Gly Ser
         35                  40                  45

tcc aca att cag cca gag gag tcc ctt tat ggc act gat ttg ccc acc   192
Ser Thr Ile Gln Pro Glu Glu Ser Leu Tyr Gly Thr Asp Leu Pro Thr
     50                  55                  60

tat caa cat tgc ata gcc acg cgg aat tcc ttt gct gac ttg ttc act   240

Tyr Gln His Cys Ile Ala Thr Arg Asn Ser Phe Ala Asp Leu Phe Thr
65                  70                  75                  80

gtg gtg ctc tac gga ttt gtg tgc att atc gga tta ttt ggc aac acc     288
Val Val Leu Tyr Gly Phe Val Cys Ile Ile Gly Leu Phe Gly Asn Thr
                85                  90                  95

ctg gtg atc tac gtg gtg ttg cgc ttt tcc aaa atg caa acg gtc acg     336
Leu Val Ile Tyr Val Val Leu Arg Phe Ser Lys Met Gln Thr Val Thr
            100                 105                 110

aat ata tat atc ctg aat ctg gcg gtg gca gac gag tgc ttc ctg att     384
Asn Ile Tyr Ile Leu Asn Leu Ala Val Ala Asp Glu Cys Phe Leu Ile
        115                 120                 125

gga ata ccc ttt ctg ctg tac aca atg cga att tgc agc tgg cga ttc     432
Gly Ile Pro Phe Leu Leu Tyr Thr Met Arg Ile Cys Ser Trp Arg Phe
    130                 135                 140

ggg gag ttt atg tgc aaa gcc tac atg gtg agc aca tcc atc acc tcc     480
Gly Glu Phe Met Cys Lys Ala Tyr Met Val Ser Thr Ser Ile Thr Ser
145                 150                 155                 160

ttc acc tcg tcg att ttt ctg ctc atc atg tcc gcg gat cga tat ata     528
Phe Thr Ser Ser Ile Phe Leu Leu Ile Met Ser Ala Asp Arg Tyr Ile
                165                 170                 175

gcg gta tgc cac ccg att tcc tcg cca cga tat cga act ctg cat att     576
Ala Val Cys His Pro Ile Ser Ser Pro Arg Tyr Arg Thr Leu His Ile
            180                 185                 190

gcc aaa gtg gtc tca gcg att gcc tgg tca act tca gcg gtc ctc atg     624
Ala Lys Val Val Ser Ala Ile Ala Trp Ser Thr Ser Ala Val Leu Met
        195                 200                 205

ctg ccc gtg atc ctt tat gcc agc act gtg gag cag gag gat ggc atc     672
Leu Pro Val Ile Leu Tyr Ala Ser Thr Val Glu Gln Glu Asp Gly Ile
    210                 215                 220

aat tac tcg tgc aac ata atg tgg cca gat gcg tac aag aag cat tcg     720
Asn Tyr Ser Cys Asn Ile Met Trp Pro Asp Ala Tyr Lys Lys His Ser
225                 230                 235                 240

ggc acc acc ttc ata ctg tac aca ttt ttc cta gga ttc gcc aca ccg     768
Gly Thr Thr Phe Ile Leu Tyr Thr Phe Phe Leu Gly Phe Ala Thr Pro
                245                 250                 255

ctg tgc ttt atc ctg agt ttc tac tac ttg gtt ata agg aaa ctg cga     816

Leu Cys Phe Ile Leu Ser Phe Tyr Tyr Leu Val Ile Arg Lys Leu Arg
            260                 265                 270

tcg gtg ggt ccc aaa cca gga acg aag tcc aag gag aag agg cgg gct     864
Ser Val Gly Pro Lys Pro Gly Thr Lys Ser Lys Glu Lys Arg Arg Ala
        275                 280                 285

cac agg aag gtc act cga ctg gta ctg acg gtg gcc ctg att cac tcg     912
His Arg Lys Val Thr Arg Leu Val Leu Thr Val Ala Leu Ile His Ser
    290                 295                 300

aat ccc gcg caa agg gac ctc tcc cga ctg gaa ata ctc att ttc cta     960
Asn Pro Ala Gln Arg Asp Leu Ser Arg Leu Glu Ile Leu Ile Phe Leu
305                 310                 315                 320

ctt ctg ggg gca ctg gtt tac tcg aat tcg gcg gtg aat ccc ata ctt     1008
Leu Leu Gly Ala Leu Val Tyr Ser Asn Ser Ala Val Asn Pro Ile Leu
                325                 330                 335

tat gcc ttc cta agt gag aac ttc cgg aag agc ttc ttc aag gcc ttt     1056
Tyr Ala Phe Leu Ser Glu Asn Phe Arg Lys Ser Phe Phe Lys Ala Phe
            340                 345                 350

acc tgt atg aat aag cag gat atc aac gct caa ctc cag ctg gag ccc     1104
Thr Cys Met Asn Lys Gln Asp Ile Asn Ala Gln Leu Gln Leu Glu Pro
        355                 360                 365

agt gtt ttc acc aaa cag ggc agt aaa aag agg ggt ggc tcc aag cgc     1152
Ser Val Phe Thr Lys Gln Gly Ser Lys Lys Arg Gly Gly Ser Lys Arg
    370                 375                 380

ctg ttg acc agc aat ccg cag att cct cca ctg ctg cca ctg aat gcg     1200
Leu Leu Thr Ser Asn Pro Gln Ile Pro Pro Leu Leu Pro Leu Asn Ala
385                 390                 395                 400

ggt aac aac aat tca tcg acc acc aca tcc tcg acc acg aca gcg gaa     1248
Gly Asn Asn Asn Ser Ser Thr Thr Thr Ser Ser Thr Thr Thr Ala Glu
                405                 410                 415

aag acc gga acc acg ggg aca cag aaa tca tgc aat tcc aat ggc aaa     1296
Lys Thr Gly Thr Thr Gly Thr Gln Lys Ser Cys Asn Ser Asn Gly Lys
            420                 425                 430

gtg aca gct ccg ccg gag aat ttg att ata tgt ttg agc gag cag cag     1344
Val Thr Ala Pro Pro Glu Asn Leu Ile Ile Cys Leu Ser Glu Gln Gln
        435                 440                 445

gag gca ttt tgc acc acc gcg agg aga gga tcg ggc gca gtg cag cag     1392

Glu Ala Phe Cys Thr Thr Ala Arg Arg Gly Ser Gly Ala Val Gln Gln
    450                 455                 460

aca gat ttg taa                                                   1404
Thr Asp Leu
465

<210> 10
<211> 467
<212> PRT
<213> Drosophila melanogaster

<400> 10
Met Phe Thr Trp Leu Met Met Asp Val Leu Gln Phe Val Lys Gly Glu
  1               5                  10                  15

Met Thr Ala Asp Ser Glu Ala Asn Ala Thr Asn Trp Tyr Asn Thr Asn
             20                  25                  30

Glu Ser Leu Tyr Thr Thr Glu Leu Asn His Arg Trp Ile Ser Gly Ser
         35                  40                  45

Ser Thr Ile Gln Pro Glu Glu Ser Leu Tyr Gly Thr Asp Leu Pro Thr
     50                  55                  60

Tyr Gln His Cys Ile Ala Thr Arg Asn Ser Phe Ala Asp Leu Phe Thr
 65                  70                  75                  80

Val Val Leu Tyr Gly Phe Val Cys Ile Ile Gly Leu Phe Gly Asn Thr
                 85                  90                  95

Leu Val Ile Tyr Val Val Leu Arg Phe Ser Lys Met Gln Thr Val Thr
            100                 105                 110

Asn Ile Tyr Ile Leu Asn Leu Ala Val Ala Asp Glu Cys Phe Leu Ile
        115                 120                 125

Gly Ile Pro Phe Leu Leu Tyr Thr Met Arg Ile Cys Ser Trp Arg Phe
    130                 135                 140

Gly Glu Phe Met Cys Lys Ala Tyr Met Val Ser Thr Ser Ile Thr Ser
145                 150                 155                 160

Phe Thr Ser Ser Ile Phe Leu Leu Ile Met Ser Ala Asp Arg Tyr Ile
                165                 170                 175

Ala Val Cys His Pro Ile Ser Ser Pro Arg Tyr Arg Thr Leu His Ile

180                 185                 190

Ala Lys Val Val Ser Ala Ile Ala Trp Ser Thr Ser Ala Val Leu Met
        195                 200                 205

Leu Pro Val Ile Leu Tyr Ala Ser Thr Val Glu Gln Glu Asp Gly Ile
    210                 215                 220

Asn Tyr Ser Cys Asn Ile Met Trp Pro Asp Ala Tyr Lys Lys His Ser
225                 230                 235                 240

Gly Thr Thr Phe Ile Leu Tyr Thr Phe Phe Leu Gly Phe Ala Thr Pro
                245                 250                 255

Leu Cys Phe Ile Leu Ser Phe Tyr Tyr Leu Val Ile Arg Lys Leu Arg
            260                 265                 270

Ser Val Gly Pro Lys Pro Gly Thr Lys Ser Lys Glu Lys Arg Arg Ala
        275                 280                 285

His Arg Lys Val Thr Arg Leu Val Leu Thr Val Ala Leu Ile His Ser
    290                 295                 300

Asn Pro Ala Gln Arg Asp Leu Ser Arg Leu Glu Ile Leu Ile Phe Leu
305                 310                 315                 320

Leu Leu Gly Ala Leu Val Tyr Ser Asn Ser Ala Val Asn Pro Ile Leu
                325                 330                 335

Tyr Ala Phe Leu Ser Glu Asn Phe Arg Lys Ser Phe Phe Lys Ala Phe
            340                 345                 350

Thr Cys Met Asn Lys Gln Asp Ile Asn Ala Gln Leu Gln Leu Glu Pro
        355                 360                 365

Ser Val Phe Thr Lys Gln Gly Ser Lys Lys Arg Gly Gly Ser Lys Arg
    370                 375                 380

Leu Leu Thr Ser Asn Pro Gln Ile Pro Pro Leu Leu Pro Leu Asn Ala
385                 390                 395                 400

Gly Asn Asn Asn Ser Ser Thr Thr Thr Ser Ser Thr Thr Thr Ala Glu
                405                 410                 415

Lys Thr Gly Thr Thr Gly Thr Gln Lys Ser Cys Asn Ser Asn Gly Lys
            420                 425                 430

Val Thr Ala Pro Pro Glu Asn Leu Ile Ile Cys Leu Ser Glu Gln Gln

435                 440                 445

Glu Ala Phe Cys Thr Thr Ala Arg Arg Gly Ser Gly Ala Val Gln Gln
    450                 455                 460

Thr Asp Leu
465

<210> 11
<211> 957
<212> DNA
<213> Drosophila melanogaster

<220>
<221> CDS
<222> (1)..(954)

<400> 11
atg aat ctg ttc gcc tcg ttc gct gcc aat aac tcg ttg tgg ctg gtc     48
Met Asn Leu Phe Ala Ser Phe Ala Ala Asn Asn Ser Leu Trp Leu Val
  1               5                  10                  15

tgg tac ctg ctg gtc atg ccg aat tcg gag cta ctg cat cag agt ccg     96
Trp Tyr Leu Leu Val Met Pro Asn Ser Glu Leu Leu His Gln Ser Pro
             20                  25                  30

atg cgc tgc gtt gcc ctg cac ata acg cta cac tac ttc ctc ctg tcc    144
Met Arg Cys Val Ala Leu His Ile Thr Leu His Tyr Phe Leu Leu Ser
         35                  40                  45

aat tac tcc tgg atg ctc tgc gag gga ttc tat ctg cac acc gtc ctc    192
Asn Tyr Ser Trp Met Leu Cys Glu Gly Phe Tyr Leu His Thr Val Leu
     50                  55                  60

gtc gcc gca ttc att tcc gag aag agg ctg gtc aaa tgg ctc atc gca    240
Val Ala Ala Phe Ile Ser Glu Lys Arg Leu Val Lys Trp Leu Ile Ala
 65                  70                  75                  80

ttc ggc tgg ggc tcc ccg gcc atc gtc ata ttc gtc tat agc atg gct    288
Phe Gly Trp Gly Ser Pro Ala Ile Val Ile Phe Val Tyr Ser Met Ala
                 85                  90                  95

cgc ggt ctg ggc ggc acg ccc gag gac aat cgt cac tgc tgg atg aac    336
Arg Gly Leu Gly Gly Thr Pro Glu Asp Asn Arg His Cys Trp Met Asn
            100                 105                 110

caa acc aac tac caa aac att ctt atg gtg cct gtg tgt atc tcc atg 384
Gln Thr Asn Tyr Gln Asn Ile Leu Met Val Pro Val Cys Ile Ser Met
        115                 120                 125

ttc ctg aac ctc ctg ttc ctg tgc aac att gtg cga gtg gtc ctt ttg 432
Phe Leu Asn Leu Leu Phe Leu Cys Asn Ile Val Arg Val Val Leu Leu
    130                 135                 140

aaa ctg aat gcc ccg gcc agt att cag ggc agc tgc ggt cca tcg cga 480
Lys Leu Asn Ala Pro Ala Ser Ile Gln Gly Ser Cys Gly Pro Ser Arg
145                 150                 155                 160

acg gtt ttg caa gca ttt cgg gca acg ctg ctt ttg gtt cct ctg ctc 528
Thr Val Leu Gln Ala Phe Arg Ala Thr Leu Leu Leu Val Pro Leu Leu
                165                 170                 175

ggc ctc cag tac atc ctt acg ccc ttt cgt cct gca ccc aaa cat ccc 576
Gly Leu Gln Tyr Ile Leu Thr Pro Phe Arg Pro Ala Pro Lys His Pro
            180                 185                 190

tgg gag aat aca tac gaa atc atc tcg gcg ttt acg gcc tca ttt caa 624
Trp Glu Asn Thr Tyr Glu Ile Ile Ser Ala Phe Thr Ala Ser Phe Gln
        195                 200                 205

ggt ctg tgc gtt gcc att ttg ttc tgc ttt tgc aac ggc gag gtg att 672
Gly Leu Cys Val Ala Ile Leu Phe Cys Phe Cys Asn Gly Glu Val Ile
    210                 215                 220

gcc caa atg aag cgc aag tgg cgg atg atg tgc ttc agc aac cga ccg 720
Ala Gln Met Lys Arg Lys Trp Arg Met Met Cys Phe Ser Asn Arg Pro
225                 230                 235                 240

cgg acc aat tcc tat aca gcc act cag gtc tcg ccc cat cac caa agc 768
Arg Thr Asn Ser Tyr Thr Ala Thr Gln Val Ser Pro His His Gln Ser
                245                 250                 255

cac cag ttg tcc acg gat cag cag aga gct cgc agc cag agc ctg gcg 816
His Gln Leu Ser Thr Asp Gln Gln Arg Ala Arg Ser Gln Ser Leu Ala
            260                 265                 270

agt tcg tcg tcc ttc ctg gaa ggc tgg cgc gac cgg atg ccg ttc ctg 864
Ser Ser Ser Ser Phe Leu Glu Gly Trp Arg Asp Arg Met Pro Phe Leu
        275                 280                 285

aaa cgg cga caa act atc gac cac tcc cgc cag agc cag cca ctc atg 912
Lys Arg Arg Gln Thr Ile Asp His Ser Arg Gln Ser Gln Pro Leu Met
    290                 295                 300

gag gag ggc gga gag acc gtt ggc cag gcc aaa tcc gcg cca gnn 957
Glu Glu Gly Gly Glu Thr Val Gly Gln Ala Lys Ser Ala Pro
305                 310                 315

<210> 12
<211> 318
<212> PRT
<213> Drosophila melanogaster

<400> 12
Met Asn Leu Phe Ala Ser Phe Ala Ala Asn Asn Ser Leu Trp Leu Val
1               5                   10                  15

Trp Tyr Leu Leu Val Met Pro Asn Ser Glu Leu Leu His Gln Ser Pro
            20                  25                  30

Met Arg Cys Val Ala Leu His Ile Thr Leu His Tyr Phe Leu Leu Ser
        35                  40                  45

Asn Tyr Ser Trp Met Leu Cys Glu Gly Phe Tyr Leu His Thr Val Leu
    50                  55                  60

Val Ala Ala Phe Ile Ser Glu Lys Arg Leu Val Lys Trp Leu Ile Ala
65                  70                  75                  80

Phe Gly Trp Gly Ser Pro Ala Ile Val Ile Phe Val Tyr Ser Met Ala
                85                  90                  95

Arg Gly Leu Gly Gly Thr Pro Glu Asp Asn Arg His Cys Trp Met Asn
            100                 105                 110

Gln Thr Asn Tyr Gln Asn Ile Leu Met Val Pro Val Cys Ile Ser Met
        115                 120                 125

Phe Leu Asn Leu Leu Phe Leu Cys Asn Ile Val Arg Val Val Leu Leu
    130                 135                 140

Lys Leu Asn Ala Pro Ala Ser Ile Gln Gly Ser Cys Gly Pro Ser Arg
145                 150                 155                 160

Thr Val Leu Gln Ala Phe Arg Ala Thr Leu Leu Leu Val Pro Leu Leu
                165                 170                 175

Gly Leu Gln Tyr Ile Leu Thr Pro Phe Arg Pro Ala Pro Lys His Pro
            180                 185                 190

Trp Glu Asn Thr Tyr Glu Ile Ile Ser Ala Phe Thr Ala Ser Phe Gln

195                 200                 205

Gly Leu Cys Val Ala Ile Leu Phe Cys Phe Cys Asn Gly Glu Val Ile
    210                 215                 220

Ala Gln Met Lys Arg Lys Trp Arg Met Met Cys Phe Ser Asn Arg Pro
225                 230                 235                 240

Arg Thr Asn Ser Tyr Thr Ala Thr Gln Val Ser Pro His His Gln Ser
                245                 250                 255

His Gln Leu Ser Thr Asp Gln Gln Arg Ala Arg Ser Gln Ser Leu Ala
            260                 265                 270

Ser Ser Ser Ser Phe Leu Glu Gly Trp Arg Asp Arg Met Pro Phe Leu
        275                 280                 285

Lys Arg Arg Gln Thr Ile Asp His Ser Arg Gln Ser Gln Pro Leu Met
    290                 295                 300

Glu Glu Gly Gly Glu Thr Val Gly Gln Ala Lys Ser Ala Pro
305                 310                 315

<210> 13
<211> 1401
<212> DNA
<213> Drosophila melanogaster

<220>
<221> CDS
<222> (1)..(1398)

<400> 13
atg aag gat gtg ccc gac aaa tat gtg acg gca ctc tcg cac ttc ctc      48
Met Lys Asp Val Pro Asp Lys Tyr Val Thr Ala Leu Ser His Phe Leu
1               5                   10                  15

gac tgg cat tcc aat ggc acg gtt gac atg gag cga ctt agt ggt ccc      96
Asp Trp His Ser Asn Gly Thr Val Asp Met Glu Arg Leu Ser Gly Pro
            20                  25                  30

atc cta aag tcc agc atc aag tcg gtc tat tgg ctg ttt ctc atc cag      144
Ile Leu Lys Ser Ser Ile Lys Ser Val Tyr Trp Leu Phe Leu Ile Gln
        35                  40                  45

tac gca gct ctc gcc cta ctg ggc gtg gtg ctc aat gtg atc atc gtg      192

Tyr Ala Ala Leu Ala Leu Leu Gly Val Val Leu Asn Val Ile Ile Val
     50                  55                  60

gtg tac ata atg tac cat cga ctt tac aag gat gtg acc cat gcg ttc     240
Val Tyr Ile Met Tyr His Arg Leu Tyr Lys Asp Val Thr His Ala Phe
 65                  70                  75                  80

atc atc aat ctg gcc cta tgc cat ttt gtc cag tgc gcc ctc gtc ctg     288
Ile Ile Asn Leu Ala Leu Cys His Phe Val Gln Cys Ala Leu Val Leu
                 85                  90                  95

ccc gtc tcg ctg atg gtc atg ctc atc cag aac tgg atc ttt ggc cag     336
Pro Val Ser Leu Met Val Met Leu Ile Gln Asn Trp Ile Phe Gly Gln
            100                 105                 110

ttt ctg tgc ttc ttt ctg ccc atg ctg cag tcg aag att ttg gag atc     384
Phe Leu Cys Phe Phe Leu Pro Met Leu Gln Ser Lys Ile Leu Glu Ile
        115                 120                 125

ggc gac cct gtg cac atc tat agc acg aaa aaa caa cga tca aac ctt     432
Gly Asp Pro Val His Ile Tyr Ser Thr Lys Lys Gln Arg Ser Asn Leu
    130                 135                 140

aaa cag cga cag ctt tgg aac att ccc aaa ata ccg tca ttt tcc cag     480
Lys Gln Arg Gln Leu Trp Asn Ile Pro Lys Ile Pro Ser Phe Ser Gln
145                 150                 155                 160

ctt cag gat atg gaa att tca gca ggg agc gac cac gtt tcc cgg gaa     528
Leu Gln Asp Met Glu Ile Ser Ala Gly Ser Asp His Val Ser Arg Glu
                165                 170                 175

caa tgg gtc atc gac ggt ccc aac acg cat gag aag gtg atc atc ctc     576
Gln Trp Val Ile Asp Gly Pro Asn Thr His Glu Lys Val Ile Ile Leu
            180                 185                 190

tca gat cat cca tat gcc act cga gaa cga cgt tca tat gga aac att     624
Ser Asp His Pro Tyr Ala Thr Arg Glu Arg Arg Ser Tyr Gly Asn Ile
        195                 200                 205

cgt ggc gtg gag aac tat tgc gat ccg gtt gcc acc cag gac gaa cga     672
Arg Gly Val Glu Asn Tyr Cys Asp Pro Val Ala Thr Gln Asp Glu Arg
    210                 215                 220

atg gag gcc aat ccg ggc ata gtc ccg aaa gaa gaa gac cct gag cct     720
Met Glu Ala Asn Pro Gly Ile Val Pro Lys Glu Glu Asp Pro Glu Pro
225                 230                 235                 240

tct cca aaa atc acg aat gga ctt ata tat caa ggc acc ttc aat gaa     768

Ser Pro Lys Ile Thr Asn Gly Leu Ile Tyr Gln Gly Thr Phe Asn Glu
                245                 250                 255

gaa ttc gac atg ctg gac aat gag tca aaa gct aaa gca aat gga aac      816
Glu Phe Asp Met Leu Asp Asn Glu Ser Lys Ala Lys Ala Asn Gly Asn
            260                 265                 270

cta caa gat gtt aaa cca gaa gct ccg cag cat cct tta gaa aac acc      864
Leu Gln Asp Val Lys Pro Glu Ala Pro Gln His Pro Leu Glu Asn Thr
        275                 280                 285

gcc ttt cag aat aca ccg ccc agt att ggc gga cag aat aat gaa aac      912
Ala Phe Gln Asn Thr Pro Pro Ser Ile Gly Gly Gln Asn Asn Glu Asn
    290                 295                 300

act tcg gga tac ata gtc aag agg gat gaa atg gca acc ttg atg aat      960
Thr Ser Gly Tyr Ile Val Lys Arg Asp Glu Met Ala Thr Leu Met Asn
305                 310                 315                 320

gat ata gac acg gat ggt atc ttc gat tat ctg aaa ctg cag ttg gac     1008
Asp Ile Asp Thr Asp Gly Ile Phe Asp Tyr Leu Lys Leu Gln Leu Asp
                325                 330                 335

acg cag acg cca acg gag cag caa gac aat aag gag ccc cat ttg ctg     1056
Thr Gln Thr Pro Thr Glu Gln Gln Asp Asn Lys Glu Pro His Leu Leu
            340                 345                 350

acg tcc tca gga gag aag aat aaa tat att ggt gtg gtg tcc ttg cat     1104
Thr Ser Ser Gly Glu Lys Asn Lys Tyr Ile Gly Val Val Ser Leu His
        355                 360                 365

gag gca tgt ccc ttt cca acg ttc gag aag ata gtc aag gat att atc     1152
Glu Ala Cys Pro Phe Pro Thr Phe Glu Lys Ile Val Lys Asp Ile Ile
    370                 375                 380

gag cag gat tgc aag aag ata ata gag acc aag aag atg gtg gat gag     1200
Glu Gln Asp Cys Lys Lys Ile Ile Glu Thr Lys Lys Met Val Asp Glu
385                 390                 395                 400

ctg gtc aag gtg gag gtc aaa aag gag atc gag gag gtg gtc aag cag     1248
Leu Val Lys Val Glu Val Lys Lys Glu Ile Glu Glu Val Val Lys Gln
                405                 410                 415

gag gtc aaa aag gag atc gag gag gtg gtc aag cag gag gtc aaa aag     1296
Glu Val Lys Lys Glu Ile Glu Glu Val Val Lys Gln Glu Val Lys Lys
            420                 425                 430

gag atc gag gag gtg gtc aag aag gag atc aag aag gag atc aag gag     1344

Glu Ile Glu Glu Val Val Lys Lys Glu Ile Lys Lys Glu Ile Lys Glu
        435                 440                 445

gag atc gag aag gag atc cag gcg gag atc gac gat gag atc gtg gaa    1392
Glu Ile Glu Lys Glu Ile Gln Ala Glu Ile Asp Asp Glu Ile Val Glu
    450                 455                 460

gtg gac tag                                                        1401
Val Asp
465

<210> 14
<211> 466
<212> PRT
<213> Drosophila melanogaster

<400> 14
Met Lys Asp Val Pro Asp Lys Tyr Val Thr Ala Leu Ser His Phe Leu
  1               5                  10                  15

Asp Trp His Ser Asn Gly Thr Val Asp Met Glu Arg Leu Ser Gly Pro
             20                  25                  30

Ile Leu Lys Ser Ser Ile Lys Ser Val Tyr Trp Leu Phe Leu Ile Gln
         35                  40                  45

Tyr Ala Ala Leu Ala Leu Leu Gly Val Val Leu Asn Val Ile Ile Val
     50                  55                  60

Val Tyr Ile Met Tyr His Arg Leu Tyr Lys Asp Val Thr His Ala Phe
 65                  70                  75                  80

Ile Ile Asn Leu Ala Leu Cys His Phe Val Gln Cys Ala Leu Val Leu
                 85                  90                  95

Pro Val Ser Leu Met Val Met Leu Ile Gln Asn Trp Ile Phe Gly Gln
            100                 105                 110

Phe Leu Cys Phe Phe Leu Pro Met Leu Gln Ser Lys Ile Leu Glu Ile
        115                 120                 125

Gly Asp Pro Val His Ile Tyr Ser Thr Lys Lys Gln Arg Ser Asn Leu
    130                 135                 140

Lys Gln Arg Gln Leu Trp Asn Ile Pro Lys Ile Pro Ser Phe Ser Gln
145                 150                 155                 160

Leu Gln Asp Met Glu Ile Ser Ala Gly Ser Asp His Val Ser Arg Glu
                165                 170                 175

Gln Trp Val Ile Asp Gly Pro Asn Thr His Glu Lys Val Ile Ile Leu
            180                 185                 190

Ser Asp His Pro Tyr Ala Thr Arg Glu Arg Arg Ser Tyr Gly Asn Ile
        195                 200                 205

Arg Gly Val Glu Asn Tyr Cys Asp Pro Val Ala Thr Gln Asp Glu Arg
    210                 215                 220

Met Glu Ala Asn Pro Gly Ile Val Pro Lys Glu Glu Asp Pro Glu Pro
225                 230                 235                 240

Ser Pro Lys Ile Thr Asn Gly Leu Ile Tyr Gln Gly Thr Phe Asn Glu
                245                 250                 255

Glu Phe Asp Met Leu Asp Asn Glu Ser Lys Ala Lys Ala Asn Gly Asn
            260                 265                 270

Leu Gln Asp Val Lys Pro Glu Ala Pro Gln His Pro Leu Glu Asn Thr
        275                 280                 285

Ala Phe Gln Asn Thr Pro Pro Ser Ile Gly Gly Gln Asn Asn Glu Asn
    290                 295                 300

Thr Ser Gly Tyr Ile Val Lys Arg Asp Glu Met Ala Thr Leu Met Asn
305                 310                 315                 320

Asp Ile Asp Thr Asp Gly Ile Phe Asp Tyr Leu Lys Leu Gln Leu Asp
                325                 330                 335

Thr Gln Thr Pro Thr Glu Gln Gln Asp Asn Lys Glu Pro His Leu Leu
            340                 345                 350

Thr Ser Ser Gly Glu Lys Asn Lys Tyr Ile Gly Val Val Ser Leu His
        355                 360                 365

Glu Ala Cys Pro Phe Pro Thr Phe Glu Lys Ile Val Lys Asp Ile Ile
    370                 375                 380

Glu Gln Asp Cys Lys Lys Ile Ile Glu Thr Lys Lys Met Val Asp Glu
385                 390                 395                 400

Leu Val Lys Val Glu Val Lys Lys Glu Ile Glu Glu Val Val Lys Gln
                405                 410                 415

Glu Val Lys Lys Glu Ile Glu Glu Val Val Lys Gln Glu Val Lys Lys
            420                 425                 430

Glu Ile Glu Glu Val Val Lys Lys Glu Ile Lys Lys Glu Ile Lys Glu
        435                 440                 445

Glu Ile Glu Lys Glu Ile Gln Ala Glu Ile Asp Asp Glu Ile Val Glu
    450                 455                 460

Val Asp
465

<210> 15
<211> 2001
<212> DNA
<213> Drosophila melanogaster

<220>
<221> CDS
<222> (1)..(1998)

<400> 15
atg gcc aat ctg cgt ccc ctg agc gcc gaa ctt cgt cgc att gct gaa      48
Met Ala Asn Leu Arg Pro Leu Ser Ala Glu Leu Arg Arg Ile Ala Glu
  1               5                  10                  15

acg gag cta aac gaa gtg gag gag cga gtg ccc gcg gat ctg aaa gct      96
Thr Glu Leu Asn Glu Val Glu Glu Arg Val Pro Ala Asp Leu Lys Ala
             20                  25                  30

ctt cgg gat tgg ttg gcc aag caa cct cat ctg agg gcc cgc cag gat     144
Leu Arg Asp Trp Leu Ala Lys Gln Pro His Leu Arg Ala Arg Gln Asp
         35                  40                  45

gac cag ttc cta gtt ggc ttc ttg cga ggc tgc aag ttt agc ctg gag     192
Asp Gln Phe Leu Val Gly Phe Leu Arg Gly Cys Lys Phe Ser Leu Glu
     50                  55                  60

aag acc aag tcc aag ctg gat cac ttt tat act att aag acg ctt atg     240
Lys Thr Lys Ser Lys Leu Asp His Phe Tyr Thr Ile Lys Thr Leu Met
 65                  70                  75                  80

cct gag ctc ttc ggc aaa aga ttg gtg gac gaa agg aac cta atc ttg     288
Pro Glu Leu Phe Gly Lys Arg Leu Val Asp Glu Arg Asn Leu Ile Leu
                 85                  90                  95

tgc aga tct gga acc tat gtt cga ttg ccc aaa cct tgg gga acc gat    336
Cys Arg Ser Gly Thr Tyr Val Arg Leu Pro Lys Pro Trp Gly Thr Asp
            100                 105                 110

ggc ccc cgc ctg cag ctt acg aac tat gag aaa ttc gac cct aag gaa    384
Gly Pro Arg Leu Gln Leu Thr Asn Tyr Glu Lys Phe Asp Pro Lys Glu
        115                 120                 125

ttc aag tta ttg gat ctc ttc cgc tac caa acc atg ata acg gaa cag    432
Phe Lys Leu Leu Asp Leu Phe Arg Tyr Gln Thr Met Ile Thr Glu Gln
    130                 135                 140

tca atc cgt gag gat gac cac agc aac att agc ggc tac gtc gag atc    480
Ser Ile Arg Glu Asp Asp His Ser Asn Ile Ser Gly Tyr Val Glu Ile
145                 150                 155                 160

gtt gac atg gcc aaa atg agc ctc agt ttt ctg gcc caa ctg gat ttc    528
Val Asp Met Ala Lys Met Ser Leu Ser Phe Leu Ala Gln Leu Asp Phe
                165                 170                 175

acg ctc atc aaa agg atg gga ata ttc gct gag aag gca cag ccc act    576
Thr Leu Ile Lys Arg Met Gly Ile Phe Ala Glu Lys Ala Gln Pro Thr
            180                 185                 190

cgc ttg aaa gga gtc cat ctg atc aat tgc ccc aag gag gga gtg gcg    624
Arg Leu Lys Gly Val His Leu Ile Asn Cys Pro Lys Glu Gly Val Ala
        195                 200                 205

tta ctg aat ttg gcc aag agc cta atg ccc agc aag ctc cag cag cgg    672
Leu Leu Asn Leu Ala Lys Ser Leu Met Pro Ser Lys Leu Gln Gln Arg
    210                 215                 220

ttt cac gtg tat aaa aat ctc gag caa tta aac gag gtg ata cca cgc    720
Phe His Val Tyr Lys Asn Leu Glu Gln Leu Asn Glu Val Ile Pro Arg
225                 230                 235                 240

gaa tac ctg ccg gaa gaa tat ggt ggc aac aat gga cgt ata gcc gac    768
Glu Tyr Leu Pro Glu Glu Tyr Gly Gly Asn Asn Gly Arg Ile Ala Asp
                245                 250                 255

att cag gcg gaa gcg gag aag aaa ttg ctg tcc tat gag agc tat ttt    816
Ile Gln Ala Glu Ala Glu Lys Lys Leu Leu Ser Tyr Glu Ser Tyr Phe
            260                 265                 270

gct gag gat tcc cag tac gga ttt gac ttc ggc aaa tgg gac ttc cca    864
Ala Glu Asp Ser Gln Tyr Gly Phe Asp Phe Gly Lys Trp Asp Phe Pro
        275                 280                 285

gcc gag cgc atc tgg ctg cac aag ccc aat ggg gag atc acc tgg aag    912
Ala Glu Arg Ile Trp Leu His Lys Pro Asn Gly Glu Ile Thr Trp Lys
    290                 295                 300

atc tgc act ttt cta cca ctg att gct ttt ggt ctg tat gga aac ttc    960
Ile Cys Thr Phe Leu Pro Leu Ile Ala Phe Gly Leu Tyr Gly Asn Phe
305                 310                 315                 320

agc atg gtt tat gtg ata gcc acc aac cgt tct ttg cgc tcg ccc acc    1008
Ser Met Val Tyr Val Ile Ala Thr Asn Arg Ser Leu Arg Ser Pro Thr
                325                 330                 335

aac ttg att att gca aat atg gcc gtt gcg gat cta ctg acc ctg gcc    1056
Asn Leu Ile Ile Ala Asn Met Ala Val Ala Asp Leu Leu Thr Leu Ala
            340                 345                 350

att tgt cct gcc atg ttc atg gtg aac gac ttc tat cag aac tac cag    1104
Ile Cys Pro Ala Met Phe Met Val Asn Asp Phe Tyr Gln Asn Tyr Gln
        355                 360                 365

ttg gga tgc gtg ggc tgc aag ctg gag ggc ttt ttg gtg gtg gtc ttc    1152
Leu Gly Cys Val Gly Cys Lys Leu Glu Gly Phe Leu Val Val Val Phe
    370                 375                 380

ctc atc aca gcc gtg ctc aat ctc tcg gta gtc agc tac gat cgt ctc    1200
Leu Ile Thr Ala Val Leu Asn Leu Ser Val Val Ser Tyr Asp Arg Leu
385                 390                 395                 400

acg gcc atc gtg ctg cca atg gag acg cgc ctc acc ata agg ggc gtc    1248
Thr Ala Ile Val Leu Pro Met Glu Thr Arg Leu Thr Ile Arg Gly Val
                405                 410                 415

cag ata gtg gtg gtc tgc acc tgg gtc tcg ggt atc cta ttg gca tca    1296
Gln Ile Val Val Val Cys Thr Trp Val Ser Gly Ile Leu Leu Ala Ser
            420                 425                 430

cca ttg gct ttc tat cgc tct tac agg gtg cga gtg tgg aag aac ttt    1344
Pro Leu Ala Phe Tyr Arg Ser Tyr Arg Val Arg Val Trp Lys Asn Phe
        435                 440                 445

acg gag cga tat tgc aag gag aac acc tct gtg ctg ccc aag tac tgg    1392
Thr Glu Arg Tyr Cys Lys Glu Asn Thr Ser Val Leu Pro Lys Tyr Trp
    450                 455                 460

tat gtt ctg atc acc att ctg gtg tgg ctg cca tta ggc atc atg ctt    1440
Tyr Val Leu Ile Thr Ile Leu Val Trp Leu Pro Leu Gly Ile Met Leu
465                 470                 475                 480

atc tgc tac ata gcc ata ttt tat aag ctt gat cgg tat gaa aag cgg 1488
Ile Cys Tyr Ile Ala Ile Phe Tyr Lys Leu Asp Arg Tyr Glu Lys Arg
                485                 490                 495

gtc cta agt cgc gag aac ccg ctg aca gtg agc tat aaa cgc agc gtg 1536
Val Leu Ser Arg Glu Asn Pro Leu Thr Val Ser Tyr Lys Arg Ser Val
            500                 505                 510

gcc aag aca ctg ttc att gtg gtc gtt gtc ttt gca gct cta cga ctg 1584
Ala Lys Thr Leu Phe Ile Val Val Val Val Phe Ala Ala Leu Arg Leu
        515                 520                 525

ccc ttt aca atc ctg gtt gta cta cgg gaa aaa tac ttc ggt gaa gat 1632
Pro Phe Thr Ile Leu Val Val Leu Arg Glu Lys Tyr Phe Gly Glu Asp
    530                 535                 540

gtc tct gtc agc agt gga atg cag cta ttc tgg tac ata tca cag tat 1680
Val Ser Val Ser Ser Gly Met Gln Leu Phe Trp Tyr Ile Ser Gln Tyr
545                 550                 555                 560

ttg atg ttt ctg aac gcc gcc gtc aat ccg ctg atc tat ggc ttc aac 1728
Leu Met Phe Leu Asn Ala Ala Val Asn Pro Leu Ile Tyr Gly Phe Asn
                565                 570                 575

aat gag aac ttt aga agg gcc tac tac caa att tct tgg gtt cgc cga 1776
Asn Glu Asn Phe Arg Arg Ala Tyr Tyr Gln Ile Ser Trp Val Arg Arg
            580                 585                 590

tgg cga gac gcc acc caa atg aag aag ttt tct cga tca cca gac cac 1824
Trp Arg Asp Ala Thr Gln Met Lys Lys Phe Ser Arg Ser Pro Asp His
        595                 600                 605

tgc tgc tac tgt gcc ttt atg aag aat gga aaa cga acc tca gag gca 1872
Cys Cys Tyr Cys Ala Phe Met Lys Asn Gly Lys Arg Thr Ser Glu Ala
    610                 615                 620

gca caa aag gca gga aat tta gaa aaa gac ata agc aag gat atg tcg 1920
Ala Gln Lys Ala Gly Asn Leu Glu Lys Asp Ile Ser Lys Asp Met Ser
625                 630                 635                 640

tcg gca caa caa tcg gcc aag tca acg aag ata gtt gaa aat gaa ttt 1968
Ser Ala Gln Gln Ser Ala Lys Ser Thr Lys Ile Val Glu Asn Glu Phe
                645                 650                 655

gta tca gaa atc gag gca gac gga ttt ata taa 2001
Val Ser Glu Ile Glu Ala Asp Gly Phe Ile
            660                 665

<210> 16
<211> 666
<212> PRT
<213> Drosophila melanogaster

<400> 16
Met Ala Asn Leu Arg Pro Leu Ser Ala Glu Leu Arg Arg Ile Ala Glu
1               5                   10                  15

Thr Glu Leu Asn Glu Val Glu Glu Arg Val Pro Ala Asp Leu Lys Ala
            20                  25                  30

Leu Arg Asp Trp Leu Ala Lys Gln Pro His Leu Arg Ala Arg Gln Asp
        35                  40                  45

Asp Gln Phe Leu Val Gly Phe Leu Arg Gly Cys Lys Phe Ser Leu Glu
    50                  55                  60

Lys Thr Lys Ser Lys Leu Asp His Phe Tyr Thr Ile Lys Thr Leu Met
65                  70                  75                  80

Pro Glu Leu Phe Gly Lys Arg Leu Val Asp Glu Arg Asn Leu Ile Leu
                85                  90                  95

Cys Arg Ser Gly Thr Tyr Val Arg Leu Pro Lys Pro Trp Gly Thr Asp
            100                 105                 110

Gly Pro Arg Leu Gln Leu Thr Asn Tyr Glu Lys Phe Asp Pro Lys Glu
        115                 120                 125

Phe Lys Leu Leu Asp Leu Phe Arg Tyr Gln Thr Met Ile Thr Glu Gln
    130                 135                 140

Ser Ile Arg Glu Asp Asp His Ser Asn Ile Ser Gly Tyr Val Glu Ile
145                 150                 155                 160

Val Asp Met Ala Lys Met Ser Leu Ser Phe Leu Ala Gln Leu Asp Phe
                165                 170                 175

Thr Leu Ile Lys Arg Met Gly Ile Phe Ala Glu Lys Ala Gln Pro Thr
            180                 185                 190

Arg Leu Lys Gly Val His Leu Ile Asn Cys Pro Lys Glu Gly Val Ala
        195                 200                 205

Leu Leu Asn Leu Ala Lys Ser Leu Met Pro Ser Lys Leu Gln Gln Arg
    210                 215                 220

Phe His Val Tyr Lys Asn Leu Glu Gln Leu Asn Glu Val Ile Pro Arg
225                 230                 235                 240

Glu Tyr Leu Pro Glu Glu Tyr Gly Gly Asn Asn Gly Arg Ile Ala Asp
                245                 250                 255

Ile Gln Ala Glu Ala Glu Lys Lys Leu Leu Ser Tyr Glu Ser Tyr Phe
            260                 265                 270

Ala Glu Asp Ser Gln Tyr Gly Phe Asp Phe Gly Lys Trp Asp Phe Pro
        275                 280                 285

Ala Glu Arg Ile Trp Leu His Lys Pro Asn Gly Glu Ile Thr Trp Lys
    290                 295                 300

Ile Cys Thr Phe Leu Pro Leu Ile Ala Phe Gly Leu Tyr Gly Asn Phe
305                 310                 315                 320

Ser Met Val Tyr Val Ile Ala Thr Asn Arg Ser Leu Arg Ser Pro Thr
                325                 330                 335

Asn Leu Ile Ile Ala Asn Met Ala Val Ala Asp Leu Leu Thr Leu Ala
            340                 345                 350

Ile Cys Pro Ala Met Phe Met Val Asn Asp Phe Tyr Gln Asn Tyr Gln
        355                 360                 365

Leu Gly Cys Val Gly Cys Lys Leu Glu Gly Phe Leu Val Val Val Phe
    370                 375                 380

Leu Ile Thr Ala Val Leu Asn Leu Ser Val Val Ser Tyr Asp Arg Leu
385                 390                 395                 400

Thr Ala Ile Val Leu Pro Met Glu Thr Arg Leu Thr Ile Arg Gly Val
                405                 410                 415

Gln Ile Val Val Val Cys Thr Trp Val Ser Gly Ile Leu Leu Ala Ser
            420                 425                 430

Pro Leu Ala Phe Tyr Arg Ser Tyr Arg Val Arg Val Trp Lys Asn Phe
        435                 440                 445

Thr Glu Arg Tyr Cys Lys Glu Asn Thr Ser Val Leu Pro Lys Tyr Trp
    450                 455                 460

Tyr Val Leu Ile Thr Ile Leu Val Trp Leu Pro Leu Gly Ile Met Leu
465                 470                 475                 480

Ile Cys Tyr Ile Ala Ile Phe Tyr Lys Leu Asp Arg Tyr Glu Lys Arg
                485                 490                 495

Val Leu Ser Arg Glu Asn Pro Leu Thr Val Ser Tyr Lys Arg Ser Val
            500                 505                 510

Ala Lys Thr Leu Phe Ile Val Val Val Val Phe Ala Ala Leu Arg Leu
        515                 520                 525

Pro Phe Thr Ile Leu Val Val Leu Arg Glu Lys Tyr Phe Gly Glu Asp
    530                 535                 540

Val Ser Val Ser Ser Gly Met Gln Leu Phe Trp Tyr Ile Ser Gln Tyr
545                 550                 555                 560

Leu Met Phe Leu Asn Ala Ala Val Asn Pro Leu Ile Tyr Gly Phe Asn
                565                 570                 575

Asn Glu Asn Phe Arg Arg Ala Tyr Tyr Gln Ile Ser Trp Val Arg Arg
            580                 585                 590

Trp Arg Asp Ala Thr Gln Met Lys Lys Phe Ser Arg Ser Pro Asp His
        595                 600                 605

Cys Cys Tyr Cys Ala Phe Met Lys Asn Gly Lys Arg Thr Ser Glu Ala
    610                 615                 620

Ala Gln Lys Ala Gly Asn Leu Glu Lys Asp Ile Ser Lys Asp Met Ser
625                 630                 635                 640

Ser Ala Gln Gln Ser Ala Lys Ser Thr Lys Ile Val Glu Asn Glu Phe
                645                 650                 655

Val Ser Glu Ile Glu Ala Asp Gly Phe Ile
            660                 665

<210> 17
<211> 1233
<212> DNA
<213> Drosophila melanogaster

<220>
<221> CDS
<222> (1)..(1230)

<400> 17
atg gaa acg gac ttg gcc atg aac atc ggc ctg gac act tcc ggt gaa    48
Met Glu Thr Asp Leu Ala Met Asn Ile Gly Leu Asp Thr Ser Gly Glu
1               5                   10                  15

gcc ccc aca gcc cta ccc ccg atg ccg aat gtc acc gag acg ctg tgg    96
Ala Pro Thr Ala Leu Pro Pro Met Pro Asn Val Thr Glu Thr Leu Trp
            20                  25                  30

gat ctg gcc atg gtg gtc agt cag tcc aca cag tgg ccc ctc ctg gac    144
Asp Leu Ala Met Val Val Ser Gln Ser Thr Gln Trp Pro Leu Leu Asp
        35                  40                  45

acc ggc tcg tcc gag aac ttc agc gaa ctg gtc acc acc gag aca ccc    192
Thr Gly Ser Ser Glu Asn Phe Ser Glu Leu Val Thr Thr Glu Thr Pro
    50                  55                  60

tac gtg ccc tac ggc cgt cga ccg gaa acc tac atc gtg ccc atc ctc    240
Tyr Val Pro Tyr Gly Arg Arg Pro Glu Thr Tyr Ile Val Pro Ile Leu
65                  70                  75                  80

ttt gcc ctg atc ttc gtg gtc ggt gtc ctg ggc aat ggc act ttg att    288
Phe Ala Leu Ile Phe Val Val Gly Val Leu Gly Asn Gly Thr Leu Ile
                85                  90                  95

gtg gtc ttc ctc agt gtg cga cag atg cga aat gtt cca aac acc ggc    336
Val Val Phe Leu Ser Val Arg Gln Met Arg Asn Val Pro Asn Thr Gly
            100                 105                 110

ggc cag aga acg tgg caa aca aag tgc cac atg cag ctt att tat ggt    384
Gly Gln Arg Thr Trp Gln Thr Lys Cys His Met Gln Leu Ile Tyr Gly
        115                 120                 125

aag cgg cgt ggt tgg ata aag tcg gaa tta ctg cat gag atc acg gac    432
Lys Arg Arg Gly Trp Ile Lys Ser Glu Leu Leu His Glu Ile Thr Asp
    130                 135                 140

cga att gga tgt gcc act act gag cac cat tcg ttc gct ggc ctc aag    480
Arg Ile Gly Cys Ala Thr Thr Glu His His Ser Phe Ala Gly Leu Lys
145                 150                 155                 160

ttt tac aca gtt gaa tat tgg cca tat ggc agc ttc ctg tgc agc ctg    528
Phe Tyr Thr Val Glu Tyr Trp Pro Tyr Gly Ser Phe Leu Cys Ser Leu
                165                 170                 175

tcg gag ttc atg aag gat gtg tcc atc gga gtg tca gtg ttt acg ctg    576
Ser Glu Phe Met Lys Asp Val Ser Ile Gly Val Ser Val Phe Thr Leu
            180                 185                 190

act gca tta tcc ggc gat cgg tac ttt gcc atc gtg gat cct ttg agg 624
Thr Ala Leu Ser Gly Asp Arg Tyr Phe Ala Ile Val Asp Pro Leu Arg
        195                 200                 205

aag ttc cac gct cat ggt ggt ggt cgt cgg gcc acc aga atg acc ttg 672
Lys Phe His Ala His Gly Gly Gly Arg Arg Ala Thr Arg Met Thr Leu
    210                 215                 220

gcc acc gcg gtt tcc att tgg ctg ctg gcc ata ctc tgc gga ctt cca 720
Ala Thr Ala Val Ser Ile Trp Leu Leu Ala Ile Leu Cys Gly Leu Pro
225                 230                 235                 240

gcg cta att gga agt aac ctg aag cat ctc ggc ata aac gaa aag tcc 768
Ala Leu Ile Gly Ser Asn Leu Lys His Leu Gly Ile Asn Glu Lys Ser
                245                 250                 255

ata gtc att tgc tat ccg tat ccg gag gag tgg ggc atc aac tac gcc 816
Ile Val Ile Cys Tyr Pro Tyr Pro Glu Glu Trp Gly Ile Asn Tyr Ala
            260                 265                 270

aag tcc atg gtc ctg ttg cac ttc ctg gtc tac tac gcc att cct ttg 864
Lys Ser Met Val Leu Leu His Phe Leu Val Tyr Tyr Ala Ile Pro Leu
        275                 280                 285

gtc gtc ata gca gtt ttc tac gtg ctc atc gcc ctc cat ttg atg tat 912
Val Val Ile Ala Val Phe Tyr Val Leu Ile Ala Leu His Leu Met Tyr
    290                 295                 300

tcg gcc agt gtt cct ggt gaa att cag gga gct gtg cgt cag gtt cgt 960
Ser Ala Ser Val Pro Gly Glu Ile Gln Gly Ala Val Arg Gln Val Arg
305                 310                 315                 320

gcc cgt cgc aaa gtg gct gtt act gtg ctg gcc ttc gtt gtc atc ttt 1008
Ala Arg Arg Lys Val Ala Val Thr Val Leu Ala Phe Val Val Ile Phe
                325                 330                 335

gga att tgc ttt ctg ccc tac cac gtc ttc ttc ctc tgg ttc tac ttc 1056
Gly Ile Cys Phe Leu Pro Tyr His Val Phe Phe Leu Trp Phe Tyr Phe
            340                 345                 350

tgg ccc acc gcc cag gat gat tac aat gcc ttc tgg cat gtg ctg cgc 1104
Trp Pro Thr Ala Gln Asp Asp Tyr Asn Ala Phe Trp His Val Leu Arg
        355                 360                 365

atc gtc gcc tac tgc atg tcc ttt gcc aac agc tgt gcg aat cct gtg 1152
Ile Val Ala Tyr Cys Met Ser Phe Ala Asn Ser Cys Ala Asn Pro Val
    370                 375                 380

gcc cta tac ttc gtg agc ggc gcc ttt cgc aag cat ttc aat agg tat    1200
Ala Leu Tyr Phe Val Ser Gly Ala Phe Arg Lys His Phe Asn Arg Tyr
385                 390                 395                 400

ttg ttc gtt tac atc aat cca cca gcc gca taa                         1233
Leu Phe Val Tyr Ile Asn Pro Pro Ala Ala
                405                 410

<210> 18
<211> 410
<212> PRT
<213> Drosophila melanogaster

<400> 18
Met Glu Thr Asp Leu Ala Met Asn Ile Gly Leu Asp Thr Ser Gly Glu
1               5                   10                  15

Ala Pro Thr Ala Leu Pro Pro Met Pro Asn Val Thr Glu Thr Leu Trp
            20                  25                  30

Asp Leu Ala Met Val Val Ser Gln Ser Thr Gln Trp Pro Leu Leu Asp
        35                  40                  45

Thr Gly Ser Ser Glu Asn Phe Ser Glu Leu Val Thr Thr Glu Thr Pro
    50                  55                  60

Tyr Val Pro Tyr Gly Arg Arg Pro Glu Thr Tyr Ile Val Pro Ile Leu
65                  70                  75                  80

Phe Ala Leu Ile Phe Val Val Gly Val Leu Gly Asn Gly Thr Leu Ile
                85                  90                  95

Val Val Phe Leu Ser Val Arg Gln Met Arg Asn Val Pro Asn Thr Gly
            100                 105                 110

Gly Gln Arg Thr Trp Gln Thr Lys Cys His Met Gln Leu Ile Tyr Gly
        115                 120                 125

Lys Arg Arg Gly Trp Ile Lys Ser Glu Leu Leu His Glu Ile Thr Asp
    130                 135                 140

Arg Ile Gly Cys Ala Thr Thr Glu His His Ser Phe Ala Gly Leu Lys
145                 150                 155                 160

Phe Tyr Thr Val Glu Tyr Trp Pro Tyr Gly Ser Phe Leu Cys Ser Leu
                165                 170                 175

Ser Glu Phe Met Lys Asp Val Ser Ile Gly Val Ser Val Phe Thr Leu
            180                 185                 190

Thr Ala Leu Ser Gly Asp Arg Tyr Phe Ala Ile Val Asp Pro Leu Arg
        195                 200                 205

Lys Phe His Ala His Gly Gly Gly Arg Arg Ala Thr Arg Met Thr Leu
    210                 215                 220

Ala Thr Ala Val Ser Ile Trp Leu Leu Ala Ile Leu Cys Gly Leu Pro
225                 230                 235                 240

Ala Leu Ile Gly Ser Asn Leu Lys His Leu Gly Ile Asn Glu Lys Ser
                245                 250                 255

Ile Val Ile Cys Tyr Pro Tyr Pro Glu Glu Trp Gly Ile Asn Tyr Ala
            260                 265                 270

Lys Ser Met Val Leu Leu His Phe Leu Val Tyr Tyr Ala Ile Pro Leu
        275                 280                 285

Val Val Ile Ala Val Phe Tyr Val Leu Ile Ala Leu His Leu Met Tyr
    290                 295                 300

Ser Ala Ser Val Pro Gly Glu Ile Gln Gly Ala Val Arg Gln Val Arg
305                 310                 315                 320

Ala Arg Arg Lys Val Ala Val Thr Val Leu Ala Phe Val Val Ile Phe
                325                 330                 335

Gly Ile Cys Phe Leu Pro Tyr His Val Phe Phe Leu Trp Phe Tyr Phe
            340                 345                 350

Trp Pro Thr Ala Gln Asp Asp Tyr Asn Ala Phe Trp His Val Leu Arg
        355                 360                 365

Ile Val Ala Tyr Cys Met Ser Phe Ala Asn Ser Cys Ala Asn Pro Val
    370                 375                 380

Ala Leu Tyr Phe Val Ser Gly Ala Phe Arg Lys His Phe Asn Arg Tyr
385                 390                 395                 400

Leu Phe Val Tyr Ile Asn Pro Pro Ala Ala
                405                 410

<210> 19
<211> 2586
<212> DNA
<213> Drosophila melanogaster

<220>
<221> CDS
<222> (1)..(2583)

<400> 19
atg gag ctg gac gat aat tta ata agc agc ctg ccc gag gga ctc agc     48
Met Glu Leu Asp Asp Asn Leu Ile Ser Ser Leu Pro Glu Gly Leu Ser
1               5                   10                  15

aaa ttg tcg caa ctg cag gag ctt tcc atc acc agc aat cga ctg cgc     96
Lys Leu Ser Gln Leu Gln Glu Leu Ser Ile Thr Ser Asn Arg Leu Arg
            20                  25                  30

tgg ata aac gac acg gaa ctg ccc agg agc atg caa atg ctg gac atg     144
Trp Ile Asn Asp Thr Glu Leu Pro Arg Ser Met Gln Met Leu Asp Met
        35                  40                  45

agg gcc aat cca ctg tcc aca att tcg cca ggg gcg ttt cgg ggc atg     192
Arg Ala Asn Pro Leu Ser Thr Ile Ser Pro Gly Ala Phe Arg Gly Met
    50                  55                  60

tcc aag ttg cgg aag ctg att tta tcg gat gtg cga act ttg cgc tcg     240
Ser Lys Leu Arg Lys Leu Ile Leu Ser Asp Val Arg Thr Leu Arg Ser
65                  70                  75                  80

ttt ccc gaa ctt gag gcc tgc cat gcg ctg gaa ata ctg aaa ctg gat     288
Phe Pro Glu Leu Glu Ala Cys His Ala Leu Glu Ile Leu Lys Leu Asp
                85                  90                  95

cgt gcc ggc att cag gag gtg cca gcg aat cta tgc cgc caa acg cca     336
Arg Ala Gly Ile Gln Glu Val Pro Ala Asn Leu Cys Arg Gln Thr Pro
            100                 105                 110

aga ctg aag agt tta gat cta tcg agc aat caa att gaa aag att cag     384
Arg Leu Lys Ser Leu Asp Leu Ser Ser Asn Gln Ile Glu Lys Ile Gln
        115                 120                 125

gga aag ccc ttc aat gga ttg aag caa ttg aac gat ctg ttg ctc tcc     432
Gly Lys Pro Phe Asn Gly Leu Lys Gln Leu Asn Asp Leu Leu Leu Ser
    130                 135                 140

tac aat cga atc aag gca ctg cca caa gat gcc ttt cag ggc att ccg     480
Tyr Asn Arg Ile Lys Ala Leu Pro Gln Asp Ala Phe Gln Gly Ile Pro

145                 150                 155                 160

aag ctc caa ctt ctc aag cag ttg ggt gct tcc atg cat gat cct tgg     528
Lys Leu Gln Leu Leu Lys Gln Leu Gly Ala Ser Met His Asp Pro Trp
                165                 170                 175

gag acg gct att aac ttc aac gaa gag cag ctg caa aca caa acg gga     576
Glu Thr Ala Ile Asn Phe Asn Glu Glu Gln Leu Gln Thr Gln Thr Gly
            180                 185                 190

ggc caa atc gcc acc agc tac atg gag gag tac ttc gag gag cac gat     624
Gly Gln Ile Ala Thr Ser Tyr Met Glu Glu Tyr Phe Glu Glu His Asp
        195                 200                 205

gtg agt ggt cct gcc acg gga tac ggc ttt ggt act gga cta ttc tct     672
Val Ser Gly Pro Ala Thr Gly Tyr Gly Phe Gly Thr Gly Leu Phe Ser
    210                 215                 220

ggt atg tcc aca gag gac ttt caa ccg ggt tcg gtg cag tgc ctg cca     720
Gly Met Ser Thr Glu Asp Phe Gln Pro Gly Ser Val Gln Cys Leu Pro
225                 230                 235                 240

atg cca gga cct ttt cta ccc tgc gcc gat ctc ttc gat tgg tgg act     768
Met Pro Gly Pro Phe Leu Pro Cys Ala Asp Leu Phe Asp Trp Trp Thr
                245                 250                 255

ctg cgc tgc gga gtg tgg gtg gtt ttt ctg ttg tct ctc ctg ggc aac     816
Leu Arg Cys Gly Val Trp Val Val Phe Leu Leu Ser Leu Leu Gly Asn
            260                 265                 270

gga acg gtg gtg ttt gtg ctg ctc tgc tcg cga tca aag atg gat gtg     864
Gly Thr Val Val Phe Val Leu Leu Cys Ser Arg Ser Lys Met Asp Val
        275                 280                 285

ccc cga ttt cta gtc tgt aat ctg gcc gcg gcc gac ttc ttc atg ggc     912
Pro Arg Phe Leu Val Cys Asn Leu Ala Ala Ala Asp Phe Phe Met Gly
    290                 295                 300

atc tac ctg ggt att ctg gcc att gtg gat gcg gcg acg ttg ggc gaa     960
Ile Tyr Leu Gly Ile Leu Ala Ile Val Asp Ala Ala Thr Leu Gly Glu
305                 310                 315                 320

ttt cgc atg ttt gcc att cct tgg cag atg tcc gtg ctc tgc cag ttg     1008
Phe Arg Met Phe Ala Ile Pro Trp Gln Met Ser Val Leu Cys Gln Leu
                325                 330                 335

tcc ggt ttt ctg gcc gta ctc agc tca gaa tta tcc gtt tac acg ttg     1056
Ser Gly Phe Leu Ala Val Leu Ser Ser Glu Leu Ser Val Tyr Thr Leu

            340                 345                 350

gct gtg atc act ttg gag cgc aat tac gcc atc act cat gcc att cac 1104
Ala Val Ile Thr Leu Glu Arg Asn Tyr Ala Ile Thr His Ala Ile His
        355                 360                 365

ctg aac aag agg ctc tcc ttg aag cag gcg gga tat ata atg agt gta 1152
Leu Asn Lys Arg Leu Ser Leu Lys Gln Ala Gly Tyr Ile Met Ser Val
    370                 375                 380

gga tgg gtt ttt gcc ctg atc atg gct ttg atg cct ttg gtt ggt gtt 1200
Gly Trp Val Phe Ala Leu Ile Met Ala Leu Met Pro Leu Val Gly Val
385                 390                 395                 400

tcg gac tac agg aag ttt gcc gtg tgt ttg cca ttc gag acc acc act 1248
Ser Asp Tyr Arg Lys Phe Ala Val Cys Leu Pro Phe Glu Thr Thr Thr
                405                 410                 415

gga ccg gcc agt ttg acc tat gta atc tca ctg atg ttc atc aac gga 1296
Gly Pro Ala Ser Leu Thr Tyr Val Ile Ser Leu Met Phe Ile Asn Gly
            420                 425                 430

tgc gca ttt ctc act ctg atg ggt tgc tac ctg aag atg tac tgg gcc 1344
Cys Ala Phe Leu Thr Leu Met Gly Cys Tyr Leu Lys Met Tyr Trp Ala
        435                 440                 445

ata agg ggc agc cag gca tgg aac acg aat gat tcg cga att gcc aag 1392
Ile Arg Gly Ser Gln Ala Trp Asn Thr Asn Asp Ser Arg Ile Ala Lys
    450                 455                 460

cga atg gct ctg ttg gtc ttt act gac ttc ctc tgc tgg tca cct atc 1440
Arg Met Ala Leu Leu Val Phe Thr Asp Phe Leu Cys Trp Ser Pro Ile
465                 470                 475                 480

gcc ttc ttc tcg atc act gcc atc ttt ggc ctg caa ctg att tcg ctg 1488
Ala Phe Phe Ser Ile Thr Ala Ile Phe Gly Leu Gln Leu Ile Ser Leu
                485                 490                 495

gag cag gcc aag atc ttt acg gta ttt gtg ctg ccc ttg aat agt tgc 1536
Glu Gln Ala Lys Ile Phe Thr Val Phe Val Leu Pro Leu Asn Ser Cys
            500                 505                 510

tgc aat ccc ttt ctc tac gcc ata atg acc aag cag ttc aag aag gac 1584
Cys Asn Pro Phe Leu Tyr Ala Ile Met Thr Lys Gln Phe Lys Lys Asp
        515                 520                 525

tgt gtg acg ttg tgc aaa cat ttc gaa gaa tcc cgc gtc gtg gga ggc 1632
Cys Val Thr Leu Cys Lys His Phe Glu Glu Ser Arg Val Val Gly Gly

530                 535                 540

ggt ggt ccg ggt gga cgt ggt gca gtg gca aga acc aag agg ggt gat     1680
Gly Gly Pro Gly Gly Arg Gly Ala Val Ala Arg Thr Lys Arg Gly Asp
545                 550                 555                 560

ctc ccg cca cca ctc tta cca gct gca gcg gtg gca cat ccg cca ggc     1728
Leu Pro Pro Pro Leu Leu Pro Ala Ala Ala Val Ala His Pro Pro Gly
                565                 570                 575

tgt cga tgt cta cgg atg ctg ccc agc gaa atg ccc aac tgg cat aaa     1776
Cys Arg Cys Leu Arg Met Leu Pro Ser Glu Met Pro Asn Trp His Lys
            580                 585                 590

atg gag cag aca ccg agc atg tgg cag aga ttg aga aca ttc tgc tgc     1824
Met Glu Gln Thr Pro Ser Met Trp Gln Arg Leu Arg Thr Phe Cys Cys
        595                 600                 605

ggg gag aac cga agg cgt cgc aaa cag cga cgg caa ccg caa cag cga     1872
Gly Glu Asn Arg Arg Arg Arg Lys Gln Arg Arg Gln Pro Gln Gln Arg
    610                 615                 620

cgc cag aga gcc tat act gcc gca gcc gcc aat ccg tat cag tat cag     1920
Arg Gln Arg Ala Tyr Thr Ala Ala Ala Ala Asn Pro Tyr Gln Tyr Gln
625                 630                 635                 640

ttt gct gaa ctg cgt cag caa cgc cag aat cga gcc agt tcc att tcc     1968
Phe Ala Glu Leu Arg Gln Gln Arg Gln Asn Arg Ala Ser Ser Ile Ser
                645                 650                 655

agc gag aat ttc tgc agc tca aga tca tcc agc tgg cga cat ggt cca     2016
Ser Glu Asn Phe Cys Ser Ser Arg Ser Ser Ser Trp Arg His Gly Pro
            660                 665                 670

cca tcc tca gca ccc gtg cca ccg ggt aac tgc agc atg ccg ctg aag     2064
Pro Ser Ser Ala Pro Val Pro Pro Gly Asn Cys Ser Met Pro Leu Lys
        675                 680                 685

atg ctg gag cca cat gct cat ccg cac ggt cat ggg cgc aga cga cac     2112
Met Leu Glu Pro His Ala His Pro His Gly His Gly Arg Arg Arg His
    690                 695                 700

tcc gcc tgg ctg atc acg cgg aaa acg tcg cag gac tcg aac ttg tcc     2160
Ser Ala Trp Leu Ile Thr Arg Lys Thr Ser Gln Asp Ser Asn Leu Ser
705                 710                 715                 720

agc tcg cgc aat gac tcc tcc gcc tcg gcc acc acc gcc agc acc tcc     2208
Ser Ser Arg Asn Asp Ser Ser Ala Ser Ala Thr Thr Ala Ser Thr Ser

                725                 730                 735

acg ttc cgg tta tcc cgg tca agt gcc ggc agc agc aca ccc ctg ccc 2256
Thr Phe Arg Leu Ser Arg Ser Ser Ala Gly Ser Ser Thr Pro Leu Pro
            740                 745                 750

tcg atc ata gcc cac aac ggg aag gcc cag ttg gat gca gtt aaa cca 2304
Ser Ile Ile Ala His Asn Gly Lys Ala Gln Leu Asp Ala Val Lys Pro
        755                 760                 765

cga ttg gtc cgc cag gaa gca gtc cag gag gaa gag gac tca tca cca 2352
Arg Leu Val Arg Gln Glu Ala Val Gln Glu Glu Glu Asp Ser Ser Pro
    770                 775                 780

cct cgc ctg gga gta cgc ttc cta ccc acc ata ccc tcg gct gcc gac 2400
Pro Arg Leu Gly Val Arg Phe Leu Pro Thr Ile Pro Ser Ala Ala Asp
785                 790                 795                 800

agt tct gtc gtc atg gaa gat ggc gac tcg gcc aac acg gga gta gcc 2448
Ser Ser Val Val Met Glu Asp Gly Asp Ser Ala Asn Thr Gly Val Ala
                805                 810                 815

agt ttt cta ggc atg cca ctt cct ggg gcg tcc agt ggc ttc ctt ata 2496
Ser Phe Leu Gly Met Pro Leu Pro Gly Ala Ser Ser Gly Phe Leu Ile
            820                 825                 830

gct cca acc acc gct gcc acc acc tcc cca ccc ccg gtg gtc ctg cag 2544
Ala Pro Thr Thr Ala Ala Thr Thr Ser Pro Pro Pro Val Val Leu Gln
        835                 840                 845

ccg gca aag cct cct ccc gat ccc aac gat gca cca cta tga 2586
Pro Ala Lys Pro Pro Pro Asp Pro Asn Asp Ala Pro Leu
    850                 855                 860

<210> 20
<211> 861
<212> PRT
<213> Drosophila melanogaster

<400> 20
Met Glu Leu Asp Asp Asn Leu Ile Ser Ser Leu Pro Glu Gly Leu Ser
1               5                   10                  15

Lys Leu Ser Gln Leu Gln Glu Leu Ser Ile Thr Ser Asn Arg Leu Arg
            20                  25                  30

Trp Ile Asn Asp Thr Glu Leu Pro Arg Ser Met Gln Met Leu Asp Met

35                  40                  45

Arg Ala Asn Pro Leu Ser Thr Ile Ser Pro Gly Ala Phe Arg Gly Met
    50                  55                  60

Ser Lys Leu Arg Lys Leu Ile Leu Ser Asp Val Arg Thr Leu Arg Ser
65                  70                  75                  80

Phe Pro Glu Leu Glu Ala Cys His Ala Leu Glu Ile Leu Lys Leu Asp
                85                  90                  95

Arg Ala Gly Ile Gln Glu Val Pro Ala Asn Leu Cys Arg Gln Thr Pro
            100                 105                 110

Arg Leu Lys Ser Leu Asp Leu Ser Ser Asn Gln Ile Glu Lys Ile Gln
        115                 120                 125

Gly Lys Pro Phe Asn Gly Leu Lys Gln Leu Asn Asp Leu Leu Leu Ser
    130                 135                 140

Tyr Asn Arg Ile Lys Ala Leu Pro Gln Asp Ala Phe Gln Gly Ile Pro
145                 150                 155                 160

Lys Leu Gln Leu Leu Lys Gln Leu Gly Ala Ser Met His Asp Pro Trp
                165                 170                 175

Glu Thr Ala Ile Asn Phe Asn Glu Glu Gln Leu Gln Thr Gln Thr Gly
            180                 185                 190

Gly Gln Ile Ala Thr Ser Tyr Met Glu Glu Tyr Phe Glu Glu His Asp
        195                 200                 205

Val Ser Gly Pro Ala Thr Gly Tyr Gly Phe Gly Thr Gly Leu Phe Ser
    210                 215                 220

Gly Met Ser Thr Glu Asp Phe Gln Pro Gly Ser Val Gln Cys Leu Pro
225                 230                 235                 240

Met Pro Gly Pro Phe Leu Pro Cys Ala Asp Leu Phe Asp Trp Trp Thr
                245                 250                 255

Leu Arg Cys Gly Val Trp Val Val Phe Leu Leu Ser Leu Leu Gly Asn
            260                 265                 270

Gly Thr Val Val Phe Val Leu Leu Cys Ser Arg Ser Lys Met Asp Val
        275                 280                 285

Pro Arg Phe Leu Val Cys Asn Leu Ala Ala Ala Asp Phe Phe Met Gly

290                 295                 300

Ile Tyr Leu Gly Ile Leu Ala Ile Val Asp Ala Ala Thr Leu Gly Glu
305                 310                 315                 320

Phe Arg Met Phe Ala Ile Pro Trp Gln Met Ser Val Leu Cys Gln Leu
                325                 330                 335

Ser Gly Phe Leu Ala Val Leu Ser Ser Glu Leu Ser Val Tyr Thr Leu
            340                 345                 350

Ala Val Ile Thr Leu Glu Arg Asn Tyr Ala Ile Thr His Ala Ile His
        355                 360                 365

Leu Asn Lys Arg Leu Ser Leu Lys Gln Ala Gly Tyr Ile Met Ser Val
    370                 375                 380

Gly Trp Val Phe Ala Leu Ile Met Ala Leu Met Pro Leu Val Gly Val
385                 390                 395                 400

Ser Asp Tyr Arg Lys Phe Ala Val Cys Leu Pro Phe Glu Thr Thr Thr
                405                 410                 415

Gly Pro Ala Ser Leu Thr Tyr Val Ile Ser Leu Met Phe Ile Asn Gly
            420                 425                 430

Cys Ala Phe Leu Thr Leu Met Gly Cys Tyr Leu Lys Met Tyr Trp Ala
        435                 440                 445

Ile Arg Gly Ser Gln Ala Trp Asn Thr Asn Asp Ser Arg Ile Ala Lys
    450                 455                 460

Arg Met Ala Leu Leu Val Phe Thr Asp Phe Leu Cys Trp Ser Pro Ile
465                 470                 475                 480

Ala Phe Phe Ser Ile Thr Ala Ile Phe Gly Leu Gln Leu Ile Ser Leu
                485                 490                 495

Glu Gln Ala Lys Ile Phe Thr Val Phe Val Leu Pro Leu Asn Ser Cys
            500                 505                 510

Cys Asn Pro Phe Leu Tyr Ala Ile Met Thr Lys Gln Phe Lys Lys Asp
        515                 520                 525

Cys Val Thr Leu Cys Lys His Phe Glu Glu Ser Arg Val Val Gly Gly
    530                 535                 540

Gly Gly Pro Gly Gly Arg Gly Ala Val Ala Arg Thr Lys Arg Gly Asp

545                 550                 555                 560

Leu Pro Pro Pro Leu Leu Pro Ala Ala Ala Val Ala His Pro Pro Gly
                565                 570                 575

Cys Arg Cys Leu Arg Met Leu Pro Ser Glu Met Pro Asn Trp His Lys
            580                 585                 590

Met Glu Gln Thr Pro Ser Met Trp Gln Arg Leu Arg Thr Phe Cys Cys
        595                 600                 605

Gly Glu Asn Arg Arg Arg Arg Lys Gln Arg Arg Gln Pro Gln Gln Arg
    610                 615                 620

Arg Gln Arg Ala Tyr Thr Ala Ala Ala Ala Asn Pro Tyr Gln Tyr Gln
625                 630                 635                 640

Phe Ala Glu Leu Arg Gln Gln Arg Gln Asn Arg Ala Ser Ser Ile Ser
                645                 650                 655

Ser Glu Asn Phe Cys Ser Ser Arg Ser Ser Ser Trp Arg His Gly Pro
            660                 665                 670

Pro Ser Ser Ala Pro Val Pro Pro Gly Asn Cys Ser Met Pro Leu Lys
        675                 680                 685

Met Leu Glu Pro His Ala His Pro His Gly His Gly Arg Arg Arg His
    690                 695                 700

Ser Ala Trp Leu Ile Thr Arg Lys Thr Ser Gln Asp Ser Asn Leu Ser
705                 710                 715                 720

Ser Ser Arg Asn Asp Ser Ser Ala Ser Ala Thr Thr Ala Ser Thr Ser
                725                 730                 735

Thr Phe Arg Leu Ser Arg Ser Ser Ala Gly Ser Ser Thr Pro Leu Pro
            740                 745                 750

Ser Ile Ile Ala His Asn Gly Lys Ala Gln Leu Asp Ala Val Lys Pro
        755                 760                 765

Arg Leu Val Arg Gln Glu Ala Val Gln Glu Glu Glu Asp Ser Ser Pro
    770                 775                 780

Pro Arg Leu Gly Val Arg Phe Leu Pro Thr Ile Pro Ser Ala Ala Asp
785                 790                 795                 800

Ser Ser Val Val Met Glu Asp Gly Asp Ser Ala Asn Thr Gly Val Ala

805                 810                 815

Ser Phe Leu Gly Met Pro Leu Pro Gly Ala Ser Ser Gly Phe Leu Ile
            820                 825                 830

Ala Pro Thr Thr Ala Ala Thr Thr Ser Pro Pro Pro Val Val Leu Gln
        835                 840                 845

Pro Ala Lys Pro Pro Pro Asp Pro Asn Asp Ala Pro Leu
    850                 855                 860

<210> 21
<211> 606
<212> DNA
<213> Drosophila melanogaster

<220>
<221> CDS
<222> (1)..(603)

<400> 21
atg ttc aac tac gag gag ggg gat gcc gac cag gcg gcc atg gct gca     48
Met Phe Asn Tyr Glu Glu Gly Asp Ala Asp Gln Ala Ala Met Ala Ala
1               5                   10                  15

gcg gct gcc tat agg gca ctg ctc gac tac tat gcc aat gcg cca agt     96
Ala Ala Ala Tyr Arg Ala Leu Leu Asp Tyr Tyr Ala Asn Ala Pro Ser
            20                  25                  30

gcg gcg ggt cac ata gtg tcg ctc aac gtg gca ccc tac aat gga act     144
Ala Ala Gly His Ile Val Ser Leu Asn Val Ala Pro Tyr Asn Gly Thr
        35                  40                  45

gga aac gga ggc act gtc tcc ttg gcg ggc aat gcg aca agc agc tat     192
Gly Asn Gly Gly Thr Val Ser Leu Ala Gly Asn Ala Thr Ser Ser Tyr
    50                  55                  60

ggc gat gat gat agg gat ggc tat atg gac acc gag ccc agt gac ctg     240
Gly Asp Asp Asp Arg Asp Gly Tyr Met Asp Thr Glu Pro Ser Asp Leu
65                  70                  75                  80

gtc acc gaa ctg gcc ttc tcc ctg ggc acc agt tca agt cca agt ccc     288
Val Thr Glu Leu Ala Phe Ser Leu Gly Thr Ser Ser Ser Pro Ser Pro
                85                  90                  95

agt tcc aca ccc gct tcc agc tcc agt act tcc act ggc atg ccc gtc     336

Ser Ser Thr Pro Ala Ser Ser Ser Ser Thr Ser Thr Gly Met Pro Val
            100                 105                 110

tgg ctg ata ccc agc tat agc atg att ctg ctg ttc gcc gtg ctg ggc     384
Trp Leu Ile Pro Ser Tyr Ser Met Ile Leu Leu Phe Ala Val Leu Gly
        115                 120                 125

aac ctg ctg gtc atc tcg acg ctg gtg cag aat cgc cgg atg cgt acc     432
Asn Leu Leu Val Ile Ser Thr Leu Val Gln Asn Arg Arg Met Arg Thr
    130                 135                 140

ata acc aac gtg ttc ctg ctc aac ctg gcc ata tcg gac atg ctg ctg     480
Ile Thr Asn Val Phe Leu Leu Asn Leu Ala Ile Ser Asp Met Leu Leu
145                 150                 155                 160

ggc gtg ctc tgc atg ccc gtc acc ctg gtg ggc acc ctg ctg cga aac     528
Gly Val Leu Cys Met Pro Val Thr Leu Val Gly Thr Leu Leu Arg Asn
                165                 170                 175

ttc atc ttt ggc gag ttc ctc tgc aag ctc ttt cag ttc tcg caa ggt     576
Phe Ile Phe Gly Glu Phe Leu Cys Lys Leu Phe Gln Phe Ser Gln Gly
            180                 185                 190

tct tgg gct ctt ggg ttc ctg cgc att tag                             606
Ser Trp Ala Leu Gly Phe Leu Arg Ile
        195                 200

<210> 22
<211> 201
<212> PRT
<213> Drosophila melanogaster

<400> 22
Met Phe Asn Tyr Glu Glu Gly Asp Ala Asp Gln Ala Ala Met Ala Ala
  1               5                  10                  15

Ala Ala Ala Tyr Arg Ala Leu Leu Asp Tyr Tyr Ala Asn Ala Pro Ser
             20                  25                  30

Ala Ala Gly His Ile Val Ser Leu Asn Val Ala Pro Tyr Asn Gly Thr
         35                  40                  45

Gly Asn Gly Gly Thr Val Ser Leu Ala Gly Asn Ala Thr Ser Ser Tyr
     50                  55                  60

Gly Asp Asp Asp Arg Asp Gly Tyr Met Asp Thr Glu Pro Ser Asp Leu
 65                  70                  75                  80

Val Thr Glu Leu Ala Phe Ser Leu Gly Thr Ser Ser Ser Pro Ser Pro
                85                  90                  95

Ser Ser Thr Pro Ala Ser Ser Ser Ser Thr Ser Thr Gly Met Pro Val
            100                 105                 110

Trp Leu Ile Pro Ser Tyr Ser Met Ile Leu Leu Phe Ala Val Leu Gly
        115                 120                 125

Asn Leu Leu Val Ile Ser Thr Leu Val Gln Asn Arg Arg Met Arg Thr
    130                 135                 140

Ile Thr Asn Val Phe Leu Leu Asn Leu Ala Ile Ser Asp Met Leu Leu
145                 150                 155                 160

Gly Val Leu Cys Met Pro Val Thr Leu Val Gly Thr Leu Leu Arg Asn
                165                 170                 175

Phe Ile Phe Gly Glu Phe Leu Cys Lys Leu Phe Gln Phe Ser Gln Gly
            180                 185                 190

Ser Trp Ala Leu Gly Phe Leu Arg Ile
        195                 200

<210> 23
<211> 1224
<212> DNA
<213> Drosophila melanogaster

<220>
<221> CDS
<222> (1)..(1221)

<400> 23
atg gcc tcc gtg gcc gtt tcg tcc tgg acc ttg gtg gcc ata tcc tgt    48
Met Ala Ser Val Ala Val Ser Ser Trp Thr Leu Val Ala Ile Ser Cys
1               5                   10                  15

gag cgc tac tac gcg ata tgc cat cca ctg cgc tcg cga tcc tgg cag    96
Glu Arg Tyr Tyr Ala Ile Cys His Pro Leu Arg Ser Arg Ser Trp Gln
            20                  25                  30

aca atc agt cac gcc tac aag atc atc ggc ttc atc tgg ctg ggc ggc    144
Thr Ile Ser His Ala Tyr Lys Ile Ile Gly Phe Ile Trp Leu Gly Gly
        35                  40                  45

atc ctc tgc atg acg ccc ata gcg gtc ttt agt caa ttg ata ccc acc 192
Ile Leu Cys Met Thr Pro Ile Ala Val Phe Ser Gln Leu Ile Pro Thr
    50                  55                  60

agt cga ccg ggc tac tgc aag tgc cgt gag ttt tgg ccc gac cag gga 240
Ser Arg Pro Gly Tyr Cys Lys Cys Arg Glu Phe Trp Pro Asp Gln Gly
65                  70                  75                  80

tac gag ctc ttc tac aac atc ctg ctg gac ttc ctg ctg ctc gtc ctg 288
Tyr Glu Leu Phe Tyr Asn Ile Leu Leu Asp Phe Leu Leu Leu Val Leu
                85                  90                  95

ccg ctt ctc gtc ctc tgc gtg gcc tac atc ctc atc acg cgt acc ctg 336
Pro Leu Leu Val Leu Cys Val Ala Tyr Ile Leu Ile Thr Arg Thr Leu
            100                 105                 110

tac gta ggc atg gcc aag gac agc gga cgc atc ctg cag caa tcg ctg 384
Tyr Val Gly Met Ala Lys Asp Ser Gly Arg Ile Leu Gln Gln Ser Leu
        115                 120                 125

cct gtt tcc gct aca acg gcc ggc gga agc gca ccg aat ccg ggc acc 432
Pro Val Ser Ala Thr Thr Ala Gly Gly Ser Ala Pro Asn Pro Gly Thr
    130                 135                 140

agc agc agt agt aac tgc atc ctg gtc ctg acc gcc acc gca gtc tat 480
Ser Ser Ser Ser Asn Cys Ile Leu Val Leu Thr Ala Thr Ala Val Tyr
145                 150                 155                 160

aat ggt tgg cgg agg atc aac caa tat ggc aac gac cac ctt gac aac 528
Asn Gly Trp Arg Arg Ile Asn Gln Tyr Gly Asn Asp His Leu Asp Asn
                165                 170                 175

gag acc aac ggc tcc aac tgt gat cac cac cac cac gac gac cac ggt 576
Glu Thr Asn Gly Ser Asn Cys Asp His His His His Asp Asp His Gly
            180                 185                 190

gac gct ggc caa gac ctc ctc gcc cag cat tcg cgt cca cga tgc ggc 624
Asp Ala Gly Gln Asp Leu Leu Ala Gln His Ser Arg Pro Arg Cys Gly
        195                 200                 205

act tcg cag gta gga ttc gtg tcc gat aac cgg tct gcc aat cct att 672
Thr Ser Gln Val Gly Phe Val Ser Asp Asn Arg Ser Ala Asn Pro Ile
    210                 215                 220

ccc tat tcc cat ttc tcc aca atc cac aat cca cta tcc agg tcc aac 720
Pro Tyr Ser His Phe Ser Thr Ile His Asn Pro Leu Ser Arg Ser Asn
225                 230                 235                 240

gag gcc aag acc ctg gag agc aag aag cgt gtg gtc aag atg ctg ttc    768
Glu Ala Lys Thr Leu Glu Ser Lys Lys Arg Val Val Lys Met Leu Phe
                245                 250                 255

gtc ctg gtg ctg gag ttt ttc atc tgc tgg act ccg ctg tac gtg atc    816
Val Leu Val Leu Glu Phe Phe Ile Cys Trp Thr Pro Leu Tyr Val Ile
            260                 265                 270

aac acg atg gtc atg ctg atc gga ccg gtg gtg tac gag tat gtc gac    864
Asn Thr Met Val Met Leu Ile Gly Pro Val Val Tyr Glu Tyr Val Asp
        275                 280                 285

tac acg gcc atc agt ttc ctc cag ctg ctg gcc tac tca tcc agc tgc    912
Tyr Thr Ala Ile Ser Phe Leu Gln Leu Leu Ala Tyr Ser Ser Ser Cys
    290                 295                 300

tgc aat ccg atc acc tac tgc ttc atg aac gcc agc ttc cgg cgc gcc    960
Cys Asn Pro Ile Thr Tyr Cys Phe Met Asn Ala Ser Phe Arg Arg Ala
305                 310                 315                 320

ttt gtc gac acc ttc aag ggt ctg ccc tgg cgt cgt gga gca ggt gcc    1008
Phe Val Asp Thr Phe Lys Gly Leu Pro Trp Arg Arg Gly Ala Gly Ala
                325                 330                 335

agc gga ggc gtc ggt ggt gct gct ggt gga gga ctc tcc gcc agc cag    1056
Ser Gly Gly Val Gly Gly Ala Ala Gly Gly Gly Leu Ser Ala Ser Gln
            340                 345                 350

gcg ggc gca ggc ccg ggc gcc tat gcg agt gcc aac acc aac att agt    1104
Ala Gly Ala Gly Pro Gly Ala Tyr Ala Ser Ala Asn Thr Asn Ile Ser
        355                 360                 365

ctc aat ccc ggc cta gcc atg ggt atg ggc acc tgg cgg agt cgc tca    1152
Leu Asn Pro Gly Leu Ala Met Gly Met Gly Thr Trp Arg Ser Arg Ser
    370                 375                 380

cgc cac gag ttt ctc aat gcg gtg gtg acc acc aat agt gcc gcc gcc    1200
Arg His Glu Phe Leu Asn Ala Val Val Thr Thr Asn Ser Ala Ala Ala
385                 390                 395                 400

gcc gtc aac agt cct cag ctc taa                                    1224
Ala Val Asn Ser Pro Gln Leu
                405

<210> 24
<211> 407

<212> PRT
<213> Drosophila melanogaster

<400> 24
Met Ala Ser Val Ala Val Ser Ser Trp Thr Leu Val Ala Ile Ser Cys
1               5                   10                  15

Glu Arg Tyr Tyr Ala Ile Cys His Pro Leu Arg Ser Arg Ser Trp Gln
            20                  25                  30

Thr Ile Ser His Ala Tyr Lys Ile Ile Gly Phe Ile Trp Leu Gly Gly
        35                  40                  45

Ile Leu Cys Met Thr Pro Ile Ala Val Phe Ser Gln Leu Ile Pro Thr
    50                  55                  60

Ser Arg Pro Gly Tyr Cys Lys Cys Arg Glu Phe Trp Pro Asp Gln Gly
65                  70                  75                  80

Tyr Glu Leu Phe Tyr Asn Ile Leu Leu Asp Phe Leu Leu Leu Val Leu
                85                  90                  95

Pro Leu Leu Val Leu Cys Val Ala Tyr Ile Leu Ile Thr Arg Thr Leu
            100                 105                 110

Tyr Val Gly Met Ala Lys Asp Ser Gly Arg Ile Leu Gln Gln Ser Leu
        115                 120                 125

Pro Val Ser Ala Thr Thr Ala Gly Gly Ser Ala Pro Asn Pro Gly Thr
    130                 135                 140

Ser Ser Ser Ser Asn Cys Ile Leu Val Leu Thr Ala Thr Ala Val Tyr
145                 150                 155                 160

Asn Gly Trp Arg Arg Ile Asn Gln Tyr Gly Asn Asp His Leu Asp Asn
                165                 170                 175

Glu Thr Asn Gly Ser Asn Cys Asp His His His His Asp Asp His Gly
            180                 185                 190

Asp Ala Gly Gln Asp Leu Leu Ala Gln His Ser Arg Pro Arg Cys Gly
        195                 200                 205

Thr Ser Gln Val Gly Phe Val Ser Asp Asn Arg Ser Ala Asn Pro Ile
    210                 215                 220

Pro Tyr Ser His Phe Ser Thr Ile His Asn Pro Leu Ser Arg Ser Asn
225                 230                 235                 240

Glu Ala Lys Thr Leu Glu Ser Lys Lys Arg Val Val Lys Met Leu Phe
                245                 250                 255

Val Leu Val Leu Glu Phe Phe Ile Cys Trp Thr Pro Leu Tyr Val Ile
            260                 265                 270

Asn Thr Met Val Met Leu Ile Gly Pro Val Val Tyr Glu Tyr Val Asp
        275                 280                 285

Tyr Thr Ala Ile Ser Phe Leu Gln Leu Leu Ala Tyr Ser Ser Ser Cys
    290                 295                 300

Cys Asn Pro Ile Thr Tyr Cys Phe Met Asn Ala Ser Phe Arg Arg Ala
305                 310                 315                 320

Phe Val Asp Thr Phe Lys Gly Leu Pro Trp Arg Arg Gly Ala Gly Ala
                325                 330                 335

Ser Gly Gly Val Gly Gly Ala Ala Gly Gly Gly Leu Ser Ala Ser Gln
            340                 345                 350

Ala Gly Ala Gly Pro Gly Ala Tyr Ala Ser Ala Asn Thr Asn Ile Ser
        355                 360                 365

Leu Asn Pro Gly Leu Ala Met Gly Met Gly Thr Trp Arg Ser Arg Ser
    370                 375                 380

Arg His Glu Phe Leu Asn Ala Val Val Thr Thr Asn Ser Ala Ala Ala
385                 390                 395                 400

Ala Val Asn Ser Pro Gln Leu
                405

<210> 25
<211> 1258
<212> DNA
<213> Drosophila melanogaster

<220>
<221> CDS
<222> (1)..(1257)

<400> 25
atg tcc gct ggc aat atg agc cat gat ctt gga ccg cca cgc gat ccg    48
Met Ser Ala Gly Asn Met Ser His Asp Leu Gly Pro Pro Arg Asp Pro

1                   5                   10                  15

ctg gcc atc gtg ata ccc gta acg gtg gtc tac tcc ctc att ttc ata    96
Leu Ala Ile Val Ile Pro Val Thr Val Val Tyr Ser Leu Ile Phe Ile
                20                  25                  30

acc ggt gta gtg ggc aac ata agt acc tgc att gtg att aag aag aac    144
Thr Gly Val Val Gly Asn Ile Ser Thr Cys Ile Val Ile Lys Lys Asn
            35                  40                  45

cgt tca atg cac acg gcc acg aat tac tac ctc ttt tcg ctg gcc atc    192
Arg Ser Met His Thr Ala Thr Asn Tyr Tyr Leu Phe Ser Leu Ala Ile
    50                  55                  60

tcg gat ttc ctg ctc ctg ttg tcg ggc gtt ccg cag gag gtg tcc tac    240
Ser Asp Phe Leu Leu Leu Leu Ser Gly Val Pro Gln Glu Val Ser Tyr
65                  70                  75                  80

atc tgg tcc aag tac ccg tac gtg ttt ggg gag tac atc tgc atc gga    288
Ile Trp Ser Lys Tyr Pro Tyr Val Phe Gly Glu Tyr Ile Cys Ile Gly
                85                  90                  95

cgt ggt ctg ttg gcg gag aca tcg gcg aat gcc acg gtg cta acg att    336
Arg Gly Leu Leu Ala Glu Thr Ser Ala Asn Ala Thr Val Leu Thr Ile
            100                 105                 110

acg gcc ttc acg gtg gag cgg tat att gcc att tgc cat ccg ttt ctg    384
Thr Ala Phe Thr Val Glu Arg Tyr Ile Ala Ile Cys His Pro Phe Leu
        115                 120                 125

ggc cag gcc atg agt aaa ctc agt cgc gcc att cgc atc atc gtc ctg    432
Gly Gln Ala Met Ser Lys Leu Ser Arg Ala Ile Arg Ile Ile Val Leu
    130                 135                 140

gtt tgg att atg gcc ata gtt acg gcc att ccg cag gct gcc caa ttt    480
Val Trp Ile Met Ala Ile Val Thr Ala Ile Pro Gln Ala Ala Gln Phe
145                 150                 155                 160

gga atc gag cac tat tcg gga gtg gag caa tgc ggc ata gtg cgg gtc    528
Gly Ile Glu His Tyr Ser Gly Val Glu Gln Cys Gly Ile Val Arg Val
                165                 170                 175

ata gtg aag cac tca ttc cag ctg tcc acg ttc ata ttc ttc ctg gct    576
Ile Val Lys His Ser Phe Gln Leu Ser Thr Phe Ile Phe Phe Leu Ala
            180                 185                 190

ccg atg tct att atc cta gtg ttg tac cta ctt atc ggt gtg cac ctg    624
Pro Met Ser Ile Ile Leu Val Leu Tyr Leu Leu Ile Gly Val His Leu

195                 200                 205

tat cga tcc act ttg gtg gag ggt cct gcc tcg gtt gcc aga cgg cag     672
Tyr Arg Ser Thr Leu Val Glu Gly Pro Ala Ser Val Ala Arg Arg Gln
    210                 215                 220

cag ctg aag agt gtg ccc agt gat acg atc cta tat cgc tat ggt gga     720
Gln Leu Lys Ser Val Pro Ser Asp Thr Ile Leu Tyr Arg Tyr Gly Gly
225                 230                 235                 240

tcc ggt acc gct atg agt ttc aac ggc gga gga agt gga gca ggg aca     768
Ser Gly Thr Ala Met Ser Phe Asn Gly Gly Gly Ser Gly Ala Gly Thr
                245                 250                 255

gcg ggc ttg atg ggc ggc tcg ggg gct caa ctt agc tcg gtg aga ggt     816
Ala Gly Leu Met Gly Gly Ser Gly Ala Gln Leu Ser Ser Val Arg Gly
            260                 265                 270

cgg ctc aat cac tat ggc acc cgg cga gta ctc agg atg cta gtg gcc     864
Arg Leu Asn His Tyr Gly Thr Arg Arg Val Leu Arg Met Leu Val Ala
        275                 280                 285

gtg gtg gtg tgc ttc ttc ctc tgc tgg gcc ccc ttc cac gcc cag cga     912
Val Val Val Cys Phe Phe Leu Cys Trp Ala Pro Phe His Ala Gln Arg
    290                 295                 300

ctg att gcc atc tac gcc cct gca cgg ggg gcc aaa ctg cgg gat cag     960
Leu Ile Ala Ile Tyr Ala Pro Ala Arg Gly Ala Lys Leu Arg Asp Gln
305                 310                 315                 320

cac gag ttt gtc tac acg gtg atg acc tat gtc tcc ggt gtc ctc tac    1008
His Glu Phe Val Tyr Thr Val Met Thr Tyr Val Ser Gly Val Leu Tyr
                325                 330                 335

tac ttg tcc acc tgc atc aac ccg ctg tta tat aac att atg agc cac    1056
Tyr Leu Ser Thr Cys Ile Asn Pro Leu Leu Tyr Asn Ile Met Ser His
            340                 345                 350

aag ttc cga gag gca ttc aag gcc gtt ctg ttt ggc aag aag gta tcg    1104
Lys Phe Arg Glu Ala Phe Lys Ala Val Leu Phe Gly Lys Lys Val Ser
        355                 360                 365

aag ggt tcg ctg aat tcg cga aac aac atc gaa tcg cgc cgc ctg agg    1152
Lys Gly Ser Leu Asn Ser Arg Asn Asn Ile Glu Ser Arg Arg Leu Arg
    370                 375                 380

agg gca cta acc aat tcc agt caa acg cag cgc ttc tcc att gag tcg    1200
Arg Ala Leu Thr Asn Ser Ser Gln Thr Gln Arg Phe Ser Ile Glu Ser

385                 390                 395                 400

gcg gag cag ccg aaa ccg tcg ata atg cag gca agt gcc ccc tat aat    1248
Ala Glu Gln Pro Lys Pro Ser Ile Met Gln Ala Ser Ala Pro Tyr Asn
                405                 410                 415

caa tgg ata g                                                      1258
Gln Trp Ile

<210> 26
<211> 419
<212> PRT
<213> Drosophila melanogaster

<400> 26
Met Ser Ala Gly Asn Met Ser His Asp Leu Gly Pro Pro Arg Asp Pro
1               5                   10                  15

Leu Ala Ile Val Ile Pro Val Thr Val Val Tyr Ser Leu Ile Phe Ile
            20                  25                  30

Thr Gly Val Val Gly Asn Ile Ser Thr Cys Ile Val Ile Lys Lys Asn
        35                  40                  45

Arg Ser Met His Thr Ala Thr Asn Tyr Tyr Leu Phe Ser Leu Ala Ile
    50                  55                  60

Ser Asp Phe Leu Leu Leu Leu Ser Gly Val Pro Gln Glu Val Ser Tyr
65                  70                  75                  80

Ile Trp Ser Lys Tyr Pro Tyr Val Phe Gly Glu Tyr Ile Cys Ile Gly
                85                  90                  95

Arg Gly Leu Leu Ala Glu Thr Ser Ala Asn Ala Thr Val Leu Thr Ile
            100                 105                 110

Thr Ala Phe Thr Val Glu Arg Tyr Ile Ala Ile Cys His Pro Phe Leu
        115                 120                 125

Gly Gln Ala Met Ser Lys Leu Ser Arg Ala Ile Arg Ile Ile Val Leu
    130                 135                 140

Val Trp Ile Met Ala Ile Val Thr Ala Ile Pro Gln Ala Ala Gln Phe
145                 150                 155                 160

Gly Ile Glu His Tyr Ser Gly Val Glu Gln Cys Gly Ile Val Arg Val
                165                 170                 175

Ile Val Lys His Ser Phe Gln Leu Ser Thr Phe Ile Phe Phe Leu Ala
            180                 185                 190

Pro Met Ser Ile Ile Leu Val Leu Tyr Leu Leu Ile Gly Val His Leu
        195                 200                 205

Tyr Arg Ser Thr Leu Val Glu Gly Pro Ala Ser Val Ala Arg Arg Gln
    210                 215                 220

Gln Leu Lys Ser Val Pro Ser Asp Thr Ile Leu Tyr Arg Tyr Gly Gly
225                 230                 235                 240

Ser Gly Thr Ala Met Ser Phe Asn Gly Gly Gly Ser Gly Ala Gly Thr
                245                 250                 255

Ala Gly Leu Met Gly Gly Ser Gly Ala Gln Leu Ser Ser Val Arg Gly
            260                 265                 270

Arg Leu Asn His Tyr Gly Thr Arg Arg Val Leu Arg Met Leu Val Ala
        275                 280                 285

Val Val Val Cys Phe Phe Leu Cys Trp Ala Pro Phe His Ala Gln Arg
    290                 295                 300

Leu Ile Ala Ile Tyr Ala Pro Ala Arg Gly Ala Lys Leu Arg Asp Gln
305                 310                 315                 320

His Glu Phe Val Tyr Thr Val Met Thr Tyr Val Ser Gly Val Leu Tyr
                325                 330                 335

Tyr Leu Ser Thr Cys Ile Asn Pro Leu Leu Tyr Asn Ile Met Ser His
            340                 345                 350

Lys Phe Arg Glu Ala Phe Lys Ala Val Leu Phe Gly Lys Lys Val Ser
        355                 360                 365

Lys Gly Ser Leu Asn Ser Arg Asn Asn Ile Glu Ser Arg Arg Leu Arg
    370                 375                 380

Arg Ala Leu Thr Asn Ser Ser Gln Thr Gln Arg Phe Ser Ile Glu Ser
385                 390                 395                 400

Ala Glu Gln Pro Lys Pro Ser Ile Met Gln Ala Ser Ala Pro Tyr Asn
                405                 410                 415

Gln Trp Ile

<210> 27
<211> 1602
<212> DNA
<213> Drosophila melanogaster

<220>
<221> CDS
<222> (1)..(1599)

<400> 27
atg gaa ggt gga tgg tgg cga gga gga gga gga gga gga cgt ttg ggc     48
Met Glu Gly Gly Trp Trp Arg Gly Gly Gly Gly Gly Gly Arg Leu Gly
1               5                   10                  15

ggg aaa gcc atc atg gaa gga cac tcg aca cca aat ggg gca gct gca     96
Gly Lys Ala Ile Met Glu Gly His Ser Thr Pro Asn Gly Ala Ala Ala
            20                  25                  30

agc cac aga aac aac agc aca agg acc aat atc gcg acc aac ggc tgt     144
Ser His Arg Asn Asn Ser Thr Arg Thr Asn Ile Ala Thr Asn Gly Cys
        35                  40                  45

gcc cat tcg ggc atc ctg tta ttt gtg ctg aca gct atg aca cta acg     192
Ala His Ser Gly Ile Leu Leu Phe Val Leu Thr Ala Met Thr Leu Thr
    50                  55                  60

agc tta ata acg ccc aca gag cag ctg gca gtg gcg cca aat ggc acc     240
Ser Leu Ile Thr Pro Thr Glu Gln Leu Ala Val Ala Pro Asn Gly Thr
65                  70                  75                  80

aca ctg cat caa ctg gag tcc gtg gag tcc gag tcg tat cca tcc ata     288
Thr Leu His Gln Leu Glu Ser Val Glu Ser Glu Ser Tyr Pro Ser Ile
                85                  90                  95

aat ggc act cag aat gaa aca atg gtg acc agt gtg cga ccc cac ctg     336
Asn Gly Thr Gln Asn Glu Thr Met Val Thr Ser Val Arg Pro His Leu
            100                 105                 110

gac cac agg aat cga ccg acg cag cag aat ggc agt cac tat ttg gag     384
Asp His Arg Asn Arg Pro Thr Gln Gln Asn Gly Ser His Tyr Leu Glu
        115                 120                 125

tac gac gat gat ggc ccg gac tgt tcg tac agc tac aac ttc atc ctg     432
Tyr Asp Asp Asp Gly Pro Asp Cys Ser Tyr Ser Tyr Asn Phe Ile Leu
    130                 135                 140

aag ctc atc acg atg atc ctg tac gca ctg gtc tgc atc att gga ctc    480
Lys Leu Ile Thr Met Ile Leu Tyr Ala Leu Val Cys Ile Ile Gly Leu
145                 150                 155                 160

ttt ggc aac acc ctg gtg atc tat gtg gtg atg agg ttc tcc aag atg    528
Phe Gly Asn Thr Leu Val Ile Tyr Val Val Met Arg Phe Ser Lys Met
                165                 170                 175

cag acc gta acc aac ata tac ata ctg aat ctg gct atc gcg gat gag    576
Gln Thr Val Thr Asn Ile Tyr Ile Leu Asn Leu Ala Ile Ala Asp Glu
            180                 185                 190

tgc ttc ctg atc ggc att ccc ttc ctg ctc tac aca atg cag gtg ggc    624
Cys Phe Leu Ile Gly Ile Pro Phe Leu Leu Tyr Thr Met Gln Val Gly
        195                 200                 205

aac tgg ccc ttc ggc aac tat atg tgc aag gcc tac atg gtg agc acc    672
Asn Trp Pro Phe Gly Asn Tyr Met Cys Lys Ala Tyr Met Val Ser Thr
    210                 215                 220

tcg atc acc tcc ttc acc tcc tcg atc ttc ctg ttg atc atg tcg gcg    720
Ser Ile Thr Ser Phe Thr Ser Ser Ile Phe Leu Leu Ile Met Ser Ala
225                 230                 235                 240

gat cgc tac ata gcc gtt tgc cat ccc ata tcc tcg cct cgc tac cga    768
Asp Arg Tyr Ile Ala Val Cys His Pro Ile Ser Ser Pro Arg Tyr Arg
                245                 250                 255

acg ccc ttt gta tcc aag ttg gtt tcg gcc ttc gcc tgg atg aca tcc    816
Thr Pro Phe Val Ser Lys Leu Val Ser Ala Phe Ala Trp Met Thr Ser
            260                 265                 270

gtg ctg ctg atg ctg ccg gtt atc ctt ttt gcc agc acc gtg cag tcg    864
Val Leu Leu Met Leu Pro Val Ile Leu Phe Ala Ser Thr Val Gln Ser
        275                 280                 285

agc aac ggc aat gtg tcc tgc aac atc gag tgg cca gac act cag aac    912
Ser Asn Gly Asn Val Ser Cys Asn Ile Glu Trp Pro Asp Thr Gln Asn
    290                 295                 300

tcg cac acc gac tcc acc ttc att ttg tac tcg ctg gtc ttg gga ttc    960
Ser His Thr Asp Ser Thr Phe Ile Leu Tyr Ser Leu Val Leu Gly Phe
305                 310                 315                 320

gcc act cca ctg act ttt atc ctg gtg ttc tac tgc ctg gtg atc agg    1008
Ala Thr Pro Leu Thr Phe Ile Leu Val Phe Tyr Cys Leu Val Ile Arg
                325                 330                 335

aaa ctt cac acc gtg gga ccg aag cac aag tct aag gag aag aag cgc 1056
Lys Leu His Thr Val Gly Pro Lys His Lys Ser Lys Glu Lys Lys Arg
            340                 345                 350

tct cac agg aag gtc acc aag ttg gtg ctc acg gtg gct ttg atc agc 1104
Ser His Arg Lys Val Thr Lys Leu Val Leu Thr Val Ala Leu Ile Ser
        355                 360                 365

tcc gct cct caa cgc tgt gca tct cgt ctg gag ctg gcc gtc ttc ctg 1152
Ser Ala Pro Gln Arg Cys Ala Ser Arg Leu Glu Leu Ala Val Phe Leu
    370                 375                 380

gcc tgc gga tgc ctc agc tac tcc aac tcg gcc atg aac cca ata ctg 1200
Ala Cys Gly Cys Leu Ser Tyr Ser Asn Ser Ala Met Asn Pro Ile Leu
385                 390                 395                 400

tac gcc ttt ttg agc gat aac ttc aag aag agc ttc atg aag gcc tgc 1248
Tyr Ala Phe Leu Ser Asp Asn Phe Lys Lys Ser Phe Met Lys Ala Cys
                405                 410                 415

acg tgt gct gcc cgc aag gat gtg aat gcc cag ttg cag ctg gag aac 1296
Thr Cys Ala Ala Arg Lys Asp Val Asn Ala Gln Leu Gln Leu Glu Asn
            420                 425                 430

agt ttc ttc ccc aag ttt ggc aag ggc agg caa tcg gag cgt ctt ctt 1344
Ser Phe Phe Pro Lys Phe Gly Lys Gly Arg Gln Ser Glu Arg Leu Leu
        435                 440                 445

ggt ggc aat gga aaa ggt ggc gcc cag cgt ggg gca tta acc aag aag 1392
Gly Gly Asn Gly Lys Gly Gly Ala Gln Arg Gly Ala Leu Thr Lys Lys
    450                 455                 460

aag tgc ttg gcg acg aga aac aac aat gct ccg atg gcc act aca acg 1440
Lys Cys Leu Ala Thr Arg Asn Asn Asn Ala Pro Met Ala Thr Thr Thr
465                 470                 475                 480

acg acg aca acc acc aca acg ggc acg gat gca gtg acc tgt ctc cag 1488
Thr Thr Thr Thr Thr Thr Thr Gly Thr Asp Ala Val Thr Cys Leu Gln
                485                 490                 495

ccg cca gta cac cag gtg cca gcc gag atc cag gtg gga aat ccg gcc 1536
Pro Pro Val His Gln Val Pro Ala Glu Ile Gln Val Gly Asn Pro Ala
            500                 505                 510

acc gtg ctg gtg gtc aat gct gag acc aac aac tgt aaa ccg ccc gtg 1584
Thr Val Leu Val Val Asn Ala Glu Thr Asn Asn Cys Lys Pro Pro Val
        515                 520                 525

ctc cac acg gac tta taa                                                1602
Leu His Thr Asp Leu
    530

<210> 28
<211> 533
<212> PRT
<213> Drosophila melanogaster

<400> 28
Met Glu Gly Gly Trp Trp Arg Gly Gly Gly Gly Gly Gly Arg Leu Gly
  1               5                  10                  15

Gly Lys Ala Ile Met Glu Gly His Ser Thr Pro Asn Gly Ala Ala Ala
             20                  25                  30

Ser His Arg Asn Asn Ser Thr Arg Thr Asn Ile Ala Thr Asn Gly Cys
         35                  40                  45

Ala His Ser Gly Ile Leu Leu Phe Val Leu Thr Ala Met Thr Leu Thr
     50                  55                  60

Ser Leu Ile Thr Pro Thr Glu Gln Leu Ala Val Ala Pro Asn Gly Thr
 65                  70                  75                  80

Thr Leu His Gln Leu Glu Ser Val Glu Ser Glu Ser Tyr Pro Ser Ile
                 85                  90                  95

Asn Gly Thr Gln Asn Glu Thr Met Val Thr Ser Val Arg Pro His Leu
            100                 105                 110

Asp His Arg Asn Arg Pro Thr Gln Gln Asn Gly Ser His Tyr Leu Glu
        115                 120                 125

Tyr Asp Asp Asp Gly Pro Asp Cys Ser Tyr Ser Tyr Asn Phe Ile Leu
    130                 135                 140

Lys Leu Ile Thr Met Ile Leu Tyr Ala Leu Val Cys Ile Ile Gly Leu
145                 150                 155                 160

Phe Gly Asn Thr Leu Val Ile Tyr Val Val Met Arg Phe Ser Lys Met
                165                 170                 175

Gln Thr Val Thr Asn Ile Tyr Ile Leu Asn Leu Ala Ile Ala Asp Glu
            180                 185                 190

Cys Phe Leu Ile Gly Ile Pro Phe Leu Leu Tyr Thr Met Gln Val Gly
        195                 200                 205

Asn Trp Pro Phe Gly Asn Tyr Met Cys Lys Ala Tyr Met Val Ser Thr
    210                 215                 220

Ser Ile Thr Ser Phe Thr Ser Ser Ile Phe Leu Leu Ile Met Ser Ala
225                 230                 235                 240

Asp Arg Tyr Ile Ala Val Cys His Pro Ile Ser Ser Pro Arg Tyr Arg
                245                 250                 255

Thr Pro Phe Val Ser Lys Leu Val Ser Ala Phe Ala Trp Met Thr Ser
            260                 265                 270

Val Leu Leu Met Leu Pro Val Ile Leu Phe Ala Ser Thr Val Gln Ser
        275                 280                 285

Ser Asn Gly Asn Val Ser Cys Asn Ile Glu Trp Pro Asp Thr Gln Asn
    290                 295                 300

Ser His Thr Asp Ser Thr Phe Ile Leu Tyr Ser Leu Val Leu Gly Phe
305                 310                 315                 320

Ala Thr Pro Leu Thr Phe Ile Leu Val Phe Tyr Cys Leu Val Ile Arg
                325                 330                 335

Lys Leu His Thr Val Gly Pro Lys His Lys Ser Lys Glu Lys Lys Arg
            340                 345                 350

Ser His Arg Lys Val Thr Lys Leu Val Leu Thr Val Ala Leu Ile Ser
        355                 360                 365

Ser Ala Pro Gln Arg Cys Ala Ser Arg Leu Glu Leu Ala Val Phe Leu
    370                 375                 380

Ala Cys Gly Cys Leu Ser Tyr Ser Asn Ser Ala Met Asn Pro Ile Leu
385                 390                 395                 400

Tyr Ala Phe Leu Ser Asp Asn Phe Lys Lys Ser Phe Met Lys Ala Cys
                405                 410                 415

Thr Cys Ala Ala Arg Lys Asp Val Asn Ala Gln Leu Gln Leu Glu Asn
            420                 425                 430

Ser Phe Phe Pro Lys Phe Gly Lys Gly Arg Gln Ser Glu Arg Leu Leu
        435                 440                 445

Gly Gly Asn Gly Lys Gly Gly Ala Gln Arg Gly Ala Leu Thr Lys Lys
    450                 455                 460

Lys Cys Leu Ala Thr Arg Asn Asn Asn Ala Pro Met Ala Thr Thr Thr
465                 470                 475                 480

Thr Thr Thr Thr Thr Thr Thr Gly Thr Asp Ala Val Thr Cys Leu Gln
                485                 490                 495

Pro Pro Val His Gln Val Pro Ala Glu Ile Gln Val Gly Asn Pro Ala
            500                 505                 510

Thr Val Leu Val Val Asn Ala Glu Thr Asn Asn Cys Lys Pro Pro Val
        515                 520                 525

Leu His Thr Asp Leu
    530

<210> 29
<211> 1347
<212> DNA
<213> Drosophila melanogaster

<220>
<221> CDS
<222> (1)..(1344)

<400> 29
atg ttt cta tat aag aat tgt tac aat ttt aga ttt gag tgc tat gaa      48
Met Phe Leu Tyr Lys Asn Cys Tyr Asn Phe Arg Phe Glu Cys Tyr Glu
  1               5                  10                  15

ttg aat ttc gaa tgc ctg gag tat aag ttc tat aaa tta aat acc aag      96
Leu Asn Phe Glu Cys Leu Glu Tyr Lys Phe Tyr Lys Leu Asn Thr Lys
             20                  25                  30

cca cgc att ttg agc tgt gaa act gcc tta aaa gtc gaa aga aat att     144
Pro Arg Ile Leu Ser Cys Glu Thr Ala Leu Lys Val Glu Arg Asn Ile
         35                  40                  45

ttt cga agt gca ggt cct tca tct tgg cag caa ttt cct gct gga ata     192
Phe Arg Ser Ala Gly Pro Ser Ser Trp Gln Gln Phe Pro Ala Gly Ile
     50                  55                  60

acg gtg gct gtc tgt ctt cca tca cgt ctg ggt gtg tgg acc gaa cag     240
Thr Val Ala Val Cys Leu Pro Ser Arg Leu Gly Val Trp Thr Glu Gln

65                  70                  75                  80

ttt gct gtg ctc tgg atc ctg ttc acc gtc atc gtt ctg ggc aat tca     288
Phe Ala Val Leu Trp Ile Leu Phe Thr Val Ile Val Leu Gly Asn Ser
                85                  90                  95

gct gtt ctg ttc gtg atg ttc atc aac aag aat cgc aag tcg cgg atg     336
Ala Val Leu Phe Val Met Phe Ile Asn Lys Asn Arg Lys Ser Arg Met
            100                 105                 110

aac tac ttc att aaa cag ctg gca ttg gca gat ctg tgc gtg gga ctg     384
Asn Tyr Phe Ile Lys Gln Leu Ala Leu Ala Asp Leu Cys Val Gly Leu
        115                 120                 125

ctc aac gtc ctc acc gac atc ata tgg cgc atc acg att tcg tgg cgg     432
Leu Asn Val Leu Thr Asp Ile Ile Trp Arg Ile Thr Ile Ser Trp Arg
    130                 135                 140

gca ggc aac ctg gcc tgc aag gcc atc cgc ttc tcg cag gtc tgc gtc     480
Ala Gly Asn Leu Ala Cys Lys Ala Ile Arg Phe Ser Gln Val Cys Val
145                 150                 155                 160

aca tac tcg tcc acc tac gtg ctg gtg gcc atg agc atc gac aga tac     528
Thr Tyr Ser Ser Thr Tyr Val Leu Val Ala Met Ser Ile Asp Arg Tyr
                165                 170                 175

gat gcc atc aca cac ccc atg aac ttc tca aag tcg tgg aaa aga gcc     576
Asp Ala Ile Thr His Pro Met Asn Phe Ser Lys Ser Trp Lys Arg Ala
            180                 185                 190

cgt cac ctg gtg gct ggc gca tgg ctc atc tcg gcg ttg ttt tcg ctt     624
Arg His Leu Val Ala Gly Ala Trp Leu Ile Ser Ala Leu Phe Ser Leu
        195                 200                 205

ccc atc ctg gtt ttg tac gag gag aag ctc atc caa gga cat ccg caa     672
Pro Ile Leu Val Leu Tyr Glu Glu Lys Leu Ile Gln Gly His Pro Gln
    210                 215                 220

tgc tgg att gag ttg ggt tca ccg atc gcc tgg cag gtg tac atg agc     720
Cys Trp Ile Glu Leu Gly Ser Pro Ile Ala Trp Gln Val Tyr Met Ser
225                 230                 235                 240

ctg gtg tcg gcc act cta ttt gcc att cct gcg ctg atc ata tct gcc     768
Leu Val Ser Ala Thr Leu Phe Ala Ile Pro Ala Leu Ile Ile Ser Ala
                245                 250                 255

tgc tat gcg atc atc gta aag acg att tgg gca aag ggt tcc att ttt     816
Cys Tyr Ala Ile Ile Val Lys Thr Ile Trp Ala Lys Gly Ser Ile Phe

            260                 265                 270

gta ccc acg gaa cgt gct ggt ttt gga gct gca cct gcc agg agg gcc 864
Val Pro Thr Glu Arg Ala Gly Phe Gly Ala Ala Pro Ala Arg Arg Ala
        275                 280                 285

agc tcg agg ggc att att cca cgg gca aag gtc aaa acg gtc aag atg 912
Ser Ser Arg Gly Ile Ile Pro Arg Ala Lys Val Lys Thr Val Lys Met
    290                 295                 300

aca ttg acc atc gtg ttt gtg ttc atc atc tgc tgg tcg ccg tat atc 960
Thr Leu Thr Ile Val Phe Val Phe Ile Ile Cys Trp Ser Pro Tyr Ile
305                 310                 315                 320

atc ttc gat ctg ctg cag gtc ttt ggc cag att cca cac tca cag acc 1008
Ile Phe Asp Leu Leu Gln Val Phe Gly Gln Ile Pro His Ser Gln Thr
                325                 330                 335

aac att gcc atc gcc acc ttc atc caa agt ctg gca ccg ctg aac tcg 1056
Asn Ile Ala Ile Ala Thr Phe Ile Gln Ser Leu Ala Pro Leu Asn Ser
            340                 345                 350

gcg gcg aat cca cta atc tat tgc ctc ttc tca tcg cag gtc ttt cgc 1104
Ala Ala Asn Pro Leu Ile Tyr Cys Leu Phe Ser Ser Gln Val Phe Arg
        355                 360                 365

aca tta agt cgc ttt ccg cct ttt aag tgg ttc aca tgc tgc tgc aag 1152
Thr Leu Ser Arg Phe Pro Pro Phe Lys Trp Phe Thr Cys Cys Cys Lys
    370                 375                 380

tca tac cgc aac aac tcg cag caa aac cgc tgc cac acg gtt ggt cgt 1200
Ser Tyr Arg Asn Asn Ser Gln Gln Asn Arg Cys His Thr Val Gly Arg
385                 390                 395                 400

cgg ctt cac aac agt tgc gat tcg atg agg aca ctg acc act tcg ttg 1248
Arg Leu His Asn Ser Cys Asp Ser Met Arg Thr Leu Thr Thr Ser Leu
                405                 410                 415

acg gtt tcc cga agg tcc acc aac aag acg aac gcc cgt gtg gta atc 1296
Thr Val Ser Arg Arg Ser Thr Asn Lys Thr Asn Ala Arg Val Val Ile
            420                 425                 430

tgc gaa cgt ccc acc aag gtg gtt acc gtg cca gcc atg tcg gag gta 1344
Cys Glu Arg Pro Thr Lys Val Val Thr Val Pro Ala Met Ser Glu Val
        435                 440                 445

tga 1347

<210> 30
<211> 448
<212> PRT
<213> Drosophila melanogaster

<400> 30
Met Phe Leu Tyr Lys Asn Cys Tyr Asn Phe Arg Phe Glu Cys Tyr Glu
1               5                   10                  15

Leu Asn Phe Glu Cys Leu Glu Tyr Lys Phe Tyr Lys Leu Asn Thr Lys
            20                  25                  30

Pro Arg Ile Leu Ser Cys Glu Thr Ala Leu Lys Val Glu Arg Asn Ile
        35                  40                  45

Phe Arg Ser Ala Gly Pro Ser Ser Trp Gln Gln Phe Pro Ala Gly Ile
    50                  55                  60

Thr Val Ala Val Cys Leu Pro Ser Arg Leu Gly Val Trp Thr Glu Gln
65                  70                  75                  80

Phe Ala Val Leu Trp Ile Leu Phe Thr Val Ile Val Leu Gly Asn Ser
                85                  90                  95

Ala Val Leu Phe Val Met Phe Ile Asn Lys Asn Arg Lys Ser Arg Met
            100                 105                 110

Asn Tyr Phe Ile Lys Gln Leu Ala Leu Ala Asp Leu Cys Val Gly Leu
        115                 120                 125

Leu Asn Val Leu Thr Asp Ile Ile Trp Arg Ile Thr Ile Ser Trp Arg
    130                 135                 140

Ala Gly Asn Leu Ala Cys Lys Ala Ile Arg Phe Ser Gln Val Cys Val
145                 150                 155                 160

Thr Tyr Ser Ser Thr Tyr Val Leu Val Ala Met Ser Ile Asp Arg Tyr
                165                 170                 175

Asp Ala Ile Thr His Pro Met Asn Phe Ser Lys Ser Trp Lys Arg Ala
            180                 185                 190

Arg His Leu Val Ala Gly Ala Trp Leu Ile Ser Ala Leu Phe Ser Leu
        195                 200                 205

Pro Ile Leu Val Leu Tyr Glu Glu Lys Leu Ile Gln Gly His Pro Gln
    210                 215                 220

Cys Trp Ile Glu Leu Gly Ser Pro Ile Ala Trp Gln Val Tyr Met Ser
225                 230                 235                 240

Leu Val Ser Ala Thr Leu Phe Ala Ile Pro Ala Leu Ile Ile Ser Ala
                245                 250                 255

Cys Tyr Ala Ile Ile Val Lys Thr Ile Trp Ala Lys Gly Ser Ile Phe
            260                 265                 270

Val Pro Thr Glu Arg Ala Gly Phe Gly Ala Ala Pro Ala Arg Arg Ala
        275                 280                 285

Ser Ser Arg Gly Ile Ile Pro Arg Ala Lys Val Lys Thr Val Lys Met
    290                 295                 300

Thr Leu Thr Ile Val Phe Val Phe Ile Ile Cys Trp Ser Pro Tyr Ile
305                 310                 315                 320

Ile Phe Asp Leu Leu Gln Val Phe Gly Gln Ile Pro His Ser Gln Thr
                325                 330                 335

Asn Ile Ala Ile Ala Thr Phe Ile Gln Ser Leu Ala Pro Leu Asn Ser
            340                 345                 350

Ala Ala Asn Pro Leu Ile Tyr Cys Leu Phe Ser Ser Gln Val Phe Arg
        355                 360                 365

Thr Leu Ser Arg Phe Pro Pro Phe Lys Trp Phe Thr Cys Cys Cys Lys
    370                 375                 380

Ser Tyr Arg Asn Asn Ser Gln Gln Asn Arg Cys His Thr Val Gly Arg
385                 390                 395                 400

Arg Leu His Asn Ser Cys Asp Ser Met Arg Thr Leu Thr Thr Ser Leu
                405                 410                 415

Thr Val Ser Arg Arg Ser Thr Asn Lys Thr Asn Ala Arg Val Val Ile
            420                 425                 430

Cys Glu Arg Pro Thr Lys Val Val Thr Val Pro Ala Met Ser Glu Val
        435                 440                 445

<210> 31
<211> 1449
<212> DNA

<213> Drosophila melanogaster

<220>
<221> CDS
<222> (1)..(1446)

<400> 31
atg aac gag ggc gtt att gac atg cga aac gtg gac gag gag ctg gcg     48
Met Asn Glu Gly Val Ile Asp Met Arg Asn Val Asp Glu Glu Leu Ala
1               5                   10                  15

gag aag gag gag ctg atg gcc acc gtc gtc agt gcc acg atg gcc aca     96
Glu Lys Glu Glu Leu Met Ala Thr Val Val Ser Ala Thr Met Ala Thr
            20                  25                  30

aat cag gag gag aac cga ctc ttt tgc ccc ctg aac ttt gac gga tat     144
Asn Gln Glu Glu Asn Arg Leu Phe Cys Pro Leu Asn Phe Asp Gly Tyr
        35                  40                  45

ctc tgc tgg cca agg act ccg gcc ggc act gtg ctg agt caa tat tgt     192
Leu Cys Trp Pro Arg Thr Pro Ala Gly Thr Val Leu Ser Gln Tyr Cys
    50                  55                  60

ccc gat ttc gtc gaa gga ttc aac aga aaa ttt ctg gcc cac aag acc     240
Pro Asp Phe Val Glu Gly Phe Asn Arg Lys Phe Leu Ala His Lys Thr
65                  70                  75                  80

tgc ctg gag aac ggc tcc tgg tac cgt cat ccg gtt agc aat cag acc     288
Cys Leu Glu Asn Gly Ser Trp Tyr Arg His Pro Val Ser Asn Gln Thr
                85                  90                  95

tgg tcc aat tac acc aac tgc gtg gac tat gag gac ctg gag ttt cgc     336
Trp Ser Asn Tyr Thr Asn Cys Val Asp Tyr Glu Asp Leu Glu Phe Arg
            100                 105                 110

cag ttc atc aac gag ctg tac gtg aag ggt tat gcc ctc tcc ctg ctg     384
Gln Phe Ile Asn Glu Leu Tyr Val Lys Gly Tyr Ala Leu Ser Leu Leu
        115                 120                 125

gcc ctg ctc ata tcg att att att ttc ttg ggc ttc aaa tcc ctg cgc     432
Ala Leu Leu Ile Ser Ile Ile Ile Phe Leu Gly Phe Lys Ser Leu Arg
    130                 135                 140

tgc acc cgc atc cgc atc cat gta cat ctg ttc gcc tcg ctg gcc tgc     480
Cys Thr Arg Ile Arg Ile His Val His Leu Phe Ala Ser Leu Ala Cys
145                 150                 155                 160

acc tgt gtg gcc tgg ata tta tgg tat cgt ctg gtg gtg gag cgt tcc     528

Thr Cys Val Ala Trp Ile Leu Trp Tyr Arg Leu Val Val Glu Arg Ser
                165                 170                 175

gaa acc ata gcc gaa aat ccg gta aga tgc act gag aaa agt aac tat     576
Glu Thr Ile Ala Glu Asn Pro Val Arg Cys Thr Glu Lys Ser Asn Tyr
            180                 185                 190

gaa aat aac ata gcc cta caa gta gct agg caa tta tac aaa acc ata     624
Glu Asn Asn Ile Ala Leu Gln Val Ala Arg Gln Leu Tyr Lys Thr Ile
        195                 200                 205

aac ata act aac ttt cag ctg tgg tgc att ggg ctg cat ctg gtg gtg     672
Asn Ile Thr Asn Phe Gln Leu Trp Cys Ile Gly Leu His Leu Val Val
    210                 215                 220

cac tac ttt atg ttg gtc aac tat ttc tgg atg ttt tgc gag ggc ctg     720
His Tyr Phe Met Leu Val Asn Tyr Phe Trp Met Phe Cys Glu Gly Leu
225                 230                 235                 240

cac ctg cac ctg gtg ctc gtt gtg gtg agt tat cct tac tcg tct ggc     768
His Leu His Leu Val Leu Val Val Val Ser Tyr Pro Tyr Ser Ser Gly
                245                 250                 255

aat ctg gca att gcc tgc aca agt ctg cat tct tta tgg gcg act tct     816
Asn Leu Ala Ile Ala Cys Thr Ser Leu His Ser Leu Trp Ala Thr Ser
            260                 265                 270

gtg cat ggc aga tgt gat ttc ctg cag ccg ctg aca tgg caa tgg ttc     864
Val His Gly Arg Cys Asp Phe Leu Gln Pro Leu Thr Trp Gln Trp Phe
        275                 280                 285

acc ccc agc aat ttg tct gag agc aaa tcg caa cgt tgg cca act gct     912
Thr Pro Ser Asn Leu Ser Glu Ser Lys Ser Gln Arg Trp Pro Thr Ala
    290                 295                 300

tta cgt ctg cgt caa gtt gag atg tca gga atg tcc gtt cag atc ctg     960
Leu Arg Leu Arg Gln Val Glu Met Ser Gly Met Ser Val Gln Ile Leu
305                 310                 315                 320

acg ccc ctt ctt ctt tcc att cgc cag gtc ttt gtc aag gac acg att     1008
Thr Pro Leu Leu Leu Ser Ile Arg Gln Val Phe Val Lys Asp Thr Ile
                325                 330                 335

gtg atg cgc tgg ttt att gtc atc agc tgg ttt tca ccc ata ccc ata     1056
Val Met Arg Trp Phe Ile Val Ile Ser Trp Phe Ser Pro Ile Pro Ile
            340                 345                 350

gcc atc gtc tac gtt ttt ctg atc aac gta ctg cga gtg atc gtg cga     1104

Ala Ile Val Tyr Val Phe Leu Ile Asn Val Leu Arg Val Ile Val Arg
        355                 360                 365

aag tta cat ccc cag tcc gcc cag ccg gca ccg ttg gcc atc cgg aag     1152
Lys Leu His Pro Gln Ser Ala Gln Pro Ala Pro Leu Ala Ile Arg Lys
    370                 375                 380

gcg gtt cgg gcc acc att att ctg gta ccg ctg ttc gga ctg caa cac     1200
Ala Val Arg Ala Thr Ile Ile Leu Val Pro Leu Phe Gly Leu Gln His
385                 390                 395                 400

ttc ctg ctg ccc tac cgt ccg gat gcc ggc acc cag ctg gac cac ttc     1248
Phe Leu Leu Pro Tyr Arg Pro Asp Ala Gly Thr Gln Leu Asp His Phe
                405                 410                 415

tat cag atg cta tcg gtg gtg ttg gtc agc ctg cag ggc ttt gtg gtc     1296
Tyr Gln Met Leu Ser Val Val Leu Val Ser Leu Gln Gly Phe Val Val
            420                 425                 430

tcc ttc ctg ttc tgc ttc gcc aat cac gat gtc acc ttt gcc att cgc     1344
Ser Phe Leu Phe Cys Phe Ala Asn His Asp Val Thr Phe Ala Ile Arg
        435                 440                 445

acg ctg ctg aac aag ttg ctg ccc agt tta gtg acc ccg ccg ccg gcc     1392
Thr Leu Leu Asn Lys Leu Leu Pro Ser Leu Val Thr Pro Pro Pro Ala
    450                 455                 460

ggg agt aat act gga cag atg gcc acg acc acg ccc agc cga gag ttg     1440
Gly Ser Asn Thr Gly Gln Met Ala Thr Thr Thr Pro Ser Arg Glu Leu
465                 470                 475                 480

ggc gtt taa                                                         1449
Gly Val

<210> 32
<211> 482
<212> PRT
<213> Drosophila melanogaster

<400> 32
Met Asn Glu Gly Val Ile Asp Met Arg Asn Val Asp Glu Glu Leu Ala
  1               5                  10                  15

Glu Lys Glu Glu Leu Met Ala Thr Val Val Ser Ala Thr Met Ala Thr
             20                  25                  30

Asn Gln Glu Glu Asn Arg Leu Phe Cys Pro Leu Asn Phe Asp Gly Tyr

        35                  40                  45

Leu Cys Trp Pro Arg Thr Pro Ala Gly Thr Val Leu Ser Gln Tyr Cys
    50                  55                  60

Pro Asp Phe Val Glu Gly Phe Asn Arg Lys Phe Leu Ala His Lys Thr
65                  70                  75                  80

Cys Leu Glu Asn Gly Ser Trp Tyr Arg His Pro Val Ser Asn Gln Thr
                85                  90                  95

Trp Ser Asn Tyr Thr Asn Cys Val Asp Tyr Glu Asp Leu Glu Phe Arg
            100                 105                 110

Gln Phe Ile Asn Glu Leu Tyr Val Lys Gly Tyr Ala Leu Ser Leu Leu
        115                 120                 125

Ala Leu Leu Ile Ser Ile Ile Ile Phe Leu Gly Phe Lys Ser Leu Arg
    130                 135                 140

Cys Thr Arg Ile Arg Ile His Val His Leu Phe Ala Ser Leu Ala Cys
145                 150                 155                 160

Thr Cys Val Ala Trp Ile Leu Trp Tyr Arg Leu Val Val Glu Arg Ser
                165                 170                 175

Glu Thr Ile Ala Glu Asn Pro Val Arg Cys Thr Glu Lys Ser Asn Tyr
            180                 185                 190

Glu Asn Asn Ile Ala Leu Gln Val Ala Arg Gln Leu Tyr Lys Thr Ile
        195                 200                 205

Asn Ile Thr Asn Phe Gln Leu Trp Cys Ile Gly Leu His Leu Val Val
    210                 215                 220

His Tyr Phe Met Leu Val Asn Tyr Phe Trp Met Phe Cys Glu Gly Leu
225                 230                 235                 240

His Leu His Leu Val Leu Val Val Val Ser Tyr Pro Tyr Ser Ser Gly
                245                 250                 255

Asn Leu Ala Ile Ala Cys Thr Ser Leu His Ser Leu Trp Ala Thr Ser
            260                 265                 270

Val His Gly Arg Cys Asp Phe Leu Gln Pro Leu Thr Trp Gln Trp Phe
        275                 280                 285

Thr Pro Ser Asn Leu Ser Glu Ser Lys Ser Gln Arg Trp Pro Thr Ala

290                 295                 300

Leu Arg Leu Arg Gln Val Glu Met Ser Gly Met Ser Val Gln Ile Leu
305                 310                 315                 320

Thr Pro Leu Leu Leu Ser Ile Arg Gln Val Phe Val Lys Asp Thr Ile
                325                 330                 335

Val Met Arg Trp Phe Ile Val Ile Ser Trp Phe Ser Pro Ile Pro Ile
            340                 345                 350

Ala Ile Val Tyr Val Phe Leu Ile Asn Val Leu Arg Val Ile Val Arg
        355                 360                 365

Lys Leu His Pro Gln Ser Ala Gln Pro Ala Pro Leu Ala Ile Arg Lys
    370                 375                 380

Ala Val Arg Ala Thr Ile Ile Leu Val Pro Leu Phe Gly Leu Gln His
385                 390                 395                 400

Phe Leu Leu Pro Tyr Arg Pro Asp Ala Gly Thr Gln Leu Asp His Phe
                405                 410                 415

Tyr Gln Met Leu Ser Val Val Leu Val Ser Leu Gln Gly Phe Val Val
            420                 425                 430

Ser Phe Leu Phe Cys Phe Ala Asn His Asp Val Thr Phe Ala Ile Arg
        435                 440                 445

Thr Leu Leu Asn Lys Leu Leu Pro Ser Leu Val Thr Pro Pro Pro Ala
    450                 455                 460

Gly Ser Asn Thr Gly Gln Met Ala Thr Thr Thr Pro Ser Arg Glu Leu
465                 470                 475                 480

Gly Val

<210> 33
<211> 1521
<212> DNA
<213> Drosophila melanogaster

<220>
<221> CDS
<222> (1)..(1518)

<400> 33
atg tac gcc tcc ttg atg gac gtt ggc cag acg ttg gca gcc agg ctg    48
Met Tyr Ala Ser Leu Met Asp Val Gly Gln Thr Leu Ala Ala Arg Leu
1               5                   10                  15

gcg gat agc gac ggc aac ggg gcc aat gac agc gga ctc ctg gca acc    96
Ala Asp Ser Asp Gly Asn Gly Ala Asn Asp Ser Gly Leu Leu Ala Thr
            20                  25                  30

gga caa ggt ctg gag cag gag cag gag ggt ctg gca ctg gat atg ggc    144
Gly Gln Gly Leu Glu Gln Glu Gln Glu Gly Leu Ala Leu Asp Met Gly
        35                  40                  45

cac aat gcc agc gcc gac ggc gga ata gta ccg tat gtg ccc gtg ctg    192
His Asn Ala Ser Ala Asp Gly Gly Ile Val Pro Tyr Val Pro Val Leu
    50                  55                  60

gac cgc ccg gag acg tac att gtc acc gtg ctg tac acg ctc atc ttc    240
Asp Arg Pro Glu Thr Tyr Ile Val Thr Val Leu Tyr Thr Leu Ile Phe
65                  70                  75                  80

att gtg gga gtt ttg ggc aac ggc acg ctg gtc atc atc ttc ttt cgc    288
Ile Val Gly Val Leu Gly Asn Gly Thr Leu Val Ile Ile Phe Phe Arg
                85                  90                  95

cac cgc tcc atg cgc aac ata ccc aac aca tac att ctt tca ctg gcc    336
His Arg Ser Met Arg Asn Ile Pro Asn Thr Tyr Ile Leu Ser Leu Ala
            100                 105                 110

ctg gct gat ctg ttg gtt ata ttg gtg tgt gta cct gtg gcc acg att    384
Leu Ala Asp Leu Leu Val Ile Leu Val Cys Val Pro Val Ala Thr Ile
        115                 120                 125

gtc tac acg cag gaa agc tgg ccc ttt gag cgg aac atg tgc cgc atc    432
Val Tyr Thr Gln Glu Ser Trp Pro Phe Glu Arg Asn Met Cys Arg Ile
    130                 135                 140

agc gag ttc ttt aag gac ata tcc atc ggg gtg tcc gtg ttt aca ctg    480
Ser Glu Phe Phe Lys Asp Ile Ser Ile Gly Val Ser Val Phe Thr Leu
145                 150                 155                 160

acc gcc ctt tcc ggc gag cgg tac tgc gcc att gta aat ccc cta cgc    528
Thr Ala Leu Ser Gly Glu Arg Tyr Cys Ala Ile Val Asn Pro Leu Arg
                165                 170                 175

aag ctt cag acc aag ccg ctc act gtc ttt act gcg gtg atg atc tgg    576
Lys Leu Gln Thr Lys Pro Leu Thr Val Phe Thr Ala Val Met Ile Trp

            180                 185                 190

atc ctg gcc atc cta ctg ggc atg cct tcg gtt ctt ttc tcc gac atc 624
Ile Leu Ala Ile Leu Leu Gly Met Pro Ser Val Leu Phe Ser Asp Ile
        195                 200                 205

aag tcc tac cct gtg ttc aca gcc acc ggt aac atg acc att gaa gtg 672
Lys Ser Tyr Pro Val Phe Thr Ala Thr Gly Asn Met Thr Ile Glu Val
    210                 215                 220

tgc tcc cca ttt cgc gac ccg gag tat gca aag ttc atg gtg gcg ggc 720
Cys Ser Pro Phe Arg Asp Pro Glu Tyr Ala Lys Phe Met Val Ala Gly
225                 230                 235                 240

aag gca ctg gtg tac tac ctg ttg ccg ctg tcc atc att ggg gcg cta 768
Lys Ala Leu Val Tyr Tyr Leu Leu Pro Leu Ser Ile Ile Gly Ala Leu
                245                 250                 255

tac atc atg atg gcc aag cgg ctc cat atg agc gcc cgc aac atg ccc 816
Tyr Ile Met Met Ala Lys Arg Leu His Met Ser Ala Arg Asn Met Pro
            260                 265                 270

ggc gaa cag cag agc atg cag agc cgc acc cag gct agg gcc cga ctc 864
Gly Glu Gln Gln Ser Met Gln Ser Arg Thr Gln Ala Arg Ala Arg Leu
        275                 280                 285

cat gtg gcg cgc atg gtg gta gca ttc gtg gtg gtg ttc ttc atc tgc 912
His Val Ala Arg Met Val Val Ala Phe Val Val Val Phe Phe Ile Cys
    290                 295                 300

ttc ttc ccg tac cac gtg ttt gag ctg tgg tac cac ttc tac cca acg 960
Phe Phe Pro Tyr His Val Phe Glu Leu Trp Tyr His Phe Tyr Pro Thr
305                 310                 315                 320

gct gag gag gac ttc gat gag ttc tgg aac gtg ctg cgc atc gtt gga 1008
Ala Glu Glu Asp Phe Asp Glu Phe Trp Asn Val Leu Arg Ile Val Gly
                325                 330                 335

ttt tgc acg agc gca gta aaa ctc aaa aat aat gtg tca gca att tgg 1056
Phe Cys Thr Ser Ala Val Lys Leu Lys Asn Asn Val Ser Ala Ile Trp
            340                 345                 350

tgg ctt aat tca ctg ccg ctc gtc gtc aaa gcc att tgc att ctg gcc 1104
Trp Leu Asn Ser Leu Pro Leu Val Val Lys Ala Ile Cys Ile Leu Ala
        355                 360                 365

aaa tgg ctt cct aaa ctc gtg cgt caa ccc cgt ggc ctc tac tgc gtg 1152
Lys Trp Leu Pro Lys Leu Val Arg Gln Pro Arg Gly Leu Tyr Cys Val

370                 375                 380

tcc ggg gtg ttt cgg cag cac ttt aat cgc tac ctc tgc tgc atc tgc    1200
Ser Gly Val Phe Arg Gln His Phe Asn Arg Tyr Leu Cys Cys Ile Cys
385                 390                 395                 400

gtc aag cgg cag ccg cac ctg cgg cag cac tca acg gcc act gga atg    1248
Val Lys Arg Gln Pro His Leu Arg Gln His Ser Thr Ala Thr Gly Met
                405                 410                 415

atg gac aat acc agt gtg atg tcc atg cgc cgc tcc acg tac gtg ggt    1296
Met Asp Asn Thr Ser Val Met Ser Met Arg Arg Ser Thr Tyr Val Gly
            420                 425                 430

gga acc gct ggc aat ctg cgg gcc tcg ctg cac cgg aac agc aat cac    1344
Gly Thr Ala Gly Asn Leu Arg Ala Ser Leu His Arg Asn Ser Asn His
        435                 440                 445

gga gtt ggt gga gct gga ggt gga gta gga gga gga gta ggg tca ggt    1392
Gly Val Gly Gly Ala Gly Gly Gly Val Gly Gly Gly Val Gly Ser Gly
    450                 455                 460

cgt gtg ggc agc ttt cat cgg cag gac tcg atg ccc ctg cag cac gga    1440
Arg Val Gly Ser Phe His Arg Gln Asp Ser Met Pro Leu Gln His Gly
465                 470                 475                 480

aat gcc cac gga ggt ggt gcg ggc ggg gga tcc tcc gga ctt gga gcc    1488
Asn Ala His Gly Gly Gly Ala Gly Gly Gly Ser Ser Gly Leu Gly Ala
                485                 490                 495

ggc ggg cgg acg gcg gca gtg agc gaa aag agn                        1521
Gly Gly Arg Thr Ala Ala Val Ser Glu Lys
            500                 505

<210> 34
<211> 506
<212> PRT
<213> Drosophila melanogaster

<400> 34
Met Tyr Ala Ser Leu Met Asp Val Gly Gln Thr Leu Ala Ala Arg Leu
  1               5                  10                  15

Ala Asp Ser Asp Gly Asn Gly Ala Asn Asp Ser Gly Leu Leu Ala Thr
             20                  25                  30

Gly Gln Gly Leu Glu Gln Glu Gln Glu Gly Leu Ala Leu Asp Met Gly

35                  40                  45

His Asn Ala Ser Ala Asp Gly Gly Ile Val Pro Tyr Val Pro Val Leu
    50                  55                  60

Asp Arg Pro Glu Thr Tyr Ile Val Thr Val Leu Tyr Thr Leu Ile Phe
65                  70                  75                  80

Ile Val Gly Val Leu Gly Asn Gly Thr Leu Val Ile Ile Phe Phe Arg
                85                  90                  95

His Arg Ser Met Arg Asn Ile Pro Asn Thr Tyr Ile Leu Ser Leu Ala
            100                 105                 110

Leu Ala Asp Leu Leu Val Ile Leu Val Cys Val Pro Val Ala Thr Ile
        115                 120                 125

Val Tyr Thr Gln Glu Ser Trp Pro Phe Glu Arg Asn Met Cys Arg Ile
    130                 135                 140

Ser Glu Phe Phe Lys Asp Ile Ser Ile Gly Val Ser Val Phe Thr Leu
145                 150                 155                 160

Thr Ala Leu Ser Gly Glu Arg Tyr Cys Ala Ile Val Asn Pro Leu Arg
                165                 170                 175

Lys Leu Gln Thr Lys Pro Leu Thr Val Phe Thr Ala Val Met Ile Trp
            180                 185                 190

Ile Leu Ala Ile Leu Leu Gly Met Pro Ser Val Leu Phe Ser Asp Ile
        195                 200                 205

Lys Ser Tyr Pro Val Phe Thr Ala Thr Gly Asn Met Thr Ile Glu Val
    210                 215                 220

Cys Ser Pro Phe Arg Asp Pro Glu Tyr Ala Lys Phe Met Val Ala Gly
225                 230                 235                 240

Lys Ala Leu Val Tyr Tyr Leu Leu Pro Leu Ser Ile Ile Gly Ala Leu
                245                 250                 255

Tyr Ile Met Met Ala Lys Arg Leu His Met Ser Ala Arg Asn Met Pro
            260                 265                 270

Gly Glu Gln Gln Ser Met Gln Ser Arg Thr Gln Ala Arg Ala Arg Leu
        275                 280                 285

His Val Ala Arg Met Val Val Ala Phe Val Val Val Phe Phe Ile Cys

290                 295                 300

Phe Phe Pro Tyr His Val Phe Glu Leu Trp Tyr His Phe Tyr Pro Thr
305                 310                 315                 320

Ala Glu Glu Asp Phe Asp Glu Phe Trp Asn Val Leu Arg Ile Val Gly
                325                 330                 335

Phe Cys Thr Ser Ala Val Lys Leu Lys Asn Asn Val Ser Ala Ile Trp
            340                 345                 350

Trp Leu Asn Ser Leu Pro Leu Val Val Lys Ala Ile Cys Ile Leu Ala
        355                 360                 365

Lys Trp Leu Pro Lys Leu Val Arg Gln Pro Arg Gly Leu Tyr Cys Val
    370                 375                 380

Ser Gly Val Phe Arg Gln His Phe Asn Arg Tyr Leu Cys Cys Ile Cys
385                 390                 395                 400

Val Lys Arg Gln Pro His Leu Arg Gln His Ser Thr Ala Thr Gly Met
                405                 410                 415

Met Asp Asn Thr Ser Val Met Ser Met Arg Arg Ser Thr Tyr Val Gly
            420                 425                 430

Gly Thr Ala Gly Asn Leu Arg Ala Ser Leu His Arg Asn Ser Asn His
        435                 440                 445

Gly Val Gly Gly Ala Gly Gly Gly Val Gly Gly Gly Val Gly Ser Gly
    450                 455                 460

Arg Val Gly Ser Phe His Arg Gln Asp Ser Met Pro Leu Gln His Gly
465                 470                 475                 480

Asn Ala His Gly Gly Gly Ala Gly Gly Gly Ser Ser Gly Leu Gly Ala
                485                 490                 495

Gly Gly Arg Thr Ala Ala Val Ser Glu Lys
            500                 505

<210> 35
<211> 1578
<212> DNA
<213> Drosophila melanogaster

<220>
<221> CDS
<222> (1)..(1575)

<400> 35
atg acg tcc ttt att ggc ggc cca agg agt cgt cgc cac acg cac gaa     48
Met Thr Ser Phe Ile Gly Gly Pro Arg Ser Arg Arg His Thr His Glu
1               5                   10                  15

atg att atg acg atg atg cag acg gta agg gca tgg caa cag gaa tcg     96
Met Ile Met Thr Met Met Gln Thr Val Arg Ala Trp Gln Gln Glu Ser
            20                  25                  30

gat gtg gag cac cga aaa caa cat aag cag cga tgg cga cct gat ggg     144
Asp Val Glu His Arg Lys Gln His Lys Gln Arg Trp Arg Pro Asp Gly
        35                  40                  45

gca cac ata tca gcg gct tac gac ctc aac agt gat aat gac gac ggc     192
Ala His Ile Ser Ala Ala Tyr Asp Leu Asn Ser Asp Asn Asp Asp Gly
    50                  55                  60

cac cac agg gtg gtg cat aat caa aac aac ggc agc cca aac agc tct     240
His His Arg Val Val His Asn Gln Asn Asn Gly Ser Pro Asn Ser Ser
65                  70                  75                  80

ccg aat cag agc act tcc gcc ttc cga cag cgg caa ccg cac cac cca     288
Pro Asn Gln Ser Thr Ser Ala Phe Arg Gln Arg Gln Pro His His Pro
                85                  90                  95

ccc acc gga caa caa ccg ccc cgg ctg ccc tgc acc gta acc cac ttc     336
Pro Thr Gly Gln Gln Pro Pro Arg Leu Pro Cys Thr Val Thr His Phe
            100                 105                 110

tcc gca cac tgg aaa acg ctg ctc atc ctg ctc acc ctg ctc tcc gct     384
Ser Ala His Trp Lys Thr Leu Leu Ile Leu Leu Thr Leu Leu Ser Ala
        115                 120                 125

tcc acg ctg aca gcc tcc gcc aat gtc acc agc acc atc agt ccg ccc     432
Ser Thr Leu Thr Ala Ser Ala Asn Val Thr Ser Thr Ile Ser Pro Pro
    130                 135                 140

atc aac ggc agc agc acc gat tac atc ctg ctc tat ggc gaa tcc acc     480
Ile Asn Gly Ser Ser Thr Asp Tyr Ile Leu Leu Tyr Gly Glu Ser Thr
145                 150                 155                 160

aca tcc ctt gtg ccc gcc ctg acc acc gga ctc tcg ggg gac gga agt     528
Thr Ser Leu Val Pro Ala Leu Thr Thr Gly Leu Ser Gly Asp Gly Ser
                165                 170                 175

ggt gcg gtt atc gag gac gag gag gat gcc gag aag gcc agc gag tac 576
Gly Ala Val Ile Glu Asp Glu Glu Asp Ala Glu Lys Ala Ser Glu Tyr
            180                 185                 190

atc ttt gat cgc acc gat gtg cgc ata ata ttc atc aca ctc tac aca 624
Ile Phe Asp Arg Thr Asp Val Arg Ile Ile Phe Ile Thr Leu Tyr Thr
        195                 200                 205

ctg gtg ttt tgc tgc tgt ttc ttt ggt aac ctc ctt gtt att ttg gtg 672
Leu Val Phe Cys Cys Cys Phe Phe Gly Asn Leu Leu Val Ile Leu Val
    210                 215                 220

gtc aca tta tca cga cga ctg cgc tca ata acc aac ttt ttc ctc gcc 720
Val Thr Leu Ser Arg Arg Leu Arg Ser Ile Thr Asn Phe Phe Leu Ala
225                 230                 235                 240

aac ttg gcg ttt gcc gac ttc tgt gtt gga ctc ttt tgt gtg atg cag 768
Asn Leu Ala Phe Ala Asp Phe Cys Val Gly Leu Phe Cys Val Met Gln
                245                 250                 255

aat ctg tcc atc tat ctg ata gaa agc tgg gtc ttt ggc gag ttc ctc 816
Asn Leu Ser Ile Tyr Leu Ile Glu Ser Trp Val Phe Gly Glu Phe Leu
            260                 265                 270

tgc cgc atg tac cag ttt gtc cac tcc ctg agc tac acg gcc tcc att 864
Cys Arg Met Tyr Gln Phe Val His Ser Leu Ser Tyr Thr Ala Ser Ile
        275                 280                 285

ttc atc ctg gtt gtc atc tgt atg gag cgc tac ttt gcc atc gta cac 912
Phe Ile Leu Val Val Ile Cys Met Glu Arg Tyr Phe Ala Ile Val His
    290                 295                 300

ccc att acc tgc aaa cag atc cta act gcg gct cgt ctg agg atg gtc 960
Pro Ile Thr Cys Lys Gln Ile Leu Thr Ala Ala Arg Leu Arg Met Val
305                 310                 315                 320

att gtc aca gtt tgg att aca tcg gca gtt tac tcc acg ccc aag ttt 1008
Ile Val Thr Val Trp Ile Thr Ser Ala Val Tyr Ser Thr Pro Lys Phe
                325                 330                 335

gtt ttc agc aag acc att aag aac atc cac acg cag gac ggg cag gag 1056
Val Phe Ser Lys Thr Ile Lys Asn Ile His Thr Gln Asp Gly Gln Glu
            340                 345                 350

gag gag ata tgt gtc ctg gac cgt gag atg ttc aac tcc aag ttg ctg 1104
Glu Glu Ile Cys Val Leu Asp Arg Glu Met Phe Asn Ser Lys Leu Leu
        355                 360                 365

gac atg att aac ttt gtg ctt ctc tac gtt atg ccg ctc ttg gtg atg    1152
Asp Met Ile Asn Phe Val Leu Leu Tyr Val Met Pro Leu Leu Val Met
    370                 375                 380

acg gtg ctg tac agc aaa atc gcc atc gcc ttg tgg cgc agt tcg cgc    1200
Thr Val Leu Tyr Ser Lys Ile Ala Ile Ala Leu Trp Arg Ser Ser Arg
385                 390                 395                 400

ggc ctc acg ccg cat gtg gtg cag cat cag cat cag cag ccg cag cag    1248
Gly Leu Thr Pro His Val Val Gln His Gln His Gln Gln Pro Gln Gln
                405                 410                 415

cca tcc tgc cag gac att ggc atg ggt atg cac aac agc atg tac cat    1296
Pro Ser Cys Gln Asp Ile Gly Met Gly Met His Asn Ser Met Tyr His
            420                 425                 430

cac cat ccg cat cat cat cac cac cac cac cag cat cac cag ctg caa    1344
His His Pro His His His His His His His Gln His His Gln Leu Gln
        435                 440                 445

tcg gcg gct tcg tcg gca gga gtg gtg gga gtg ggt ttg ggt ggt ggt    1392
Ser Ala Ala Ser Ser Ala Gly Val Val Gly Val Gly Leu Gly Gly Gly
    450                 455                 460

ggt ggg ggc ggt cct ggt ccg tca ctc gct tcc ggc ggc agc agc acc    1440
Gly Gly Gly Gly Pro Gly Pro Ser Leu Ala Ser Gly Gly Ser Ser Thr
465                 470                 475                 480

acg tcc ttg tcc cgc aaa cag agc agt aaa tat gag aag cgc ggc gtc    1488
Thr Ser Leu Ser Arg Lys Gln Ser Ser Lys Tyr Glu Lys Arg Gly Val
                485                 490                 495

agc atc acg gag agc cag gtg agc tgc cag gag aac act gca tca gaa    1536
Ser Ile Thr Glu Ser Gln Val Ser Cys Gln Glu Asn Thr Ala Ser Glu
            500                 505                 510

aaa tcg ccg gtt gag cac gga gga gta gcc tta aaa gcc taa            1578
Lys Ser Pro Val Glu His Gly Gly Val Ala Leu Lys Ala
        515                 520                 525

<210> 36
<211> 525
<212> PRT
<213> Drosophila melanogaster

<400> 36

Met Thr Ser Phe Ile Gly Gly Pro Arg Ser Arg Arg His Thr His Glu
1               5                   10                  15

Met Ile Met Thr Met Met Gln Thr Val Arg Ala Trp Gln Gln Glu Ser
            20                  25                  30

Asp Val Glu His Arg Lys Gln His Lys Gln Arg Trp Arg Pro Asp Gly
        35                  40                  45

Ala His Ile Ser Ala Ala Tyr Asp Leu Asn Ser Asp Asn Asp Asp Gly
    50                  55                  60

His His Arg Val Val His Asn Gln Asn Asn Gly Ser Pro Asn Ser Ser
65                  70                  75                  80

Pro Asn Gln Ser Thr Ser Ala Phe Arg Gln Arg Gln Pro His His Pro
                85                  90                  95

Pro Thr Gly Gln Gln Pro Pro Arg Leu Pro Cys Thr Val Thr His Phe
            100                 105                 110

Ser Ala His Trp Lys Thr Leu Leu Ile Leu Leu Thr Leu Leu Ser Ala
        115                 120                 125

Ser Thr Leu Thr Ala Ser Ala Asn Val Thr Ser Thr Ile Ser Pro Pro
    130                 135                 140

Ile Asn Gly Ser Ser Thr Asp Tyr Ile Leu Leu Tyr Gly Glu Ser Thr
145                 150                 155                 160

Thr Ser Leu Val Pro Ala Leu Thr Thr Gly Leu Ser Gly Asp Gly Ser
                165                 170                 175

Gly Ala Val Ile Glu Asp Glu Glu Asp Ala Glu Lys Ala Ser Glu Tyr
            180                 185                 190

Ile Phe Asp Arg Thr Asp Val Arg Ile Ile Phe Ile Thr Leu Tyr Thr
        195                 200                 205

Leu Val Phe Cys Cys Cys Phe Phe Gly Asn Leu Leu Val Ile Leu Val
    210                 215                 220

Val Thr Leu Ser Arg Arg Leu Arg Ser Ile Thr Asn Phe Phe Leu Ala
225                 230                 235                 240

Asn Leu Ala Phe Ala Asp Phe Cys Val Gly Leu Phe Cys Val Met Gln
                245                 250                 255

Asn Leu Ser Ile Tyr Leu Ile Glu Ser Trp Val Phe Gly Glu Phe Leu
            260                 265                 270

Cys Arg Met Tyr Gln Phe Val His Ser Leu Ser Tyr Thr Ala Ser Ile
        275                 280                 285

Phe Ile Leu Val Val Ile Cys Met Glu Arg Tyr Phe Ala Ile Val His
    290                 295                 300

Pro Ile Thr Cys Lys Gln Ile Leu Thr Ala Ala Arg Leu Arg Met Val
305                 310                 315                 320

Ile Val Thr Val Trp Ile Thr Ser Ala Val Tyr Ser Thr Pro Lys Phe
                325                 330                 335

Val Phe Ser Lys Thr Ile Lys Asn Ile His Thr Gln Asp Gly Gln Glu
            340                 345                 350

Glu Glu Ile Cys Val Leu Asp Arg Glu Met Phe Asn Ser Lys Leu Leu
        355                 360                 365

Asp Met Ile Asn Phe Val Leu Leu Tyr Val Met Pro Leu Leu Val Met
    370                 375                 380

Thr Val Leu Tyr Ser Lys Ile Ala Ile Ala Leu Trp Arg Ser Ser Arg
385                 390                 395                 400

Gly Leu Thr Pro His Val Val Gln His Gln His Gln Gln Pro Gln Gln
                405                 410                 415

Pro Ser Cys Gln Asp Ile Gly Met Gly Met His Asn Ser Met Tyr His
            420                 425                 430

His His Pro His His His His His His His Gln His His Gln Leu Gln
        435                 440                 445

Ser Ala Ala Ser Ser Ala Gly Val Val Gly Val Gly Leu Gly Gly Gly
    450                 455                 460

Gly Gly Gly Gly Pro Gly Pro Ser Leu Ala Ser Gly Gly Ser Ser Thr
465                 470                 475                 480

Thr Ser Leu Ser Arg Lys Gln Ser Ser Lys Tyr Glu Lys Arg Gly Val
                485                 490                 495

Ser Ile Thr Glu Ser Gln Val Ser Cys Gln Glu Asn Thr Ala Ser Glu
            500                 505                 510

Lys Ser Pro Val Glu His Gly Gly Val Ala Leu Lys Ala
        515                 520                 525

<210> 37
<211> 1569
<212> DNA
<213> Drosophila melanogaster

<220>
<221> CDS
<222> (1)..(1566)

<400> 37
atg aat cct ttg gtg ctg ggc cac cta ctg gca cca aac aaa ctt ttt     48
Met Asn Pro Leu Val Leu Gly His Leu Leu Ala Pro Asn Lys Leu Phe
  1               5                  10                  15

ata cga tta ttg ccc cgt att tca agc cac aaa gca tta agc aag cca     96
Ile Arg Leu Leu Pro Arg Ile Ser Ser His Lys Ala Leu Ser Lys Pro
             20                  25                  30

gtg gaa ttt gcc att cgc agc tcg cta gaa agt gtg ata atc agc atg    144
Val Glu Phe Ala Ile Arg Ser Ser Leu Glu Ser Val Ile Ile Ser Met
         35                  40                  45

aat cag acg gag ccc gcc cag ctg gca gat ggg gag cat ctg agt gga    192
Asn Gln Thr Glu Pro Ala Gln Leu Ala Asp Gly Glu His Leu Ser Gly
     50                  55                  60

tac gcc agc agc agc aac agc gtg cgc tat ctg gac gac cgg cat ccg    240
Tyr Ala Ser Ser Ser Asn Ser Val Arg Tyr Leu Asp Asp Arg His Pro
 65                  70                  75                  80

ctg gac tac ctt gac ctg ggc acg gtg cac gcc ctc aac acc act gcc    288
Leu Asp Tyr Leu Asp Leu Gly Thr Val His Ala Leu Asn Thr Thr Ala
                 85                  90                  95

atc aac acc tcg gat ctg aat gag act ggg agc agg ccg ctg gac ccg    336
Ile Asn Thr Ser Asp Leu Asn Glu Thr Gly Ser Arg Pro Leu Asp Pro
            100                 105                 110

gtg ctt atc gat agg ttc ctg agc aac agg gcg gtg gac agc ccc tgg    384
Val Leu Ile Asp Arg Phe Leu Ser Asn Arg Ala Val Asp Ser Pro Trp
        115                 120                 125

tac cac atg ctc atc agc atg tac ggc gtg cta atc gtc ttc ggc gcc    432

Tyr His Met Leu Ile Ser Met Tyr Gly Val Leu Ile Val Phe Gly Ala
    130                 135                 140

cta ggc aac acc ctg gtt gtt ata gcc gtc atc cgg aag ccc atc atg     480
Leu Gly Asn Thr Leu Val Val Ile Ala Val Ile Arg Lys Pro Ile Met
145                 150                 155                 160

cgc act gct cgc aat ctg ttc atc ctc aac ctg gcc ata tcg gtc acc     528
Arg Thr Ala Arg Asn Leu Phe Ile Leu Asn Leu Ala Ile Ser Val Thr
                165                 170                 175

atg ccg ctg acc ttg atg gag atc ctg tcc aag tac tgg ccc tac ggc     576
Met Pro Leu Thr Leu Met Glu Ile Leu Ser Lys Tyr Trp Pro Tyr Gly
            180                 185                 190

tcc tgc tcc atc ctg tgc aaa acg att gcc atg ctg cag gca ctt tgt     624
Ser Cys Ser Ile Leu Cys Lys Thr Ile Ala Met Leu Gln Ala Leu Cys
        195                 200                 205

att ttc gtg tcg aca ata tcc ata acg gcc att gcc ttc gac aga tat     672
Ile Phe Val Ser Thr Ile Ser Ile Thr Ala Ile Ala Phe Asp Arg Tyr
    210                 215                 220

cag gtg atc gtg tac ccc acg cgg gac agc ctg cag ttc gtg ggc gcg     720
Gln Val Ile Val Tyr Pro Thr Arg Asp Ser Leu Gln Phe Val Gly Ala
225                 230                 235                 240

gtg acg atc ctg gcg ggg atc tgg gca ctg gca ctg ctg ctg gcc tcg     768
Val Thr Ile Leu Ala Gly Ile Trp Ala Leu Ala Leu Leu Leu Ala Ser
                245                 250                 255

ccg ctg ttc gtc tac aag gag ctg atc aac aca gac acg ccg gca ctc     816
Pro Leu Phe Val Tyr Lys Glu Leu Ile Asn Thr Asp Thr Pro Ala Leu
            260                 265                 270

ctg cag cag atc ggc ctg cag gac acg atc ccg tac tgc att gag gac     864
Leu Gln Gln Ile Gly Leu Gln Asp Thr Ile Pro Tyr Cys Ile Glu Asp
        275                 280                 285

tgg cca agt cgc aac ggg cgc ttc tac tac tcg atc ttc tcg ctg tgc     912
Trp Pro Ser Arg Asn Gly Arg Phe Tyr Tyr Ser Ile Phe Ser Leu Cys
    290                 295                 300

gta caa tac ctg gtg ccc atc ctg atc gtc tcg gtg gca tac ttc ggg     960
Val Gln Tyr Leu Val Pro Ile Leu Ile Val Ser Val Ala Tyr Phe Gly
305                 310                 315                 320

atc tac aac aag ctg aag agc cgc atc acc gtg gtg gct gtg cag gcg     1008

Ile Tyr Asn Lys Leu Lys Ser Arg Ile Thr Val Val Ala Val Gln Ala
                325                 330                 335

tcc tcc gct cag cgg aag gtg gag cgg ggg cgg cgg atg aag cgc acc      1056
Ser Ser Ala Gln Arg Lys Val Glu Arg Gly Arg Arg Met Lys Arg Thr
            340                 345                 350

aac tgc cta ctg atc agc atc gcc atc atc ttt ggc gtt tct tgg ctg      1104
Asn Cys Leu Leu Ile Ser Ile Ala Ile Ile Phe Gly Val Ser Trp Leu
        355                 360                 365

ccg ctg aac ttt ttc aac ctg tac gcg gac atg gag cgc tcg ccg gtc      1152
Pro Leu Asn Phe Phe Asn Leu Tyr Ala Asp Met Glu Arg Ser Pro Val
    370                 375                 380

act cag agc atg cta gtc cgc tac gcc atc tgc cac atg atc ggc atg      1200
Thr Gln Ser Met Leu Val Arg Tyr Ala Ile Cys His Met Ile Gly Met
385                 390                 395                 400

agc tcc gcc tgc tcc aac ccg ttg ctc tac ggc tgg ctc aac gac aac      1248
Ser Ser Ala Cys Ser Asn Pro Leu Leu Tyr Gly Trp Leu Asn Asp Asn
                405                 410                 415

ttc cgt aaa gaa ttt caa gaa ctg ctc tgc cgt tgc tca gac act aat      1296
Phe Arg Lys Glu Phe Gln Glu Leu Leu Cys Arg Cys Ser Asp Thr Asn
            420                 425                 430

gtt gct ctt aac ggt cac acg aca ggc tgc aac gtc cag gcg gcg gcg      1344
Val Ala Leu Asn Gly His Thr Thr Gly Cys Asn Val Gln Ala Ala Ala
        435                 440                 445

cgc agg cgt cgc aag ttg ggc gcc gaa ctc tcc aaa ggc gaa ctc aag      1392
Arg Arg Arg Arg Lys Leu Gly Ala Glu Leu Ser Lys Gly Glu Leu Lys
    450                 455                 460

ctg ctg ggg cca ggc ggc gcc cag agc ggt acc gcc ggc ggg gaa ggc      1440
Leu Leu Gly Pro Gly Gly Ala Gln Ser Gly Thr Ala Gly Gly Glu Gly
465                 470                 475                 480

ggt ctg gcg gcc acc gac ttc atg acc ggc cac cac gag ggc gga ctg      1488
Gly Leu Ala Ala Thr Asp Phe Met Thr Gly His His Glu Gly Gly Leu
                485                 490                 495

cgc agc gcc ata acc gag tcg gtg gcc ctc acg gac cac aac ccc gtg      1536
Arg Ser Ala Ile Thr Glu Ser Val Ala Leu Thr Asp His Asn Pro Val
            500                 505                 510

ccc tcg gag gtc acc aag ctg atg ccg cgg taa                          1569

Pro Ser Glu Val Thr Lys Leu Met Pro Arg
        515                 520

<210> 38
<211> 522
<212> PRT
<213> Drosophila melanogaster

<400> 38
Met Asn Pro Leu Val Leu Gly His Leu Leu Ala Pro Asn Lys Leu Phe
1               5                   10                  15

Ile Arg Leu Leu Pro Arg Ile Ser Ser His Lys Ala Leu Ser Lys Pro
            20                  25                  30

Val Glu Phe Ala Ile Arg Ser Ser Leu Glu Ser Val Ile Ile Ser Met
        35                  40                  45

Asn Gln Thr Glu Pro Ala Gln Leu Ala Asp Gly Glu His Leu Ser Gly
    50                  55                  60

Tyr Ala Ser Ser Ser Asn Ser Val Arg Tyr Leu Asp Asp Arg His Pro
65                  70                  75                  80

Leu Asp Tyr Leu Asp Leu Gly Thr Val His Ala Leu Asn Thr Thr Ala
                85                  90                  95

Ile Asn Thr Ser Asp Leu Asn Glu Thr Gly Ser Arg Pro Leu Asp Pro
            100                 105                 110

Val Leu Ile Asp Arg Phe Leu Ser Asn Arg Ala Val Asp Ser Pro Trp
        115                 120                 125

Tyr His Met Leu Ile Ser Met Tyr Gly Val Leu Ile Val Phe Gly Ala
    130                 135                 140

Leu Gly Asn Thr Leu Val Val Ile Ala Val Ile Arg Lys Pro Ile Met
145                 150                 155                 160

Arg Thr Ala Arg Asn Leu Phe Ile Leu Asn Leu Ala Ile Ser Val Thr
                165                 170                 175

Met Pro Leu Thr Leu Met Glu Ile Leu Ser Lys Tyr Trp Pro Tyr Gly
            180                 185                 190

Ser Cys Ser Ile Leu Cys Lys Thr Ile Ala Met Leu Gln Ala Leu Cys
        195                 200                 205

Ile Phe Val Ser Thr Ile Ser Ile Thr Ala Ile Ala Phe Asp Arg Tyr
    210                 215                 220

Gln Val Ile Val Tyr Pro Thr Arg Asp Ser Leu Gln Phe Val Gly Ala
225                 230                 235                 240

Val Thr Ile Leu Ala Gly Ile Trp Ala Leu Ala Leu Leu Leu Ala Ser
                245                 250                 255

Pro Leu Phe Val Tyr Lys Glu Leu Ile Asn Thr Asp Thr Pro Ala Leu
            260                 265                 270

Leu Gln Gln Ile Gly Leu Gln Asp Thr Ile Pro Tyr Cys Ile Glu Asp
        275                 280                 285

Trp Pro Ser Arg Asn Gly Arg Phe Tyr Tyr Ser Ile Phe Ser Leu Cys
    290                 295                 300

Val Gln Tyr Leu Val Pro Ile Leu Ile Val Ser Val Ala Tyr Phe Gly
305                 310                 315                 320

Ile Tyr Asn Lys Leu Lys Ser Arg Ile Thr Val Val Ala Val Gln Ala
                325                 330                 335

Ser Ser Ala Gln Arg Lys Val Glu Arg Gly Arg Arg Met Lys Arg Thr
            340                 345                 350

Asn Cys Leu Leu Ile Ser Ile Ala Ile Ile Phe Gly Val Ser Trp Leu
        355                 360                 365

Pro Leu Asn Phe Phe Asn Leu Tyr Ala Asp Met Glu Arg Ser Pro Val
    370                 375                 380

Thr Gln Ser Met Leu Val Arg Tyr Ala Ile Cys His Met Ile Gly Met
385                 390                 395                 400

Ser Ser Ala Cys Ser Asn Pro Leu Leu Tyr Gly Trp Leu Asn Asp Asn
                405                 410                 415

Phe Arg Lys Glu Phe Gln Glu Leu Leu Cys Arg Cys Ser Asp Thr Asn
            420                 425                 430

Val Ala Leu Asn Gly His Thr Thr Gly Cys Asn Val Gln Ala Ala Ala
        435                 440                 445

Arg Arg Arg Arg Lys Leu Gly Ala Glu Leu Ser Lys Gly Glu Leu Lys
    450                 455                 460

Leu Leu Gly Pro Gly Gly Ala Gln Ser Gly Thr Ala Gly Gly Glu Gly
465                 470                 475                 480

Gly Leu Ala Ala Thr Asp Phe Met Thr Gly His His Glu Gly Gly Leu
                485                 490                 495

Arg Ser Ala Ile Thr Glu Ser Val Ala Leu Thr Asp His Asn Pro Val
            500                 505                 510

Pro Ser Glu Val Thr Lys Leu Met Pro Arg
        515                 520

<210> 39
<211> 540
<212> DNA
<213> Drosophila melanogaster

<220>
<221> CDS
<222> (1)..(537)

<400> 39
atg gag aac acc aca atg ctg gct aat att agc cta aat gca acc aga      48
Met Glu Asn Thr Thr Met Leu Ala Asn Ile Ser Leu Asn Ala Thr Arg
  1               5                  10                  15

aat gag gag aat atc acc tca ttc ttc acc gac gaa gag tgg ctg gcc      96
Asn Glu Glu Asn Ile Thr Ser Phe Phe Thr Asp Glu Glu Trp Leu Ala
             20                  25                  30

atc aat ggc act ttg ccg tgg ata gtg gga ttc ttc ttc ggc gtc atc     144
Ile Asn Gly Thr Leu Pro Trp Ile Val Gly Phe Phe Phe Gly Val Ile
         35                  40                  45

gcc atc acg gga ttc ttc ggc aac ctg ctg gtc atc ctg gtg gtg gtc     192
Ala Ile Thr Gly Phe Phe Gly Asn Leu Leu Val Ile Leu Val Val Val
     50                  55                  60

ttc aac aac aac atg cgc tcc acc acc aac ctg atg att gtc aat ctg     240
Phe Asn Asn Asn Met Arg Ser Thr Thr Asn Leu Met Ile Val Asn Leu
 65                  70                  75                  80

gct gcc gct gat ctg atg ttc gta atc ctc tgc att ccc ttc acg gcc     288
Ala Ala Ala Asp Leu Met Phe Val Ile Leu Cys Ile Pro Phe Thr Ala
                 85                  90                  95

acc gat tac atg gtg tac tac tgg cca tat gga agg ttc tgg tgc cgc    336
Thr Asp Tyr Met Val Tyr Tyr Trp Pro Tyr Gly Arg Phe Trp Cys Arg
            100                 105                 110

agt gtc cag tac ctg att gtg gtg acc gcc ttc gcc tcc att tac acg    384
Ser Val Gln Tyr Leu Ile Val Val Thr Ala Phe Ala Ser Ile Tyr Thr
        115                 120                 125

ctg gtg ctg atg tcc atc gat cgg ttc ctg gcg gtg gtt cat ccc att    432
Leu Val Leu Met Ser Ile Asp Arg Phe Leu Ala Val Val His Pro Ile
    130                 135                 140

cgc tcg cgg atg atg agg acg gag aac att acc ctg att gcc atc gtg    480
Arg Ser Arg Met Met Arg Thr Glu Asn Ile Thr Leu Ile Ala Ile Val
145                 150                 155                 160

act ctg tgg atc gtg gtg ctg gtc gtt tcg gtg cca gtg gcc ttc acc    528
Thr Leu Trp Ile Val Val Leu Val Val Ser Val Pro Val Ala Phe Thr
                165                 170                 175

cac gac gtg gtg                                                    540
His Asp Val

<210> 40
<211> 179
<212> PRT
<213> Drosophila melanogaster

<400> 40
Met Glu Asn Thr Thr Met Leu Ala Asn Ile Ser Leu Asn Ala Thr Arg
 1               5                  10                  15

Asn Glu Glu Asn Ile Thr Ser Phe Phe Thr Asp Glu Glu Trp Leu Ala
            20                  25                  30

Ile Asn Gly Thr Leu Pro Trp Ile Val Gly Phe Phe Phe Gly Val Ile
        35                  40                  45

Ala Ile Thr Gly Phe Phe Gly Asn Leu Leu Val Ile Leu Val Val Val
    50                  55                  60

Phe Asn Asn Asn Met Arg Ser Thr Thr Asn Leu Met Ile Val Asn Leu
65                  70                  75                  80

Ala Ala Ala Asp Leu Met Phe Val Ile Leu Cys Ile Pro Phe Thr Ala
                85                  90                  95

Thr Asp Tyr Met Val Tyr Tyr Trp Pro Tyr Gly Arg Phe Trp Cys Arg
            100                 105                 110

Ser Val Gln Tyr Leu Ile Val Val Thr Ala Phe Ala Ser Ile Tyr Thr
        115                 120                 125

Leu Val Leu Met Ser Ile Asp Arg Phe Leu Ala Val Val His Pro Ile
    130                 135                 140

Arg Ser Arg Met Met Arg Thr Glu Asn Ile Thr Leu Ile Ala Ile Val
145                 150                 155                 160

Thr Leu Trp Ile Val Val Leu Val Val Ser Val Pro Val Ala Phe Thr
                165                 170                 175

His Asp Val

<210> 41
<211> 624
<212> DNA
<213> Drosophila melanogaster

<220>
<221> CDS
<222> (1)..(621)

<400> 41
atg acg tat gcg caa tat tcg att ccc aca ccg cac acg ttt ctt cat      48
Met Thr Tyr Ala Gln Tyr Ser Ile Pro Thr Pro His Thr Phe Leu His
  1               5                  10                  15

agt tcc cac aac cac att ctc atc gat atc cgc agg tgg att acg atg      96
Ser Ser His Asn His Ile Leu Ile Asp Ile Arg Arg Trp Ile Thr Met
             20                  25                  30

caa aga aga aca tca cct acg gca tgt gca cct tca cga cga acg act     144
Gln Arg Arg Thr Ser Pro Thr Ala Cys Ala Pro Ser Arg Arg Thr Thr
         35                  40                  45

tcc ttg gtc acc ttc ttc atc agc tcc tac ctg ctg ccc ctg atg atc     192
Ser Leu Val Thr Phe Phe Ile Ser Ser Tyr Leu Leu Pro Leu Met Ile
     50                  55                  60

atc agc ggt ctc tac atg cgc atg atc atg cgg ctc tgg cgc cag gga     240

Ile Ser Gly Leu Tyr Met Arg Met Ile Met Arg Leu Trp Arg Gln Gly
65                  70                  75                  80

acc ggc gtc cgc atg tcc aag gag tcg cag cgc ggt cgc aag cgg gtc     288
Thr Gly Val Arg Met Ser Lys Glu Ser Gln Arg Gly Arg Lys Arg Val
                85                  90                  95

acc cga ctc gtc gtc gtg gtg gtc atc gcc ttc gcc tcg ctc tgg ctg     336
Thr Arg Leu Val Val Val Val Val Ile Ala Phe Ala Ser Leu Trp Leu
            100                 105                 110

cct gtc cag ctc atc ctg ctg ctc aag tca ctg gat gtc atc gag acg     384
Pro Val Gln Leu Ile Leu Leu Leu Lys Ser Leu Asp Val Ile Glu Thr
        115                 120                 125

aac acc ctc acc aag cta gtc atc cag gtc acc gcc cag act ctg gcc     432
Asn Thr Leu Thr Lys Leu Val Ile Gln Val Thr Ala Gln Thr Leu Ala
    130                 135                 140

tac agc agc tcg tgt atc aat ccg ctg ctc tac gcc ttc ctc tcc gag     480
Tyr Ser Ser Ser Cys Ile Asn Pro Leu Leu Tyr Ala Phe Leu Ser Glu
145                 150                 155                 160

aat ttc cgg aag gcc ttc tac aag aca atg cga atg cac agc gca cct     528
Asn Phe Arg Lys Ala Phe Tyr Lys Thr Met Arg Met His Ser Ala Pro
                165                 170                 175

gga aag cca agg acc gca act gcc ggc ggt tca aaa ccc gta att cat     576
Gly Lys Pro Arg Thr Ala Thr Ala Gly Gly Ser Lys Pro Val Ile His
            180                 185                 190

ttc ccc gga gcc ctg tgc tgt gtt gct gtc tct gtg acg agg cac taa     624
Phe Pro Gly Ala Leu Cys Cys Val Ala Val Ser Val Thr Arg His
        195                 200                 205

<210> 42
<211> 207
<212> PRT
<213> Drosophila melanogaster

<400> 42
Met Thr Tyr Ala Gln Tyr Ser Ile Pro Thr Pro His Thr Phe Leu His
1               5                   10                  15

Ser Ser His Asn His Ile Leu Ile Asp Ile Arg Arg Trp Ile Thr Met
            20                  25                  30

Gln Arg Arg Thr Ser Pro Thr Ala Cys Ala Pro Ser Arg Arg Thr Thr
        35                  40                  45

Ser Leu Val Thr Phe Phe Ile Ser Ser Tyr Leu Leu Pro Leu Met Ile
    50                  55                  60

Ile Ser Gly Leu Tyr Met Arg Met Ile Met Arg Leu Trp Arg Gln Gly
65                  70                  75                  80

Thr Gly Val Arg Met Ser Lys Glu Ser Gln Arg Gly Arg Lys Arg Val
                85                  90                  95

Thr Arg Leu Val Val Val Val Val Ile Ala Phe Ala Ser Leu Trp Leu
            100                 105                 110

Pro Val Gln Leu Ile Leu Leu Leu Lys Ser Leu Asp Val Ile Glu Thr
        115                 120                 125

Asn Thr Leu Thr Lys Leu Val Ile Gln Val Thr Ala Gln Thr Leu Ala
    130                 135                 140

Tyr Ser Ser Ser Cys Ile Asn Pro Leu Leu Tyr Ala Phe Leu Ser Glu
145                 150                 155                 160

Asn Phe Arg Lys Ala Phe Tyr Lys Thr Met Arg Met His Ser Ala Pro
                165                 170                 175

Gly Lys Pro Arg Thr Ala Thr Ala Gly Gly Ser Lys Pro Val Ile His
            180                 185                 190

Phe Pro Gly Ala Leu Cys Cys Val Ala Val Ser Val Thr Arg His
        195                 200                 205

<210> 43
<211> 1566
<212> DNA
<213> Drosophila melanogaster

<220>
<221> CDS
<222> (1)..(1563)

<400> 43
atg gcc ctg ctc cac tac acc ttc gac cag ctc gag ttg tac cta gaa      48
Met Ala Leu Leu His Tyr Thr Phe Asp Gln Leu Glu Leu Tyr Leu Glu
1               5                   10                  15

tgg gcg ttc gcc cag cac gga gag gcc acg ccg att ccc tcg atc cag    96
Trp Ala Phe Ala Gln His Gly Glu Ala Thr Pro Ile Pro Ser Ile Gln
            20                  25                  30

ccg tat ccg ggc gtg ttc gtg ggc gat ctc agc cag ctg aac cgc ttc    144
Pro Tyr Pro Gly Val Phe Val Gly Asp Leu Ser Gln Leu Asn Arg Phe
        35                  40                  45

aag cga cat gcc ttc tcc gcc gtc gtg ggc acg ctc ttc gtc ctc gcc    192
Lys Arg His Ala Phe Ser Ala Val Val Gly Thr Leu Phe Val Leu Ala
    50                  55                  60

ttc tgc ggc aac ctg agc acc ctc tat gtg aac agc cgc cgg aag ctg    240
Phe Cys Gly Asn Leu Ser Thr Leu Tyr Val Asn Ser Arg Arg Lys Leu
65                  70                  75                  80

cgg ccc ttc ttc cgg gcc tgc ctc atc tca ctg gcc tgc agt gac ctt    288
Arg Pro Phe Phe Arg Ala Cys Leu Ile Ser Leu Ala Cys Ser Asp Leu
                85                  90                  95

gtc tcc agc ata ttc tgc acc gta tcc tac atg gcc cag ttc cag gcg    336
Val Ser Ser Ile Phe Cys Thr Val Ser Tyr Met Ala Gln Phe Gln Ala
            100                 105                 110

cag tat ctg cag ctc tgg aca att ggt ggc ttc atg tgc aag ttt gtg    384
Gln Tyr Leu Gln Leu Trp Thr Ile Gly Gly Phe Met Cys Lys Phe Val
        115                 120                 125

ccg ttc ata acc acc acg tcg gtg ttg tcc ggc agc ctg acc ctg gtg    432
Pro Phe Ile Thr Thr Thr Ser Val Leu Ser Gly Ser Leu Thr Leu Val
    130                 135                 140

gcc atc gcc ttg gac cgc tac ttg gcc gtg atg cga ccc gtg ctg ggg    480
Ala Ile Ala Leu Asp Arg Tyr Leu Ala Val Met Arg Pro Val Leu Gly
145                 150                 155                 160

ttc tgg agt ccc gac aaa cgc ttc agc act ctg agc atg ctg ctc atc    528
Phe Trp Ser Pro Asp Lys Arg Phe Ser Thr Leu Ser Met Leu Leu Ile
                165                 170                 175

tgg gca tgt tcg ata gga tcc tct ggc ccg ctg ctg ggc atc tac gac    576
Trp Ala Cys Ser Ile Gly Ser Ser Gly Pro Leu Leu Gly Ile Tyr Asp
            180                 185                 190

tac cga aag atc tat ctc ctg gat gtg gag gac tcc agc gag gag agc    624
Tyr Arg Lys Ile Tyr Leu Leu Asp Val Glu Asp Ser Ser Glu Glu Ser
        195                 200                 205

gag gag gtc gtg acc gct gtg ccc gag gag ctg gtg gtc acc gag ctg 672
Glu Glu Val Val Thr Ala Val Pro Glu Glu Leu Val Val Thr Glu Leu
    210                 215                 220

gaa atg gtt cac atg tgc ctg gcc ggg gac cac gac gtc gga ctc tac 720
Glu Met Val His Met Cys Leu Ala Gly Asp His Asp Val Gly Leu Tyr
225                 230                 235                 240

tac gtc ata ctg ttc acc ctg atc ttc ctg ccg tgc att gtg tcc ttc 768
Tyr Val Ile Leu Phe Thr Leu Ile Phe Leu Pro Cys Ile Val Ser Phe
                245                 250                 255

cta tgg ctg aat gcc gtg att gcg agg cag ctc tgg ctg cgg aga cac 816
Leu Trp Leu Asn Ala Val Ile Ala Arg Gln Leu Trp Leu Arg Arg His
            260                 265                 270

tac cac cag gag cag cag gag cag cat cag gag ccc aag gag ggt cag 864
Tyr His Gln Glu Gln Gln Glu Gln His Gln Glu Pro Lys Glu Gly Gln
        275                 280                 285

ttt aag acc atg gcg aat ggc ggg gat ctc ttg atg ccc tcc acc ttg 912
Phe Lys Thr Met Ala Asn Gly Gly Asp Leu Leu Met Pro Ser Thr Leu
    290                 295                 300

gtc agt gcc atg gga gtg gcc gtt ccc ttt gcg ctg gac aac act ccc 960
Val Ser Ala Met Gly Val Ala Val Pro Phe Ala Leu Asp Asn Thr Pro
305                 310                 315                 320

ttg ccg ccc aag agt acc gtc aac gct cct ggc aag aag acc act gct 1008
Leu Pro Pro Lys Ser Thr Val Asn Ala Pro Gly Lys Lys Thr Thr Ala
                325                 330                 335

gct gct cta gcc agg gag gcc agg cac cgc aag atg gtg gtg gtg gtg 1056
Ala Ala Leu Ala Arg Glu Ala Arg His Arg Lys Met Val Val Val Val
            340                 345                 350

ctg cta atg atg gcg gtt ttc ata tgc ctt cga ctg ccc gcc tgg gtc 1104
Leu Leu Met Met Ala Val Phe Ile Cys Leu Arg Leu Pro Ala Trp Val
        355                 360                 365

ttc ctg atc atg cgg ctt tat gga tcc tac tcc gag ccc atc gac tgg 1152
Phe Leu Ile Met Arg Leu Tyr Gly Ser Tyr Ser Glu Pro Ile Asp Trp
    370                 375                 380

ctc ctg tac ttc agc ttc ggc ata ctg aac ctt ttc agt tgc gcc cta 1200
Leu Leu Tyr Phe Ser Phe Gly Ile Leu Asn Leu Phe Ser Cys Ala Leu
385                 390                 395                 400

aat ccc atc ttc tac acc ttc ctc acc cag acc att agg acc ttg acg     1248
Asn Pro Ile Phe Tyr Thr Phe Leu Thr Gln Thr Ile Arg Thr Leu Thr
                405                 410                 415

ctg gtg aaa cac aaa att cag gga ttt ctt ggc tgt ccg cct ggt aaa     1296
Leu Val Lys His Lys Ile Gln Gly Phe Leu Gly Cys Pro Pro Gly Lys
            420                 425                 430

gtg ccg gat ggt atg ccc acg gat cag atg gac aaa agc ggg tgc tgc     1344
Val Pro Asp Gly Met Pro Thr Asp Gln Met Asp Lys Ser Gly Cys Cys
        435                 440                 445

tgc ggc ctg agg cca ccc acc ttc aca tgg agg tgc cat ccc agc cgc     1392
Cys Gly Leu Arg Pro Pro Thr Phe Thr Trp Arg Cys His Pro Ser Arg
    450                 455                 460

gat agg gca gca gcc acc gta atc cgg gat gtg gac cag cca gat ccg     1440
Asp Arg Ala Ala Ala Thr Val Ile Arg Asp Val Asp Gln Pro Asp Pro
465                 470                 475                 480

ccc tcc gac cag gtg caa ccg gat ccg agt agc cta cgg cgg ttc ctc     1488
Pro Ser Asp Gln Val Gln Pro Asp Pro Ser Ser Leu Arg Arg Phe Leu
                485                 490                 495

agc tac aag cag gag gtg ttc acc att tac aag cag tgc ggt gac tcg     1536
Ser Tyr Lys Gln Glu Val Phe Thr Ile Tyr Lys Gln Cys Gly Asp Ser
            500                 505                 510

tcg agt gcg tca atc gag tcc tcc gcc tga                             1566
Ser Ser Ala Ser Ile Glu Ser Ser Ala
        515                 520

<210> 44
<211> 521
<212> PRT
<213> Drosophila melanogaster

<400> 44
Met Ala Leu Leu His Tyr Thr Phe Asp Gln Leu Glu Leu Tyr Leu Glu
  1               5                  10                  15

Trp Ala Phe Ala Gln His Gly Glu Ala Thr Pro Ile Pro Ser Ile Gln
             20                  25                  30

Pro Tyr Pro Gly Val Phe Val Gly Asp Leu Ser Gln Leu Asn Arg Phe
         35                  40                  45

Lys Arg His Ala Phe Ser Ala Val Val Gly Thr Leu Phe Val Leu Ala
     50                  55                  60

Phe Cys Gly Asn Leu Ser Thr Leu Tyr Val Asn Ser Arg Arg Lys Leu
 65                  70                  75                  80

Arg Pro Phe Phe Arg Ala Cys Leu Ile Ser Leu Ala Cys Ser Asp Leu
                 85                  90                  95

Val Ser Ser Ile Phe Cys Thr Val Ser Tyr Met Ala Gln Phe Gln Ala
            100                 105                 110

Gln Tyr Leu Gln Leu Trp Thr Ile Gly Gly Phe Met Cys Lys Phe Val
        115                 120                 125

Pro Phe Ile Thr Thr Thr Ser Val Leu Ser Gly Ser Leu Thr Leu Val
    130                 135                 140

Ala Ile Ala Leu Asp Arg Tyr Leu Ala Val Met Arg Pro Val Leu Gly
145                 150                 155                 160

Phe Trp Ser Pro Asp Lys Arg Phe Ser Thr Leu Ser Met Leu Leu Ile
                165                 170                 175

Trp Ala Cys Ser Ile Gly Ser Ser Gly Pro Leu Leu Gly Ile Tyr Asp
            180                 185                 190

Tyr Arg Lys Ile Tyr Leu Leu Asp Val Glu Asp Ser Ser Glu Glu Ser
        195                 200                 205

Glu Glu Val Val Thr Ala Val Pro Glu Glu Leu Val Val Thr Glu Leu
    210                 215                 220

Glu Met Val His Met Cys Leu Ala Gly Asp His Asp Val Gly Leu Tyr
225                 230                 235                 240

Tyr Val Ile Leu Phe Thr Leu Ile Phe Leu Pro Cys Ile Val Ser Phe
                245                 250                 255

Leu Trp Leu Asn Ala Val Ile Ala Arg Gln Leu Trp Leu Arg Arg His
            260                 265                 270

Tyr His Gln Glu Gln Gln Glu Gln His Gln Glu Pro Lys Glu Gly Gln
        275                 280                 285

Phe Lys Thr Met Ala Asn Gly Gly Asp Leu Leu Met Pro Ser Thr Leu
    290                 295                 300

Val Ser Ala Met Gly Val Ala Val Pro Phe Ala Leu Asp Asn Thr Pro
305                 310                 315                 320

Leu Pro Pro Lys Ser Thr Val Asn Ala Pro Gly Lys Lys Thr Thr Ala
                325                 330                 335

Ala Ala Leu Ala Arg Glu Ala Arg His Arg Lys Met Val Val Val Val
            340                 345                 350

Leu Leu Met Met Ala Val Phe Ile Cys Leu Arg Leu Pro Ala Trp Val
        355                 360                 365

Phe Leu Ile Met Arg Leu Tyr Gly Ser Tyr Ser Glu Pro Ile Asp Trp
    370                 375                 380

Leu Leu Tyr Phe Ser Phe Gly Ile Leu Asn Leu Phe Ser Cys Ala Leu
385                 390                 395                 400

Asn Pro Ile Phe Tyr Thr Phe Leu Thr Gln Thr Ile Arg Thr Leu Thr
                405                 410                 415

Leu Val Lys His Lys Ile Gln Gly Phe Leu Gly Cys Pro Pro Gly Lys
            420                 425                 430

Val Pro Asp Gly Met Pro Thr Asp Gln Met Asp Lys Ser Gly Cys Cys
        435                 440                 445

Cys Gly Leu Arg Pro Pro Thr Phe Thr Trp Arg Cys His Pro Ser Arg
    450                 455                 460

Asp Arg Ala Ala Ala Thr Val Ile Arg Asp Val Asp Gln Pro Asp Pro
465                 470                 475                 480

Pro Ser Asp Gln Val Gln Pro Asp Pro Ser Ser Leu Arg Arg Phe Leu
                485                 490                 495

Ser Tyr Lys Gln Glu Val Phe Thr Ile Tyr Lys Gln Cys Gly Asp Ser
            500                 505                 510

Ser Ser Ala Ser Ile Glu Ser Ser Ala
        515                 520

<210> 45
<211> 816
<212> DNA

<213> Drosophila melanogaster

<220>
<221> CDS
<222> (1)..(813)

<400> 45
atg gag gac gag tgg ggc tcc ttt gat cgt ctg ccc agt gtt ccg agt    48
Met Glu Asp Glu Trp Gly Ser Phe Asp Arg Leu Pro Ser Val Pro Ser
1               5                   10                  . 15

gcc tcg atg gat ttg gag acg gaa aac gag gtg gtc agc aat tgg tcc    96
Ala Ser Met Asp Leu Glu Thr Glu Asn Glu Val Val Ser Asn Trp Ser
            20                  25                  30

aca ctg gcc aac ttc acg cga ctt gtg gct ggt gcc gct cct gaa atc    144
Thr Leu Ala Asn Phe Thr Arg Leu Val Ala Gly Ala Ala Pro Glu Ile
        35                  40                  45

gtc aac tat acg ctc aac atg atc gac gtg ggt gtg ggc atg gcc acg    192
Val Asn Tyr Thr Leu Asn Met Ile Asp Val Gly Val Gly Met Ala Thr
    50                  55                  60

gat ata tcc aat ttg agc gtt ccg gag cac gtg atg gat cac gca cct    240
Asp Ile Ser Asn Leu Ser Val Pro Glu His Val Met Asp His Ala Pro
65                  70                  75                  80

caa cta tcc cga tcc ggg ttg ctg aaa gtg tat gtc ctg gcg gta atg    288
Gln Leu Ser Arg Ser Gly Leu Leu Lys Val Tyr Val Leu Ala Val Met
                85                  90                  95

gca ctg ttc tct ctg ctg ggc aac ctg ctg acc atc tgg aat atc tac    336
Ala Leu Phe Ser Leu Leu Gly Asn Leu Leu Thr Ile Trp Asn Ile Tyr
            100                 105                 110

aaa acc cgc atc tca aga aga aac tca cgg cac acg tgg agt gct atc    384
Lys Thr Arg Ile Ser Arg Arg Asn Ser Arg His Thr Trp Ser Ala Ile
        115                 120                 125

tac tca ctg atg ttc cat ctg tcc atc gcc gat gtc ctg gtc acc tgg    432
Tyr Ser Leu Met Phe His Leu Ser Ile Ala Asp Val Leu Val Thr Trp
    130                 135                 140

ttc tgc att atc ggg gag gcc gcg tgg tgc tac acc gtc cag tgg ctg    480
Phe Cys Ile Ile Gly Glu Ala Ala Trp Cys Tyr Thr Val Gln Trp Leu
145                 150                 155                 160

gcc aat gag ctc acc tgc aag ctg gtg aag ctc ttc cag atg ttt agc    528

Ala Asn Glu Leu Thr Cys Lys Leu Val Lys Leu Phe Gln Met Phe Ser
                165                 170                 175

ctc tac ctg agc acc tat gtc ctg gtc ctc atc gga gtg gac cgc tgg     576
Leu Tyr Leu Ser Thr Tyr Val Leu Val Leu Ile Gly Val Asp Arg Trp
            180                 185                 190

ata gcg gtc aag tat ccg atg aag tcg ctc aac atg gcc aag agg tgt     624
Ile Ala Val Lys Tyr Pro Met Lys Ser Leu Asn Met Ala Lys Arg Cys
        195                 200                 205

cat agg cta ctt ggc ggt act tac atc ctg tcg ctg gtg ctc agc ttg     672
His Arg Leu Leu Gly Gly Thr Tyr Ile Leu Ser Leu Val Leu Ser Leu
    210                 215                 220

cca cag ata ggc aca ctg ggt gat ata ttc ctt tgc cct ggc agt cag     720
Pro Gln Ile Gly Thr Leu Gly Asp Ile Phe Leu Cys Pro Gly Ser Gln
225                 230                 235                 240

ctg gcg gat aag cct gaa tgg gac tgc att aat ggg cgt cac tcg ctt     768
Leu Ala Asp Lys Pro Glu Trp Asp Cys Ile Asn Gly Arg His Ser Leu
                245                 250                 255

tgt tct aca act cta gaa cga ctc ggc ttc aga ttt ccg gca ccc tga     816
Cys Ser Thr Thr Leu Glu Arg Leu Gly Phe Arg Phe Pro Ala Pro
            260                 265                 270

<210> 46
<211> 271
<212> PRT
<213> Drosophila melanogaster

<400> 46
Met Glu Asp Glu Trp Gly Ser Phe Asp Arg Leu Pro Ser Val Pro Ser
  1               5                  10                  15

Ala Ser Met Asp Leu Glu Thr Glu Asn Glu Val Val Ser Asn Trp Ser
             20                  25                  30

Thr Leu Ala Asn Phe Thr Arg Leu Val Ala Gly Ala Ala Pro Glu Ile
         35                  40                  45

Val Asn Tyr Thr Leu Asn Met Ile Asp Val Gly Val Gly Met Ala Thr
     50                  55                  60

Asp Ile Ser Asn Leu Ser Val Pro Glu His Val Met Asp His Ala Pro
 65                  70                  75                  80

Gln Leu Ser Arg Ser Gly Leu Leu Lys Val Tyr Val Leu Ala Val Met
                85                  90                  95

Ala Leu Phe Ser Leu Leu Gly Asn Leu Leu Thr Ile Trp Asn Ile Tyr
            100                 105                 110

Lys Thr Arg Ile Ser Arg Arg Asn Ser Arg His Thr Trp Ser Ala Ile
        115                 120                 125

Tyr Ser Leu Met Phe His Leu Ser Ile Ala Asp Val Leu Val Thr Trp
    130                 135                 140

Phe Cys Ile Ile Gly Glu Ala Ala Trp Cys Tyr Thr Val Gln Trp Leu
145                 150                 155                 160

Ala Asn Glu Leu Thr Cys Lys Leu Val Lys Leu Phe Gln Met Phe Ser
                165                 170                 175

Leu Tyr Leu Ser Thr Tyr Val Leu Val Leu Ile Gly Val Asp Arg Trp
            180                 185                 190

Ile Ala Val Lys Tyr Pro Met Lys Ser Leu Asn Met Ala Lys Arg Cys
        195                 200                 205

His Arg Leu Leu Gly Gly Thr Tyr Ile Leu Ser Leu Val Leu Ser Leu
    210                 215                 220

Pro Gln Ile Gly Thr Leu Gly Asp Ile Phe Leu Cys Pro Gly Ser Gln
225                 230                 235                 240

Leu Ala Asp Lys Pro Glu Trp Asp Cys Ile Asn Gly Arg His Ser Leu
                245                 250                 255

Cys Ser Thr Thr Leu Glu Arg Leu Gly Phe Arg Phe Pro Ala Pro
            260                 265                 270

<210> 47
<211> 21
<212> DNA
<213> Künstliche Sequenz

<220>
<223> Beschreibung der künstlichen Sequenz: Primer

<400> 47

aaggtcatca aaatgctgat t                                                21

<210> 48
<211> 21
<212> DNA
<213> Künstliche Sequenz

<220>
<223> Beschreibung der künstlichen Sequenz: Primer

<400> 48
attgtagcag ctattgctca t                                                21

<210> 49
<211> 21
<212> DNA
<213> Künstliche Sequenz

<220>
<223> Beschreibung der künstlichen Sequenz: Primer

<400> 49
cagctcgttc gattcggtcc t                                                21

<210> 50
<211> 21
<212> DNA
<213> Künstliche Sequenz

<220>
<223> Beschreibung der künstlichen Sequenz: Primer

<400> 50
gtgacagcgg tcatagtccg a                                                21

<210> 51
<211> 21
<212> DNA
<213> Künstliche Sequenz

<220>
<223> Beschreibung der künstlichen Sequenz: Primer

<400> 51

atcgaggcat ccacctatgg c 21

<210> 52
<211> 21
<212> DNA
<213> Künstliche Sequenz

<220>
<223> Beschreibung der künstlichen Sequenz: Primer

<400> 52
aggtgggcgc aggcatcgta g 21

<210> 53
<211> 23
<212> DNA
<213> Künstliche Sequenz

<220>
<223> Beschreibung der künstlichen Sequenz: Primer

<400> 53
ttgctacgta gttctgagga atc 23

<210> 54
<211> 21
<212> DNA
<213> Künstliche Sequenz

<220>
<223> Beschreibung der künstlichen Sequenz: Primer

<400> 54
atgcaggtgg agagcttcat g 21

<210> 55
<211> 21
<212> DNA
<213> Künstliche Sequenz

<220>
<223> Beschreibung der künstlichen Sequenz: Primer

<400> 55

tggcagacga gtgcttcctg a                                                21

<210> 56
<211> 20
<212> DNA
<213> Künstliche Sequenz

<220>
<223> Beschreibung der künstlichen Sequenz: Primer

<400> 56
ggaccgctga agttgaccag                                                   20

<210> 57
<211> 21
<212> DNA
<213> Künstliche Sequenz

<220>
<223> Beschreibung der künstlichen Sequenz: Primer

<400> 57
tggtctggta cctgctggtc a                                                21

<210> 58
<211> 21
<212> DNA
<213> Künstliche Sequenz

<220>
<223> Beschreibung der künstlichen Sequenz: Primer

<400> 58
gcgatgagcc atttgaccag c                                                21

<210> 59
<211> 21
<212> DNA
<213> Künstliche Sequenz

<220>
<223> Beschreibung der künstlichen Sequenz: Primer

<400> 59

gtgacccatg cgttcatcat c 21

<210> 60
<211> 19
<212> DNA
<213> Künstliche Sequenz

<220>
<223> Beschreibung der künstlichen Sequenz: Primer

<400> 60
ctgcagcatg ggcagaaag 19

<210> 61
<211> 21
<212> DNA
<213> Künstliche Sequenz

<220>
<223> Beschreibung der künstlichen Sequenz: Primer

<400> 61
gcgatcacct ggaagatctg c 21

<210> 62
<211> 21
<212> DNA
<213> Künstliche Sequenz

<220>
<223> Beschreibung der künstlichen Sequenz: Primer

<400> 62
tggtgatgcc aataggatac c 21

<210> 63
<211> 21
<212> DNA
<213> Künstliche Sequenz

<220>
<223> Beschreibung der künstlichen Sequenz: Primer

<400> 63

ctgttgcact tcctggtcta c 21

<210> 64
<211> 21
<212> DNA
<213> Künstliche Sequenz

<220>
<223> Beschreibung der künstlichen Sequenz: Primer

<400> 64
acgcacagct ccctgaattt c 21

<210> 65
<211> 20
<212> DNA
<213> Künstliche Sequenz

<220>
<223> Beschreibung der künstlichen Sequenz: Primer

<400> 65
gaggagcacg atgtgagtgg 20

<210> 66
<211> 20
<212> DNA
<213> Künstliche Sequenz

<220>
<223> Beschreibung der künstlichen Sequenz: Primer

<400> 66
cgtgtaaacg gataattctg 20

<210> 67
<211> 20
<212> DNA
<213> Künstliche Sequenz

<220>
<223> Beschreibung der künstlichen Sequenz: Primer

<400> 67

gtctggctga tacccagcta                                                20

<210> 68
<211> 21
<212> DNA
<213> Künstliche Sequenz

<220>
<223> Beschreibung der künstlichen Sequenz: Primer

<400> 68
acgctcttga ctttctcgaa c                                               21

<210> 69
<211> 21
<212> DNA
<213> Künstliche Sequenz

<220>
<223> Beschreibung der künstlichen Sequenz: Primer

<400> 69
ttcgcacacc agttctacga c                                               21

<210> 70
<211> 21
<212> DNA
<213> Künstliche Sequenz

<220>
<223> Beschreibung der künstlichen Sequenz: Primer

<400> 70
gcgttcatga agcagtaggt g                                               21

<210> 71
<211> 20
<212> DNA
<213> Künstliche Sequenz

<220>
<223> Beschreibung der künstlichen Sequenz: Primer

<400> 71

tacatctgca tcggacgtgg                                                20

<210> 72
<211> 19
<212> DNA
<213> Künstliche Sequenz

<220>
<223> Beschreibung der künstlichen Sequenz: Primer

<400> 72
cactatgccg cattgctcc                                                 19

<210> 73
<211> 21
<212> DNA
<213> Künstliche Sequenz

<220>
<223> Beschreibung der künstlichen Sequenz: Primer

<400> 73
gtgatctatg tggtgatgag g                                              21

<210> 74
<211> 21
<212> DNA
<213> Künstliche Sequenz

<220>
<223> Beschreibung der künstlichen Sequenz: Primer

<400> 74
cctcgactgc acggtgctgg c                                              21

<210> 75
<211> 21
<212> DNA
<213> Künstliche Sequenz

<220>
<223> Beschreibung der künstlichen Sequenz: Primer

<400> 75

gtcatcgttc tgggcaattc a                                                21

<210> 76
<211> 21
<212> DNA
<213> Künstliche Sequenz

<220>
<223> Beschreibung der künstlichen Sequenz: Primer

<400> 76
gatgctcatg gccaccagca c                                                21

<210> 77
<211> 21
<212> DNA
<213> Künstliche Sequenz

<220>
<223> Beschreibung der künstlichen Sequenz: Primer

<400> 77
aacgtactgc gagtgatcgt g                                                21

<210> 78
<211> 21
<212> DNA
<213> Künstliche Sequenz

<220>
<223> Beschreibung der künstlichen Sequenz: Primer

<400> 78
aatggcaaag gtgacatcgt g                                                21

<210> 79
<211> 21
<212> DNA
<213> Künstliche Sequenz

<220>
<223> Beschreibung der künstlichen Sequenz: Primer

<400> 79

atgtgccgca tcagcgagtt c                                                21

<210> 80
<211> 21
<212> DNA
<213> Künstliche Sequenz

<220>
<223> Beschreibung der künstlichen Sequenz: Primer

<400> 80
gttaccggtg gctgtgaaca c                                                21

<210> 81
<211> 21
<212> DNA
<213> Künstliche Sequenz

<220>
<223> Beschreibung der künstlichen Sequenz: Primer

<400> 81
cagaaaccgc tcaaggagac g                                                21

<210> 82
<211> 21
<212> DNA
<213> Künstliche Sequenz

<220>
<223> Beschreibung der künstlichen Sequenz: Primer

<400> 82
cctcagacga gccgcagtta g                                                21

<210> 83
<211> 19
<212> DNA
<213> Künstliche Sequenz

<220>
<223> Beschreibung der künstlichen Sequenz: Primer

<400> 83

gcactggcac tgctgctgg                                                    19

<210> 84
<211> 20
<212> DNA
<213> Künstliche Sequenz

<220>
<223> Beschreibung der künstlichen Sequenz: Primer

<400> 84
cacagccacc acggtgatgc                                                   20

<210> 85
<211> 21
<212> DNA
<213> Künstliche Sequenz

<220>
<223> Beschreibung der künstlichen Sequenz: Primer

<400> 85
ggcactttgc cgtggatagt g                                                 21

<210> 86
<211> 21
<212> DNA
<213> Künstliche Sequenz

<220>
<223> Beschreibung der künstlichen Sequenz: Primer

<400> 86
gaaccgatcg atggacatca g                                                 21

<210> 87
<211> 20
<212> DNA
<213> Künstliche Sequenz

<220>
<223> Beschreibung der künstlichen Sequenz: Primer

<400> 87

catcagctcc tacctgctgc                                                20

<210> 88
<211> 21
<212> DNA
<213> Künstliche Sequenz

<220>
<223> Beschreibung der künstlichen Sequenz: Primer

<400> 88
cgtagagcag cggattgata c                                                21

<210> 89
<211> 21
<212> DNA
<213> Künstliche Sequenz

<220>
<223> Beschreibung der künstlichen Sequenz: Primer

<400> 89
catctcactg gcctgcagtg a                                                21

<210> 90
<211> 20
<212> DNA
<213> Künstliche Sequenz

<220>
<223> Beschreibung der künstlichen Sequenz: Primer

<400> 90
catgctcaga gtcgacttcg                                                20

<210> 91
<211> 20
<212> DNA
<213> Künstliche Sequenz

<220>
<223> Beschreibung der künstlichen Sequenz: Primer

<400> 91

gcggtaatgg cactgttctc                                                    20

<210> 92
<211> 21
<212> DNA
<213> Künstliche Sequenz

<220>
<223> Beschreibung der künstlichen Sequenz: Primer

<400> 92
ggatactgtg gagaaccggt a                                                  21